# EUROPEAN PATENT APPLICATION

(11) **EP 4 062 972 A1**
(43) Date of publication of application: **28.09.2022**
(21) Application number: 20890718.8
(22) Date of filing: 20.11.2020
(51) Int. Cl.: A61P 13/02, A61P 25/04, A61P 25/22, A61P 25/24, A61P 43/00, C07D 471/18, A61K 31/439

(54) **PYRAZOLOMORPHINAN DERIVATIVE**

(30) Priority: 22.11.2019 JP 2019211820
(71) Applicant: The Kitasato Institute, Tokyo 108-8641 (JP); Nippon Chemiphar Co., Ltd., Tokyo 101-0032 (JP)
(72) Inventor: FUJII, Hideaki, Tokyo 108-8641 (JP); HIRAYAMA, Shigeto, Tokyo 108-8641 (JP); WATANABE, Yoshikazu, Misato-shi, Saitama 341-0005 (JP)
(74) Representative: Godemeyer Blum Lenze Patentanwälte Partnerschaft mbB - werkpatent
(86) International application number: PCT/JP2020/043523
(87) International publication number: WO 2021/100878

(57) **Abstract**

The present invention provides a compound having a selective opioid δ receptor agonist effect. The present invention provides a pyrazolomorphinan derivative represented by general formula (1) (in the formula, (II) R¹ represents a hydrogen atom, an alkyl group, a cycloalkylmethyl group, or the like, R² represents a hydrogen atom or a hydroxy protecting group, R³ represents a hydroxy group, an alkyl group, a partially unsaturated heterocyclic group, an aryl group, a heteroaryl group, or the like; and R⁴ represents a hydrogen atom, an alkyl group, an aralkyl group, a cycloalkyl group, a cycloalkyl alkyl group, a saturated heterocyclic group, an aryl group, a heteroaryl group, or the like). The pyrazolomorphinan derivative can be used as an active ingredient in an analgesic, an antidepressant, an anxiolytic, or the like.

## Description

### Technical Field

The present invention relates to a pharmaceutical composition containing a morphinan derivative having an opioid δ receptor agonist effect.

### Background Art

There are three subtypes µ, δ, and κ of opioid receptors, and it is known that the agonist of each subtype has an analgesic effect.

Morphine which is an agonist with high affinity to the opioid µ receptor has a strong analgesic effect, but has a side effect such as dependence, drug abuse, tolerance, respiratory depression, constipation due to gastrointestinal motility suppression, nausea vomiting, hypotension, depilation, cough reflex control, or drowsiness.

Eptazocine also having a strong analgesic effect is a selective agonist of the opioid κ receptor, and brings about perspiration, nausea vomiting, or mouth dryness although bringing about dependence, tolerance, drowsiness, constipation, or respiratory depression slightly.

On the other hand, an enkephalin, which is an endogenous ligand of the opioid δ receptor, is known to have an analgesic effect, and activation of the opioid δ receptor is known to bring about an antidepressant and anxiolytic effect and an antidiuretic effect in addition to the analgesic effect (Patent Literatures 1 and 2, Non Patent Literatures 1 to 6). In addition, an agonist for selectively activating the opioid δ receptor is expected to have few or no side effects exhibited through activation of the opioid µ receptor and opioid κ receptor. However, the opioid δ receptor agonists that can be used as pharmaceuticals have not yet been developed.

### Citation List

### Patent Literature

Patent Literature 1: JP 2006-522775 A
Patent Literature 2: WO 2001/046192 A
Patent Literature 3: CN 101,481,376

### Non Patent Literature

Non Patent Literature 1: J. Pharmacol. Exp. Ther. 2011, 338(1), 195-204
Non Patent Literature 2: Trends Neurosci. 2013, 36(3), 195-206
Non Patent Literature 3: Behav. Brain Res. 2011, 223(2), 271-279
Non Patent Literature 4: Neuropharmacology 2013, 67, 485-493
Non Patent Literature 5: Curr. Neuropharmacol., 2012, 10(3), 231-238
Non Patent Literature 6: J. Pharmacol. Exp. Ther. 2005, 315(2), 601-608

### Summary of Invention

### Technical Problem

In developing a highly selective opioid δ receptor agonist, it is preferable to obtain various lead compounds having different chemotypes. Therefore, the present inventors have focused on a pyrazolomorphinan skeleton, and have aimed to provide a selective opioid δ receptor agonist.

Patent Literature 3 discloses a 14-hydroxymorphinan compound for treating drug abuse or developing an analgesic agent free from side effects, and in particular, a compound having a pyrazolomorphinan skeleton is described. However, it is hard to say that an example compound having no substituent in a pyrazole ring has selectivity for the opioid δ receptor.

### Solution to Problem

As a result of intensive studies, the present inventors have found that introduction of a specific substituent at the 8-position of the pyrazolomorphinan skeleton exhibits a highly selective agonist effect on the opioid δ receptor, thereby completing the present invention.

That is, the present invention is as follows.
[1] A compound represented by the following general formula (I), a stereoisomer of the compound, a pharmaceutically acceptable salt thereof, or a solvate thereof: 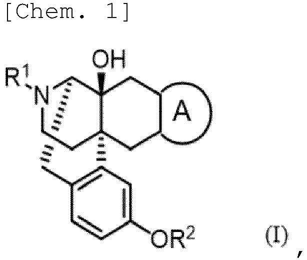 in the formula, 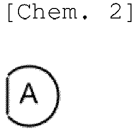 is represented by 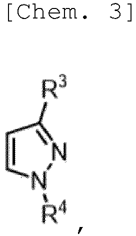 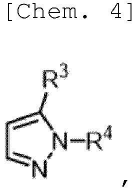 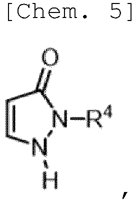 or 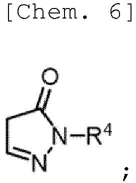
   R¹ represents a hydrogen atom, a C₁₋₆ alkyl group which may have a substituent, a C₂₋₆ alkenyl group which may have a substituent, or a C₃₋₁₀ cycloalkylmethyl group which may have a substituent;
   R² represents a hydrogen atom or a hydroxy protecting group;
   R³ represents a hydroxy group, a C₁₋₆ alkyl group which may have a substituent, a C₃₋₁₀ cycloalkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent; and
   R⁴ represents a hydrogen atom, a C₁₋₆ alkyl group which may have a substituent, a C₁₋₁₂ acyl group which may have a substituent, a C₇₋₁₂ aralkyl group which may have a substituent, a C₃₋₁₀ cycloalkyl group which may have a substituent, a C₃₋₁₀ cycloalkyl C₁₋₆ alkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent.
[2] The compound, the stereoisomer of the compound, the pharmaceutically acceptable salt thereof, or the solvate thereof according to [1], in which 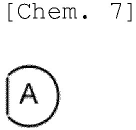 is 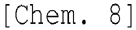 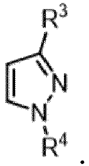
[3] The compound, the stereoisomer of the compound, the pharmaceutically acceptable salt thereof, or the solvate thereof according to [1], in which 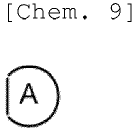 is 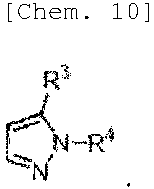
[4] The compound, the stereoisomer of the compound, the pharmaceutically acceptable salt thereof, or the solvate thereof according to any one of [1] to [3], in which R¹ is a methyl group, an allyl group, or a cyclopropylmethyl group.
[5] The compound, the stereoisomer of the compound, the pharmaceutically acceptable salt thereof, or the solvate thereof according to any one of [1] to [3], in which R¹ is a methyl group or a cyclopropylmethyl group.
[6] The compound, the stereoisomer of the compound, the pharmaceutically acceptable salt thereof, or the solvate thereof according to any one of [1] to [3], in which R¹ is a cyclopropylmethyl group.
[7] The compound, the stereoisomer of the compound, the pharmaceutically acceptable salt thereof, or the solvate thereof according to any one of [1] to [6], in which R³ is a hydroxy group, a C₁₋₆ alkyl group, a halo C₁₋₆ alkyl group, or a C₆₋₁₀ aryl group which may have a substituent.
[8] The compound, the stereoisomer of the compound, the pharmaceutically acceptable salt thereof, or the solvate thereof according to any one of [1] to [6], in which R³ is a methyl group, a trifluoromethyl group, or a phenyl group.
[9] The compound, the stereoisomer of the compound, the pharmaceutically acceptable salt thereof, or the solvate thereof according to any one of [1] to [6], in which R³ is a methyl group.
[10] The compound, the stereoisomer of the compound, the pharmaceutically acceptable salt thereof, or the solvate thereof according to any one of [1] to [9], in which R⁴ is a hydrogen atom, a C₁₋₆ alkyl group, a halo C₁₋₆ alkyl group, a C₁₋₆ alkyl group substituted with a hydroxy group, a C₁₋₆ alkyl group substituted with a C₁₋₆ alkoxy group, a benzoyl group which may have a substituent, a C₃₋₁₀ cycloalkyl group which may have a substituent, a C₃₋₁₀ cycloalkylmethyl group which may have a substituent, a C₆₋₁₀ aryl group which may have a substituent, or a 5- to 10-membered heteroaryl group which may have a substituent.
[11] The compound, the stereoisomer of the compound, the pharmaceutically acceptable salt thereof, or the solvate thereof according to [10], in which the 5- to 10-membered heteroaryl group which may have a substituent of R⁴ is a nitrogen-containing 5- or 6-membered heteroaryl group which may have a substituent.
[12] The compound, the stereoisomer of the compound, the pharmaceutically acceptable salt thereof, or the solvate thereof according to any one of [1] to [9], in which R⁴ is a hydrogen atom, a C₁₋₆ alkyl group, a C₁₋₆ alkyl group substituted with a hydroxy group, a C₁₋₆ alkyl group substituted with a C₁₋₆ alkoxy group, a benzoyl group, a C₃₋₇ cycloalkyl group, a C₃₋₇ cycloalkylmethyl group, a phenyl group, an oxazolyl group, a thiazolyl group, a pyrimidinyl group, or a pyridyl group.
[13] The compound, the stereoisomer of the compound, the pharmaceutically acceptable salt thereof, or the solvate thereof according to any one of [1] to [9], in which R⁴ is a hydrogen atom, a C₁₋₆ alkyl group, a benzoyl group, a C₃₋₇ cycloalkyl group, a C₃₋₇ cycloalkylmethyl group, a phenyl group, an oxazolyl group, a thiazolyl group, a pyrimidinyl group, or a pyridyl group.
[14] The compound, the stereoisomer of the compound, the pharmaceutically acceptable salt thereof, or the solvate thereof according to any one of [1] to [9], in which R⁴ is a hydrogen atom, a C₁₋₆ alkyl group, a benzoyl group, a C₃₋₇ cycloalkyl group, a C₃₋₇ cycloalkylmethyl group, a phenyl group, an oxazolyl group, a thiazolyl group, a pyrimidinyl group, a pyridyl group, or a 2-oxopyridyl group.
[15] The compound, the stereoisomer of the compound, the pharmaceutically acceptable salt thereof, or the solvate thereof according to any one of [1] to [9], in which R⁴ is a tetrahydropyranyl group, a tetrahydrothiopyranyl group, a 1-methylpiperidyl group, or an S-oxide-tetrahydrothiopyranyl group.
[16] The compound, the stereoisomer of the compound, the pharmaceutically acceptable salt thereof, or the solvate thereof according to any one of [1] to [9], in which R⁴ is a 2-oxopyridyl group, a tetrahydropyranyl group, a tetrahydrothiopyranyl group, a 1-methylpiperidyl group, or an S-oxide-tetrahydrothiopyranyl group.
[17] The compound, the stereoisomer of the compound, the pharmaceutically acceptable salt thereof, or the solvate thereof according to any one of [1] to [9], in which R⁴ is a hydrogen atom, an isopropyl group, a tert-butyl group, a 2-hydroxyethyl group, a benzoyl group, a cyclohexyl group, a cyclopropylmethyl group, a cyclohexylmethyl group, a phenyl group, a 2-thiazolyl group, or a 2-, 3-, or 4-pyridyl group.
[18] The compound, the stereoisomer of the compound, the pharmaceutically acceptable salt thereof, or the solvate thereof according to any one of [1] to [9], in which R⁴ is a hydrogen atom, an isopropyl group, a tert-butyl group, a benzoyl group, a cyclohexyl group, a cyclopropylmethyl group, a cyclohexylmethyl group, a phenyl group, a 2-thiazolyl group, or a 2-, 3-, or 4-pyridyl group.
[19] The compound, the stereoisomer of the compound, the pharmaceutically acceptable salt thereof, or the solvate thereof according to any one of [1] to [9], in which R⁴ is a hydrogen atom, an isopropyl group, a tert-butyl group, a benzoyl group, a cyclohexyl group, a cyclopropylmethyl group, a cyclohexylmethyl group, a phenyl group, a 2-thiazolyl group, a 2-, 3-, or 4-pyridyl group, or a 2-oxo-4-pyridyl group.
[20] The compound, the stereoisomer of the compound, the pharmaceutically acceptable salt thereof, or the solvate thereof according to any one of [1] to [9], in which R⁴ is a cyclopropyl group, a cyclobutyl group, a cyclopentyl group, a 4-tetrahydropyranyl group, a 4-tetrahydrothiopyranyl group, a 1-methylpiperidin-4-yl group, or an S-oxide-tetrahydrothiopyran-4-yl group.
[21] The compound, the stereoisomer of the compound, the pharmaceutically acceptable salt thereof, or the solvate thereof according to any one of [1] to [9], in which R⁴ is a 2-oxo-4-pyridyl group, a cyclopropyl group, a cyclobutyl group, a cyclopentyl group, a 4-tetrahydropyranyl group, a 4-tetrahydrothiopyranyl group, a 1-methylpiperidin-4-yl group, or an S-oxide-tetrahydrothiopyran-4-yl group.
[22] The compound, the stereoisomer of the compound, the pharmaceutically acceptable salt thereof, or the solvate thereof according to any one of [1] to [21], in which R² is a hydrogen atom.
[23] The compound, the stereoisomer of the compound, the pharmaceutically acceptable salt thereof, or the solvate thereof according to [1], in which the compound is selected from
   (6R,6aS,11aR)-14-(cyclopropylmethyl)-2-methoxy-8-methyl-9-phenyl-5,6,9,11-tetrahydro-6,11a-(epiminoethano)naphtho[2,1-f]indazol-6a(7H)-ol (Compound 4),
   (6R,6aS,11aR)-14-(cyclopropylmethyl)-2-methoxy-8-methyl-10-phenyl-5,6,10,11-tetrahydro-6,11a-(epiminoethano)naphtho[2,1-f]indazol-6a(7H)-ol (Compound 5),
   (6R,6aS,11aR)-14-(cyclopropylmethyl)-9-isopropyl-2-methoxy-8-methyl-5,6,9,11-tetrahydro-6,11a-(epiminoethano)naphtho[2,1-f]indazol-6a(7H)-ol (Compound 6),
   (6R,6aS,11aR)-14-(cyclopropylmethyl)-10-isopropyl-2-methoxy-8-methyl-5,6,10,11-tetrahydro-6,11a-(epiminoethano)naphtho[2,1-f]indazol-6a(7H)-ol (Compound 7),
   (6R,6aS,11aR)-9-(tert-butyl)-14-(cyclopropylmethyl)-2-methoxy-8-methyl-5,6,9,11-tetrahydro-6,11a-(epiminoethano)naphtho[2,1-f]indazol-6a(7H)-ol (Compound 8),
   (6R,6aS,11aR)-10-(tert-butyl)-14-(cyclopropylmethyl)-2-methoxy-8-methyl-5,6,10,11-tetrahydro-6,11a-(epiminoethano)naphtho[2,1-f]indazol-6a(7H)-ol (Compound 9),
   (6R,6aS,11aR)-10-benzyl-14-(cyclopropylmethyl)-2-methoxy-8-methyl-5,6,10,11-tetrahydro-6,11a-(epiminoethano)naphtho[2,1-f]indazol-6a(7H)-ol (Compound 10),
   (6R,6aS,11aR)-9-benzyl-14-(cyclopropylmethyl)-2-methoxy-8-methyl-5,6,9,11-tetrahydro-6,11a-(epiminoethano)naphtho[2,1-f]indazol-6a(7H)-ol (Compound 10a),
   (6R,6aS,11aR)-14-(cyclopropylmethyl)-2-methoxy-8-methyl-10-(pyridin-2-yl)-5,6,10,11-tetrahydro-6,11a-(epiminoethano)naphtho[2,1-f]indazol-6a(7H)-ol (Compound 11),
   (6R,6aS,11aR)-14-(cyclopropylmethyl)-2-methoxy-8-methyl-9-(pyridin-2-yl)-5,6,9,11-tetrahydro-6,11a-(epiminoethano)naphtho[2,1-f]indazol-6a(7H)-ol (Compound 11a),
   (6R,6aS,11aR)-14-(cyclopropylmethyl)-2-methoxy-8-methyl-10-(pyridin-4-yl)-5,6,10,11-tetrahydro-6,11a-(epiminoethano)naphtho[2,1-f]indazol-6a(7H)-ol (Compound 12),
   (6R,6aS,11aR)-14-(cyclopropylmethyl)-2-methoxy-8-methyl-9-(pyridin-4-yl)-5,6,9,11-tetrahydro-6,11a-(epiminoethano)naphtho[2,1-f]indazol-6a(7H)-ol (Compound 12a),
   (6R,6aS,11aR)-14-(cyclopropylmethyl)-2-methoxy-8-methyl-10-(pyrimidin-2-yl)-5,6,10,11-tetrahydro-6,11a-(epiminoethano)naphtho[2,1-f]indazol-6a(7H)-ol (Compound 13),
   (6R,6aS,11aR)-14-(cyclopropylmethyl)-2-methoxy-8-methyl-9-(pyrimidin-2-yl)-5,6,9,11-tetrahydro-6,11a-(epiminoethano)naphtho[2,1-f]indazol-6a(7H)-ol (Compound 13a),
   4-((6R,6aS,11aR)-14-(cyclopropylmethyl)-6a-hydroxy-2-methoxy-8-methyl-6,6a,7,11-tetrahydro-6,11a-(epiminoethano)naphtho[2,1-f]indazol-10(5H)-yl)pyridin-2(1H)-one (Compound 14),
   4-((6R,6aS,11aR)-14-(cyclopropylmethyl)-6a-hydroxy-2-methoxy-8-methyl-5,6,6a,7-tetrahydro-6,11a-(epiminoethano)naphtho[2,1-f]indazol-9(11H)-yl)pyridin-2(1H)-one (Compound 15),
   (6R,6aS,11aR)-10-cyclohexyl-14-(cyclopropylmethyl)-2-methoxy-8-methyl-5,6,10,11-tetrahydro-6,11a-(epiminoethano)naphtho[2,1-f]indazol-6a(7H)-ol (Compound 16),
   (6R,6aS,11aR)-9-cyclohexyl-14-(cyclopropylmethyl)-2-methoxy-8-methyl-5,6,9,11-tetrahydro-6,11a-(epiminoethano)naphtho[2,1-f]indazol-6a(7H)-ol (Compound 17),
   (6R,6aS,11aR)-14-(cyclopropylmethyl)-2-methoxy-8-methyl-5,6,9,11-tetrahydro-6,11a-(epiminoethano)naphtho[2,1-f]indazol-6a(7H)-ol (Compound 18),
   (6R,6aS,11aR)-14-(cyclopropylmethyl)-2-methoxy-8-methyl-5,6,10,11-tetrahydro-6,11a-(epiminoethano)naphtho[2,1-f]indazol-6a(7H)-ol (Compound 19),
   (6R,6aS,11aR)-14-(cyclopropylmethyl)-8-methyl-9-phenyl-5,6,9,11-tetrahydro-6,11a-(epiminoethano)naphtho[2,1-f]indazole-2,6a(7H)-diol (Compound 20),
   (6R,6aS,11aR)-14-(cyclopropylmethyl)-8-methyl-10-phenyl-5,6,10,11-tetrahydro-6,11a-(epiminoethano)naphtho[2,1-f]indazole-2,6a(7H)-diol (Compound 21),
   (6R,6aS,11aR)-14-(cyclopropylmethyl)-9-isopropyl-8-methyl-5,6,9,11-tetrahydro-6,11a-(epiminoethano)naphtho[2,1-f]indazole-2,6a(7H)-diol (Compound 22),
   (6R,6aS,11aR)-14-(cyclopropylmethyl)-10-isopropyl-8-methyl-5,6,10,11-tetrahydro-6,11a-(epiminoethano)naphtho[2,1-f]indazole-2,6a(7H)-diol (Compound 23),
   (6R,6aS,11aR)-9-(tert-butyl)-14-(cyclopropylmethyl)-8-methyl-5,6,9,11-tetrahydro-6,11a-(epiminoethano)naphtho[2,1-f]indazole-2,6a(7H)-diol (Compound 24),
   (6R,6aS,11aR)-10-(tert-butyl)-14-(cyclopropylmethyl)-8-methyl-5,6,10,11-tetrahydro-6,11a-(epiminoethano)naphtho[2,1-f]indazole-2,6a(7H)-diol (Compound 25),
   (6R,6aS,11aR)-10-benzyl-14-(cyclopropylmethyl)-8-methyl-5,6,10,11-tetrahydro-6,11a-(epiminoethano)naphtho[2,1-f]indazole-2,6a(7H)-diol (Compound 26),
   (6R,6aS,11aR)-14-(cyclopropylmethyl)-8-methyl-10-(pyridin-2-yl)-5,6,10,11-tetrahydro-6,11a-(epiminoethano)naphtho[2,1-f]indazole-2,6a(7H)-diol (Compound 27),
   (6R,6aS,11aR)-14-(cyclopropylmethyl)-8-methyl-10-(pyridin-4-yl)-5,6,10,11-tetrahydro-6,11a-(epiminoethano)naphtho[2,1-f]indazole-2,6a(7H)-diol (Compound 28),
   (6R,6aS,11aR)-14-(cyclopropylmethyl)-8-methyl-10-(pyrimidin-2-yl)-5,6,10,11-tetrahydro-6,11a-(epiminoethano)naphtho[2,1-f]indazole-2,6a(7H)-diol (Compound 29),
   4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)pyridin-2(1H)-one (Compound 30),
   4-((6R,6aS,11aR)-14-(cyclopropylmethyl)-2,6a-dihydroxy-8-methyl-5,6,6a,7-tetrahydro-6,11a-(epiminoethano)naphtho[2,1-f]indazol-9(11H)-yl)pyridin-2(1H)-one (Compound 31),
   (6R,6aS,11aR)-10-cyclohexyl-14-(cyclopropylmethyl)-8-methyl-5,6,10,11-tetrahydro-6,11a-(epiminoethano)naphtho[2,1-f]indazole-2,6a(7H)-diol (Compound 32),
   (6R,6aS,11aR)-9-cyclohexyl-14-(cyclopropylmethyl)-8-methyl-5,6,9,11-tetrahydro-6,11a-(epiminoethano)naphtho[2,1-f]indazole-2,6a(7H)-diol (Compound 33),
   (6R,6aS,11aR)-14-(cyclopropylmethyl)-8-methyl-5,6,9,11-tetrahydro-6,11a-(epiminoethano)naphtho[2,1-f]indazole-2,6a(7H)-diol (Compound 34),
   (6R,6aS,11aR)-14-(cyclopropylmethyl)-8-methyl-5,6,10,11-tetrahydro-6,11a-(epiminoethano)naphtho[2,1-f]indazole-2,6a(7H)-diol (Compound 35),
   ((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)(phenyl)methanone (Compound 36),
   (6R,6aS,11aR)-9-(cyclohexylmethyl)-14-(cyclopropylmethyl)-2-methoxy-8-methyl-5,6,9,11-tetrahydro-6,11a-(epiminoethano)naphtho[2,1-f]indazol-6a(7H)-ol (Compound 37),
   (6R,6aS,11aR)-10-(cyclohexylmethyl)-14-(cyclopropylmethyl)-2-methoxy-8-methyl-5,6,10,11-tetrahydro-6,11a-(epiminoethano)naphtho[2,1-f]indazol-6a(7H)-ol (Compound 38),
   (6R,6aS,11aR)-14-(cyclopropylmethyl)-2-methoxy-10-(2-methoxyethyl)-8-methyl-5,6,10,11-tetrahydro-6,11a-(epiminoethano)naphtho[2,1-f]indazol-6a(7H)-ol (Compound 39),
   (6R,6aS,11aR)-10,14-bis(cyclopropylmethyl)-2-methoxy-8-methyl-5,6,10,11-tetrahydro-6,11a-(epiminoethano)naphtho[2,1-f]indazol-6a(7H)-ol (Compound 40),
   (6R,6aS,11aR)-9,14-bis(cyclopropylmethyl)-2-methoxy-8-methyl-5,6,9,11-tetrahydro-6,11a-(epiminoethano)naphtho[2,1-f]indazol-6a(7H)-ol (Compound 41),
   (6R,6aS,11aR)-9-(cyclohexylmethyl)-14-(cyclopropylmethyl)-8-methyl-5,6,9,11-tetrahydro-6,11a-(epiminoethano)naphtho[2,1-f]indazole-2,6a(7H)-diol (Compound 42),
   (6R,6aS,11aR)-10-(cyclohexylmethyl)-14-(cyclopropylmethyl)-8-methyl-5,6,10,11-tetrahydro-6,11a-(epiminoethano)naphtho[2,1-f]indazole-2,6a(7H)-diol (Compound 43),
   (6R,6aS,11aR)-14-(cyclopropylmethyl)-10-(2-hydroxyethyl)-8-methyl-5,6,10,11-tetrahydro-6,11a-(epiminoethano)naphtho[2,1-f]indazole-2,6a(7H)-diol (Compound 44),
   (6R,6aS,11aR)-10,14-bis(cyclopropylmethyl)-8-methyl-5,6,10,11-tetrahydro-6,11a-(epiminoethano)naphtho[2,1-f]indazole-2,6a(7H)-diol (Compound 45),
   (6R,6aS,11aR)-9,14-bis(cyclopropylmethyl)-8-methyl-5,6,9,11-tetrahydro-6,11a-(epiminoethano)naphtho[2,1-f]indazole-2,6a(7H)-diol (Compound 46),
   (6R,6aS,11aR)-14-(cyclopropylmethyl)-2-methoxy-8-methyl-10-(pyridin-3-yl)-5,6,10,11-tetrahydro-6,11a-(epiminoethano)naphtho[2,1-f]indazol-6a(7H)-ol (Compound 47),
   (6R,6aS,11aR)-14-(cyclopropylmethyl)-2-methoxy-8-methyl-9-(pyridin-3-yl)-5,6,9,11-tetrahydro-6,11a-(epiminoethano)naphtho[2,1-f]indazol-6a(7H)-ol (Compound 48),
   (6R,6aS,11aR)-14-(cyclopropylmethyl)-8-methyl-10-(pyridin-3-yl)-5,6,10,11-tetrahydro-6,11a-(epiminoethano)naphtho[2,1-f]indazole-2,6a(7H)-diol (Compound 49),
   (6R,6aS,11aR)-14-(cyclopropylmethyl)-2-methoxy-8-methyl-10-(thiazol-2-yl)-5,6,10,11-tetrahydro-6,11a-(epiminoethano)naphtho[2,1-f]indazol-6a(7H)-ol (Compound 50),
   (6R,6aS,11aR)-14-(cyclopropylmethyl)-8-methyl-10-(thiazol-2-yl)-5,6,10,11-tetrahydro-6,11a-(epiminoethano)naphtho[2,1-f]indazole-2,6a(7H)-diol (Compound 51),
   (6R,6aS,11aR)-2-(benzyloxy)-14-(cyclopropylmethyl)-8-methyl-5,6,9,11-tetrahydro-6,11a-(epiminoethano)naphtho[2,1-f]indazol-6a(7H)-ol (Compound 56),
   (6R,6aS,11aR)-2-(benzyloxy)-14-(cyclopropylmethyl)-8-methyl-5,6,10,11-tetrahydro-6,11a-(epiminoethano)naphtho[2,1-f]indazol-6a(7H)-ol (Compound 57),
   (6R,6aS,11aR)-2-(benzyloxy)-14-(cyclopropylmethyl)-10-(2-methoxyethyl)-8-methyl-5,6,10,11-tetrahydro-6,11a-(epiminoethano)naphtho[2,1-f]indazol-6a(7H)-ol (Compound 58),
   (6R,6aS,11aR)-14-(cyclopropylmethyl)-10-(2-methoxyethyl)-8-methyl-5,6,10,11-tetrahydro-6,11a-(epiminoethano)naphtho[2,1-f]indazole-2,6a(7H)-diol (Compound 59),
   (6R,6aS,11aR)-2-methoxy-8,14-dimethyl-10-phenyl-5,6,10,11-tetrahydro-6,11a-(epiminoethano)naphtho[2,1-f]indazol-6a(7H)-ol (Compound 60),
   (6R,6aS,11aR)-8,14-dimethyl-10-phenyl-5,6,10,11-tetrahydro-6,11a-(epiminoethano)naphtho[2,1-f]indazole-2,6a(7H)-diol (Compound 61),
   (6R,6aS,11aR)-10-benzyl-2-methoxy-8,14-dimethyl-5,6,10,11-tetrahydro-6,11a-(epiminoethano)naphtho[2,1-f]indazol-6a(7H)-ol (Compound 65),
   (6R,6aS,11aR)-9-benzyl-2-methoxy-8,14-dimethyl-5,6,9,11-tetrahydro-6,11a-(epiminoethano)naphtho[2,1-f]indazol-6a(7H)-ol (Compound 65a),
   (6R,6aS,11aR)-10-benzyl-8,14-dimethyl-5,6,10,11-tetrahydro-6,11a-(epiminoethano)naphtho[2,1-f]indazole-2,6a(7H)-diol (Compound 66),
   (6R,6aS,11aR)-2-(benzyloxy)-14-(cyclopropylmethyl)-6a-hydroxy-9-phenyl-6,6a,7,7a,9,11-hexahydro-6,11a-(epiminoethano)naphtho[2,1-f]indazol-8(5H)-one (Compound 67),
   (6R,6aS,11aR)-14-(cyclopropylmethyl)-2,6a-dihydroxy-9-phenyl-6,6a,7,9,10,11-hexahydro-6,11a-(epiminoethano)naphtho[2,1-f]indazol-8(5H)-one (Compound 68), and
   (6R,6aS,11aR)-14-(cyclopropylmethyl)-2,6a-dihydroxy-9-isopropyl-6,6a,7,9,10,11-hexahydro-6,11a-(epiminoethano)naphtho[2,1-f]indazol-8(5H)-one (Compound 69) .
[24] The compound, the stereoisomer of the compound, the pharmaceutically acceptable salt thereof, or the solvate thereof according to [1], in which the compound is selected from
   (6R,6aS,11aR)-10-cyclopropyl-14-(cyclopropylmethyl)-2-methoxy-8-methyl-5,6,10,11-tetrahydro-6,11a-(epiminoethano)naphtho[2,1-f]indazol-6a(7H)-ol (Compound 70),
   (6R,6aS,11aR)-9-cyclopropyl-14-(cyclopropylmethyl)-2-methoxy-8-methyl-5,6,9,11-tetrahydro-6,11a-(epiminoethano)naphtho[2,1-f]indazol-6a(7H)-ol (Compound 71),
   (6R,6aS,11aR)-10-cyclobutyl-14-(cyclopropylmethyl)-2-methoxy-8-methyl-5,6,10,11-tetrahydro-6,11a-(epiminoethano)naphtho[2,1-f]indazol-6a(7H)-ol (Compound 72),
   (6R,6aS,11aR)-9-cyclobutyl-14-(cyclopropylmethyl)-2-methoxy-8-methyl-5,6,9,11-tetrahydro-6,11a-(epiminoethano)naphtho[2,1-f]indazol-6a(7H)-ol (Compound 73),
   (6R,6aS,11aR)-10-cyclopentyl-14-(cyclopropylmethyl)-2-methoxy-8-methyl-5,6,10,11-tetrahydro-6,11a-(epiminoethano)naphtho[2,1-f]indazol-6a(7H)-ol (Compound 74),
   (6R,6aS,11aR)-9-cyclopentyl-14-(cyclopropylmethyl)-2-methoxy-8-methyl-5,6,9,11-tetrahydro-6,11a-(epiminoethano)naphtho[2,1-f]indazol-6a(7H)-ol (Compound 75),
   (6R,6aS,11aR)-14-(cyclopropylmethyl)-2-methoxy-8-methyl-10-(tetrahydro-2H-pyran-4-yl)-5,6,10,11-tetrahydro-6,11a-(epiminoethano)naphtho[2,1-f]indazol-6a(7H)-ol (Compound 76),
   (6R,6aS,11aR)-14-(cyclopropylmethyl)-2-methoxy-8-methyl-10-(tetrahydro-2H-pyran-4-yl)-5,6,10,11-tetrahydro-6,11a-(epiminoethano)naphtho[2,1-f]indazol-6a(7H)-ol (Compound 76),
   (6R,6aS,11aR)-14-(cyclopropylmethyl)-2-methoxy-8-methyl-10-(tetrahydro-2H-pyran-4-yl)-5,6,9,11-tetrahydro-6,11a-(epiminoethano)naphtho[2,1-f]indazol-6a(7H)-ol (Compound 77),
   (6R,6aS,11aR)-14-(cyclopropylmethyl)-2-methoxy-8-methyl-10-(tetrahydro-2H-thiopyran-4-yl)-5,6,10,11-tetrahydro-6,11a-(epiminoethano)naphtho[2,1-f]indazol-6a(7H)-ol (Compound 78),
   (6R,6aS,11aR)-14-(cyclopropylmethyl)-2-methoxy-8-methyl-10-(tetrahydro-2H-thiopyran-4-yl)-5,6,9,11-tetrahydro-6,11a-(epiminoethano)naphtho[2,1-f]indazol-6a(7H)-ol (Compound 79),
   (6R,6aS,11aR)-14-(cyclopropylmethyl)-2-methoxy-8-methyl-10-(1-methylpiperidin-4-yl)-5,6,10,11-tetrahydro-6,11a-(epiminoethano)naphtho[2,1-f]indazol-6a(7H)-ol (Compound 80),
   (6R,6aS,11aR)-14-(cyclopropylmethyl)-2-methoxy-8-methyl-10-(1-methylpiperidin-4-yl)-5,6,9,11-tetrahydro-6,11a-(epiminoethano)naphtho[2,1-f]indazol-6a(7H)-ol (Compound 81),
   (6R,6aS,11aR)-10-cyclopropyl-14-(cyclopropylmethyl)-8-methyl-5,6,10,11-tetrahydro-6,11a-(epiminoethano)naphtho[2,1-f]indazole-2,6a(7H)-diol (Compound 82),
   (6R,6aS,11aR)-9-cyclopropyl-14-(cyclopropylmethyl)-8-methyl-5,6,9,11-tetrahydro-6,11a-(epiminoethano)naphtho[2,1-f]indazole-2,6a(7H)-diol (Compound 83),
   (6R,6aS,11aR)-10-cyclobutyl-14-(cyclopropylmethyl)-8-methyl-5,6,10,11-tetrahydro-6,11a-(epiminoethano)naphtho[2,1-f]indazole-2,6a(7H)-diol (Compound 84),
   (6R,6aS,11aR)-9-cyclobutyl-14-(cyclopropylmethyl)-8-methyl-5,6,9,11-tetrahydro-6,11a-(epiminoethano)naphtho[2,1-f]indazole-2,6a(7H)-diol (Compound 85),
   (6R,6aS,11aR)-10-cyclopentyl-14-(cyclopropylmethyl)-8-methyl-5,6,10,11-tetrahydro-6,11a-(epiminoethano)naphtho[2,1-f]indazole-2,6a(7H)-diol (Compound 86),
   (6R,6aS,11aR)-9-cyclopentyl-14-(cyclopropylmethyl)-8-methyl-5,6,9,11-tetrahydro-6,11a-(epiminoethano)naphtho[2,1-f]indazole-2,6a(7H)-diol (Compound 87),
   (6R,6aS,11aR)-14-(cyclopropylmethyl)-8-methyl-10-(tetrahydro-2H-pyran-4-yl)-5,6,10,11-tetrahydro-6,11a-(epiminoethano)naphtho[2,1-f]indazole-2,6a(7H)-diol (Compound 88),
   (6R,6aS,11aR)-14-(cyclopropylmethyl)-8-methyl-10-(tetrahydro-2H-thiopyran-4-yl)-5,6,10,11-tetrahydro-6,11a-(epiminoethano)naphtho[2,1-f]indazole-2,6a(7H)-diol (Compound 89),
   (6R,6aS,11aR)-14-(cyclopropylmethyl)-8-methyl-9-(tetrahydro-2H-thiopyran-4-yl)-5,6,9,11-tetrahydro-6,11a-(epiminoethano)naphtho[2,1-f]indazole-2,6a(7H)-diol (Compound 90),
   (6R,6aS,11aR)-14-(cyclopropylmethyl)-8-methyl-10-(1-methylpiperidin-4-yl)-5,6,10,11-tetrahydro-6,11a-(epiminoethano)naphtho[2,1-f]indazole-2,6a(7H)-diol (Compound 91),
   (6R,6aS,11aR)-14-(cyclopropylmethyl)-8-methyl-9-(1-methylpiperidin-4-yl)-5,6,9,11-tetrahydro-6,11a-(epiminoethano)naphtho[2,1-f]indazole-2,6a(7H)-diol (Compound 92),
   (1S,4S)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 93), and
   (1S,4R)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 94).
[25] A pharmaceutical composition containing the compound, the pharmaceutically acceptable salt thereof, or the solvate thereof according to any one of [1] to [24].
[26] A pharmaceutical composition containing the compound or the pharmaceutically acceptable salt thereof according to any one of [1] to [24].
[27] The compound, the stereoisomer of the compound, the pharmaceutically acceptable salt thereof, or the solvate thereof according to any one of [1] to [21], in which R² is a hydroxy protecting group.
[28] A pharmaceutical composition containing the compound, the stereoisomer of the compound, the pharmaceutically acceptable salt thereof, or the solvate thereof according to any one of [1] to [26].
[29] An opioid δ receptor agonist containing the compound, the stereoisomer of the compound, the pharmaceutically acceptable salt thereof, or the solvate thereof according to any one of [1] to [26].
[30] An analgesic containing the compound, the stereoisomer of the compound, the pharmaceutically acceptable salt thereof, or the solvate thereof according to any one of [1] to [26].
[31] An antidepressant containing the compound, the stereoisomer of the compound, the pharmaceutically acceptable salt thereof, or the solvate thereof according to any one of [1] to [26].
[32] An anxiolytic containing the compound, the stereoisomer of the compound, the pharmaceutically acceptable salt thereof, or the solvate thereof according to any one of [1] to [26].
[33] A therapeutic agent for dysuria containing the compound, the stereoisomer of the compound, the pharmaceutically acceptable salt thereof, or the solvate thereof according to any one of [1] to [26].
[34] A pharmaceutical composition containing the compound, the stereoisomer of the compound, the pharmaceutically acceptable salt thereof, or the solvate thereof according to any one of [1] to [26], in which the pharmaceutical composition is used for treating pain.
[35] A pharmaceutical composition containing the compound, the stereoisomer of the compound, the pharmaceutically acceptable salt thereof, or the solvate thereof according to any one of [1] to [26], in which the pharmaceutical composition is used for treating depression.
[36] A pharmaceutical composition containing the compound, the stereoisomer of the compound, the pharmaceutically acceptable salt thereof, or the solvate thereof according to any one of [1] to [26], in which the pharmaceutical composition is used for treating anxiety.
[37] A pharmaceutical composition containing the compound, the stereoisomer of the compound, the pharmaceutically acceptable salt thereof, or the solvate thereof according to any one of [1] to [26], in which the pharmaceutical composition is used for treating dysuria.
[38] A use of the compound, the stereoisomer of the compound, the pharmaceutically acceptable salt thereof, or the solvate thereof according to any one of [1] to [26], in which the use is for producing an analgesic pharmaceutical composition.
[39] A use of the compound, the stereoisomer of the compound, the pharmaceutically acceptable salt thereof, or the solvate thereof according to any one of [1] to [26], in which the use is for producing an antidepressant pharmaceutical composition.
[40] A use of the compound, the stereoisomer of the compound, the pharmaceutically acceptable salt thereof, or the solvate thereof according to any one of [1] to [26], in which the use is for producing an anxiolytic pharmaceutical composition.
[41] A use of the compound, the stereoisomer of the compound, the pharmaceutically acceptable salt thereof, or the solvate thereof according to any one of [1] to [26], in which the use is for producing an anti-dysuria pharmaceutical composition.
[42] A method for treating pain in a mammalian subject (for example, a human), the method including administering an effective amount of the compound, the stereoisomer of the compound, the pharmaceutically acceptable salt thereof, or the solvate thereof according to any one of [1] to [26] to a subject requiring treatment of pain.
[43] A method for treating depression in a mammalian subject (for example, a human), the method including administering an effective amount of the compound, the stereoisomer of the compound, the pharmaceutically acceptable salt thereof, or the solvate thereof according to any one of [1] to [26] to a subject requiring treatment of depression.
[44] A method for treating anxiety in a mammalian subject (for example, a human), the method including administering an effective amount of the compound, the stereoisomer of the compound, the pharmaceutically acceptable salt thereof, or the solvate thereof according to any one of [1] to [26] to a subject requiring treatment of anxiety.
[45] A method for treating dysuria in a mammalian subject (for example, a human), the method including administering an effective amount of the compound, the stereoisomer of the compound, the pharmaceutically acceptable salt thereof, or the solvate thereof according to any one of [1] to [26] to a subject requiring treatment of dysuria.

### Advantageous Effects of Invention

The pyrazolomorphinan derivative represented by general formula (I) of the present invention has a selective opioid δ receptor agonist effect, such that the pyrazolomorphinan derivative is useful as a therapeutic agent and/or a prophylactic agent for pain, anxiety, depression, and dysuria. In addition, the compound of the present invention exhibits no or only weak activation on opioid µ and κ receptors, and thus has no or only weak side effect such as dependence, drug abuse, tolerance, respiratory depression, constipation due to gastrointestinal motility suppression, nausea vomiting, hypotension, depilation, cough reflex control, drowsiness, perspiration, or mouth dryness.

### Description of Embodiments

Hereinafter, the present invention will be described in detail.

In the present specification, "pyrazolomorphinan" is a compound having the following basic skeleton, and a position number thereof is as shown in the following structure.

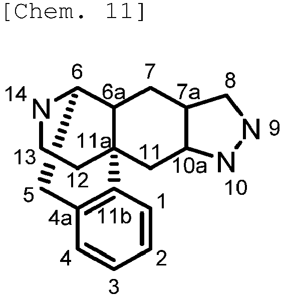

The term "C₁₋₆ alkyl group" refers to a linear or branched acyclic saturated hydrocarbon group having 1 to 6 carbon atoms. Examples of the alkyl group include a methyl group, an ethyl group, a propyl group, an isopropyl group, a butyl group, a tert-butyl group, a pentyl group, a neopentyl group, and a hexyl group. The alkyl group is preferably a C₁₋₄ alkyl group having 1 to 4 carbon atoms.

The term "C₂₋₆ alkenyl group" refers to a linear or branched acyclic unsaturated hydrocarbon group having one or more carbon-carbon double bonds and 2 to 6 carbon atoms. Examples of the alkenyl group include an ethenyl group, an allyl group, a 1-propen-2-yl group, a 2-propen-1-yl group, a 1,3-butadien-1-yl group, and a 1,3-butadien-2-yl group. The alkenyl group is preferably a C₂₋₄ alkenyl group having 2 to 4 carbon atoms.

The term "C₃₋₁₀ cycloalkyl group" refers to a saturated monocyclic or polycyclic hydrocarbon group having 3 to 10 carbon atoms, and the polycyclic hydrocarbon group may have a spiro ring, a fused ring, and a crosslinked ring. The cycloalkyl group is preferably a C₃₋₇ cycloalkyl group having 3 to 7 carbon atoms. Examples of the monocyclic cycloalkyl group include a cyclopropyl group, a cyclobutyl group, a cyclopentyl group, and a cyclohexyl group.

Examples of the spiro ring in the polycyclic hydrocarbon group include a spiro[2.2]pentyl group, a spiro[2.3]hexyl group, a spiro[3.3]heptyl group, a spiro[3.5]nonyl group, and a spiro[4.5]decanyl group.

Examples of the fused ring in the polycyclic hydrocarbon group include a bicyclo[4.2.0]octyl group, a bicyclo[3.2.0]heptyl group, a decahydronaphthyl group, and an octahydroindenyl group.

Examples of the crosslinked ring in the polycyclic hydrocarbon group include a norbornyl group, a bicyclo[2.2.2]octyl group, and an adamantyl group.

The term "C₃₋₁₀ cycloalkylmethyl group" refers to a methyl group having a C₃₋₁₀ cycloalkyl group. Examples of the cycloalkylmethyl group include a cyclopropylmethyl group, a cyclobutylmethyl group, a cyclopentylmethyl group, and a cyclohexylmethyl group. The cycloalkylmethyl group is preferably a C₃₋₇ cycloalkylmethyl group having 3 to 7 carbon atoms.

The term "saturated heterocyclic group" refers to a saturated cyclic group that is 3- to 7-membered monocyclic or 6- to 10-membered polycyclic, and contains at least one heteroatom selected from O, S, and N, in which N and S may be oxidized in various oxidation states. The polycyclic saturated heterocyclic group may have a spiro ring, a fused ring, and a crosslinked ring. Examples of the monocyclic saturated heterocyclic group include an oxiranyl group, a thiaranyl group, an aziridinyl group, an oxetanyl group, a thiatanyl group, an azetidinyl group, a tetrahydrofuranyl group, a tetrahydrothiophenyl, a pyrrolidinyl group, a tetrahydropyranyl group, tetrahydrothiopyranyl, a piperidinyl group, a 1,4-dioxanyl group, a morpholinyl group, a 1,4-dithianyl group, a piperazinyl group, a 1,4-azathianyl group, an oxepanyl group, a thiepanyl group, an azepanyl group, an S-oxide-thiaranyl group, an S,S-dioxide-thiaranyl group, an S-oxide-thiatanyl group, an S,S-dioxide-thiatanyl group, an S-oxide-tetrahydrothiophenyl group, an S,S-dioxide-tetrahydrothiophenyl group, an S-oxide-tetrahydrothiopyranyl group, and an S,S-dioxide-tetrahydrothiopyranyl group.

Examples of the spiro ring in the polycyclic saturated heterocyclic group include an azaspiro[2.3]hexyl group, an azaspiro[3.3]heptyl group, an azaspiro[3.5]nonyl group, a diazaspiro[3.3]heptyl group, an oxaspiro[2.3]hexyl group, and an oxaspiro[3.3]heptyl group.

Examples of the fused ring in the polycyclic saturated heterocyclic group include an azabicyclo[2.2.0]hexyl group, an azabicyclo[4.2.0]octyl group, an octahydroindolyl group, a decahydroquinolyl group, a decahydroisoquinolyl group, a diazabicyclo[2.2.0]hexyl group, an octahydropyrrolopyridyl group, and a decahydronaphthyldinyl group.

Examples of the crosslinked ring in the polycyclic saturated heterocyclic group include an azanorbornyl group, an azabicyclo[2.2.2]octyl group, an azabicyclo[3.2.2]nonyl group, an oxanorbornyl group, an oxabicyclo[2.2.2]octyl group, and an oxabicyclo[3.2.2]nonyl group.

The term "partially unsaturated heterocyclic group" refers to a partially unsaturated cyclic group that is 3-to 7-membered monocyclic or 4- to 10-membered polycyclic, and contains at least one heteroatom selected from O, S, and N, in which N and S may be oxidized in various oxidation states. The polycyclic partially unsaturated heterocyclic group may have a spiro ring, a fused ring, and a crosslinked ring.

Examples of a monocyclic partially unsaturated heterocyclic group include a 5-membered partially unsaturated heterocyclic group such as a pyrrolinyl group, a dihydrofuranyl group, an imidazolinyl group, a pyrazolinyl group, an oxazolinyl group, or an isooxazolinyl group; and a 6-membered partially unsaturated heterocyclic group such as a pyranyl group, a dihydropyranyl group, or a 1,2,3,6-tetrahydropyridyl group.

Examples of the spiro ring in the polycyclic partially unsaturated heterocyclic group include an azaspiro[4.4]nonenyl group, an azaspiro[4.5]decenyl group, an azaspiro[3.4]octenyl group, an oxapyro[4.4]nonenyl group, an oxaspiro[4.5]decenyl group, and an oxaspiro[3.4]octenyl group.

Examples of the fused ring in the polycyclic partially unsaturated heterocyclic group include an azabicyclo[4.2.0]octenyl group, a hexahydroindolyl group,
an octahydroquinolyl group, a hexahydroquinolyl group, an octahydroisoquinolyl group, a hexahydroisoquinolyl group, and an oxabicyclo[4.2.0]octenyl group.

Examples of the crosslinked ring in the polycyclic partially unsaturated heterocyclic ring include an azanorbornenyl group, an azabicyclo[2.2.2]octenyl group, an azabicyclo[3.2.2]nonenyl group, an oxanorbornenyl group, an oxabicyclo[2.2.2]octenyl group, and an oxabicyclo[3.2.2]nonenyl group.

The term "C₆₋₁₀ aryl group" refers to a monocyclic or polycyclic aromatic hydrocarbon group having 6 to 10 carbon atoms and having at least partially aromatic ring structure. Examples of the aryl include a phenyl group, a naphthyl group, an indanyl group, an indenyl group, and an azulenyl group.

The term "5- to 10-membered heteroaryl group" refers to a monocyclic or polycyclic heteroaryl group containing 1 to 4 heteratoms selected from a nitrogen atom, an oxygen atom, and a sulfur atom as a ring constituent atom.

Examples of the monocyclic heteroaryl group include a 5-membered ring heteroaryl group such as a furanyl group, a thienyl group, a pyrrolyl group, an imidazolyl group, a pyrazolyl group, an oxazolyl group, an isooxazolyl group, a thiazolyl group, an isothiazolyl group, a triazolyl group, or a tetrazolyl group; and a 6-membered ring heteroaryl group such as a pyridyl group, a pyridazinyl group, a pyrazinyl group, or a pyrimidyl group.

Examples of the polycyclic heteroaryl group include polycyclic heteroaryl groups such as a quinoline group, an isoquinolyl group, a quinazolyl group, a quinoxalyl group, an indolyl group, an indazolyl group, a benzoimidazolyl group, a benzofuranyl group, a benzothienyl group, a benzoxazolyl group, a benzothiazolyl group, an imidazolypyridinyl group, and a pyrazolopyridinyl group.

The term "C₁₋₁₂ acyl group" is a carbonyl having a substituent and refers to an alkanoyl group and an aroyl group. The alkanoyl group includes -C(O)- the alkyl, - C(O)- the cycloalkyl, and -C(O)- the aralkyl, and the aroyl group includes -C(O)- the aryl and -C(O)- the heteroaryl. Examples of the C₁₋₁₂ acyl group include a formyl group; a C₂₋₆ alkanoyl group such as an acetyl group, a propionyl group, a butanoyl group, a pentanoyl group, or a hexanoyl group; a C₄₋₇ cycloalkanecarbonyl group such as a cyclopropanecarbonyl group, a cyclobutanecarbonyl group, or a cyclopentanecarbonyl group; an aroyl such as a benzoyl group or a naphthoyl group; and a 5- or 6-membered heteroaroyl group such as a furoyl group, a thiophenecarbonyl group, a nicotinoyl group, or an isonicotinoyl group.

The term "C₇₋₁₂ aralkyl group" refers to a group having a total of 7 to 12 carbon atoms in which the alkyl group is substituted with an aryl group or a heteroaryl group. Examples of the aralkyl group include a benzyl group, a phenethyl group, a phenylpropyl group, and a 2-pyridylmethyl group.

The term "C₃₋₁₀ cycloalkyl C₁₋₆ alkyl group" refers to an alkyl group in which one of hydrogen atoms of the alkyl group is substituted with a C₃₋₁₀ cycloalkyl group. Examples of the C₃₋₁₀ cycloalkyl C₁₋₆ alkyl group include a cyclopropylmethyl group, a cyclopropylethyl group, a cyclopropylpropyl group, a cyclobutylmethyl group, a cyclobutylethyl group, a cyclobutylpropyl group, a cyclopentylmethyl group, a cyclopentylethyl group, a cyclopentylpropyl group, a cyclohexylmethyl group, a cyclohexylethyl group, and a cyclohexylpropyl group.

The term "hydroxy protecting group" refers to a protecting group introduced to inactivate highly reactive hydroxy. Examples of the hydroxy protecting group include an alkyl group which may have a substituent such as a methyl group, a methoxymethyl group, or an ethoxyethyl group, an aralkyl group which may have a substituent such as a benzyl group, a 4-methoxybenzyl group, or a trityl group, an alkanoyl such as an acetyl group, and a silyl group having a substituent such as a trimethylsilyl group or a tert-butyldimethylsilyl group, but are not limited thereto. For example, a protecting group described in Green's Protective Groups in Organic Synthesis, 5th ed. and the like can be used. In a case where R² is a hydroxy protecting group, a compound in which R² is the hydroxy protecting group can also be used as a synthetic intermediate of a compound in which R² is a hydrogen atom.

The term "halo C₁₋₆ alkyl group" refers to an alkyl group substituted with one or more halogen atoms. Examples of the haloalkyl group include a fluoromethyl group, a difluoromethyl group, a trifluoromethyl group, a chloromethyl group, a dichloromethyl group, and a trichloromethyl group.

The term "nitrogen-containing 5- or 6-membered heteroaryl group" refers to a 5- or 6-membered heterocyclic aromatic cyclic group containing at least one N as a ring constituent atom. Examples of the nitrogen-containing 5-membered heteroaryl group include a furanyl group, a thienyl group, a pyrrolyl group, an oxazolyl group, a thiazolyl group, a triazolyl group, and tetrazolyl group, examples of the nitrogen-containing 6-membered heteroaryl group include a pyridyl group, a pyridadinyl group, a pyrimidinyl group, a pyrazinyl group, a triazinyl group, and a tetrazinyl group.

The term "substituent" of "which may have a substituent" refers to an atom or an atomic group substituted with hydrogen in a derivative in which a hydrogen atom of an organic compound is substituted with another atom or atomic group.

In a case where the substituent is substituted to an acyclic moiety, examples of the substituent include a halogen atom, a hydroxy group, a cyano group, a nitro group, a C₁₋₆ alkoxy group, a carboxy group, an alkoxycarbonyl group, an aminocarbonyl group, a sulfo group, an alkylsulfonyl group, an aminosulfonyl group, an amino group, a mono or dialkylamino group, a carbonyl amino group, an acyl group, an acyloxy group, and an oxo group. In addition, in a case where the substituent is substituted to a cyclic moiety, examples of the substituent include a C₁₋₆ alkyl group and a C₁₋₆ alkenyl group in addition to the substituents described above. The number of substituents and substitution position are not limited, but in a case where two or more substituents are present, these substituents may be the same as or different from each other.

The term "stereoisomer" refers to a molecule in which compounds having the same bonding relationship between atoms represented by the same molecular formula have different relationships only in spatial arrangement. The stereoisomer includes a cis-trans isomer, an E-Z isomer, an enantiomer, and a diastereomer, and these stereoisomers may be a mixture of them.

The term "pharmaceutically acceptable salt" refers to an acid or base addition salt of the compound that maintains the biological efficacy and properties of the compound of the present invention and can be produced from non-toxic inorganic acid and inorganic base and organic acid and organic base.

The acid addition salt can be produced from, for example, inorganic acids such as hydrochloric acid, hydrogen bromide, sulfuric acid, and phosphoric acid, and similarly, for example, organic acids such as acetic acid, maleic acid, fumaric acid, toluenesulfonic acid, methanesulfonic acid, tartaric acid, succinic acid, citric acid, and malic acid.

The base addition salt can be produced from, for example, inorganic bases such as sodium, potassium, ammonium calcium, magnesium, zinc, and aluminum, and similarly, for example, organic bases such as meglumine, diethylamine, ethanolamine, trimethamine, lysine, and arginine.

The term "solvate" refers to an association or complex of a compound (including a salt form) and a solvent molecule. Examples of a solvent that forms a solvate include water, methanol, ethanol, 2-propanol, dimethyl sulfoxide, tetrahydrofuran, acetic acid, ethyl acetate, and diethyl ether, but are not limited thereto.

The term "pharmaceutical composition" refers to a composition containing a compound of the present invention, a stereoisomer of the compound, a pharmaceutically acceptable salt thereof, or a solvate thereof, and a pharmaceutically acceptable additive in combination. Examples of the pharmaceutically acceptable additive include an excipient, a binder, a disintegrant, a fluidizer, a lubricant, a coating agent, a colorant, a flavoring agent (a sweetener, an acidulant, a flavor, or the like), an isotonic agent, a buffering agent, a surfactant, a stabilizer, a preservative, a pH regulator, and an antioxidant. In addition, the pharmaceutically acceptable additive can be used in combination with other analgesics, anxiolytics, antidepressants, and therapeutic agents for dysuria.

Examples of an administration form of the pharmaceutical composition include, but are not limited to, oral agents such as a tablet, a capsule, a granule, a powder, a pill, a troch, buccal, sublingual, a syrup, and a liquid, and parenteral agents such as an injection, a liquid, an aerosol, a suppository, a patch, a poultice, a lotion, a liniment, an ointment, an eye drop, and a nasal drop.

In an adult, usually, a compound represented by general formula (I), a stereoisomer of the compound, a pharmaceutically acceptable salt thereof, or a solvate thereof as an active ingredient is administered once or in several divided doses as a dosage of 0.1 µg to 1 g/day and preferably 0.001 to 200 mg/day in a case of an injection, and 1 µg to 10 g/day and preferably 0.01 to 2,000 mg/day in a case of an oral agent. However, the dosage can be increased or decreased depending on age, symptom, preparation, or the like.

The term "opioid δ receptor agonist" refers to a pharmaceutical composition containing, as an active ingredient, a compound that enhances the activity thereof by binding to the opioid δ receptor. A compound that binds to a receptor and is only partially effective as an agonist is defined as a "partial agonist". In the present invention, examples of diseases with which the opioid δ receptor is involved include neurodegenerative diseases such as pain, anxiety, depression, Parkinson's disease, and Alzheimer's disease, ischemia, stroke, dysuria, HIV infection, alcoholism, and diabetes.

Next, a method for producing a pyrazolomorphinan derivative represented by general formula (I), a stereoisomer of the compound, a pharmaceutically acceptable salt thereof, or a solvate thereof will be described below.
(i) Production method in a case where 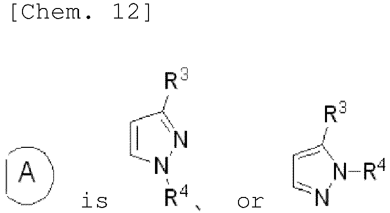 in general formula (I) (where excluding a case where R¹ is hydrogen and R³ is a hydroxy group.)

### (Method A) Method using hydrazine represented by R⁴NHNHR^{4a}

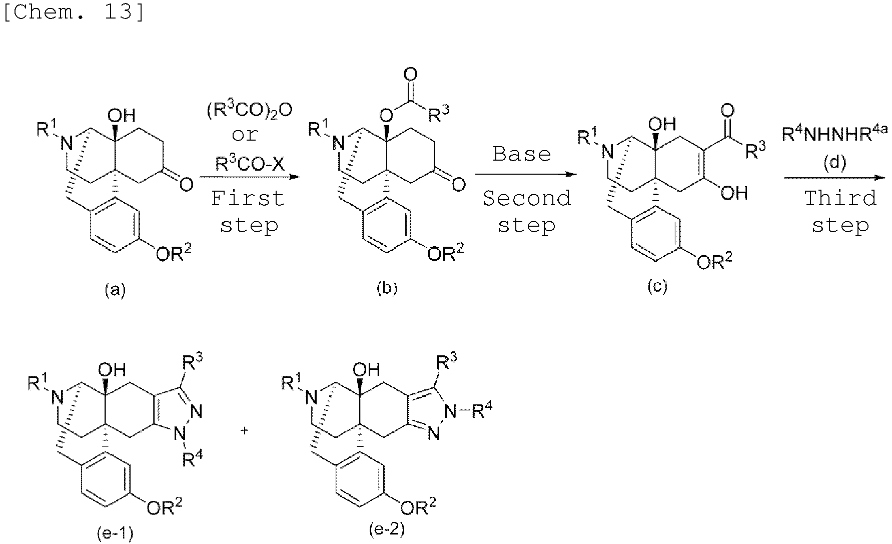

(in the formula, X is a halogen atom, R^{4a} represents a hydrogen atom or a Boc group, and R¹ to R⁴ are the same as described above, where excluding a case where R¹ is hydrogen and R³ is a hydroxy group.)

### (First step)

A compound (b) can be synthesized by reacting a raw material (a) with an acid anhydride represented by (R³CO)₂O or an acid halide represented by R³CO-X at room temperature to heating under reflux for 1 to 24 hours in aromatic hydrocarbons such as benzene, toluene, and xylene, ethers such as diethyl ether, tetrahydrofuran, dioxane, monoglyme, and diglyme, halogenated hydrocarbons such as dichloromethane, chloroform, carbon tetrachloride, 1,2-dichloroethane, and 1,1,2,2-tetrachloroethane, aliphatic hydrocarbons such as pentane, hexane, heptane, and ligroin, aprotonic polar solvents such as acetonitrile, N,N-dimethylformamide, N-methylpyrrolidone, and dimethyl sulfoxide or in no solvent in the presence or absence of bases such as potassium bis(trimethylsilyl)amide (KHMDS) and lithium diisopropylamide (LDA), organic bases such as trimethylamine, triethylamine, tributylamine, N,N-diisopropylethylamine, pyridine, N,N-dimethylaniline, N,N-dimethylaminopyridine, N-methylpiperidine, N-methylmorpholine, sodium methoxide, sodium ethoxide, and potassium tert-butoxide, and inorganic bases such as lithium carbonate, sodium carbonate, potassium carbonate, sodium hydroxide, potassium hydroxide, sodium acetate, sodium hydride, and potassium hydride.

### (Second step)

A compound (c) can be synthesized by reacting a compound (b) at -78°C to room temperature for 1 to 24 hours in aromatic hydrocarbons such as benzene, toluene, and xylene, ethers such as diethyl ether, tetrahydrofuran, dioxane, monoglyme, and diglyme, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, solvents such as water, or a mixed solvent thereof in the presence of bases such as potassium bis(trimethylsilyl)amide (KHMDS) and lithium diisopropylamide (LDA), organic bases such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, and potassium tert-butoxide, and inorganic bases such as lithium carbonate, sodium carbonate, potassium carbonate, sodium hydroxide, potassium hydroxide, sodium hydride, and potassium hydride.

### (Third step)

Each of inventive compounds (e-1) and (e-2) can be synthesized by reacting the compound (c) with hydrazines (d) represented by R⁴NHNHR^{4a} or hydrochlorides, hydrobromides, or sulfates thereof at room temperature to heating under reflux for 1 to 72 hours in aromatic hydrocarbons such as benzene, toluene, and xylene, ethers such as diethyl ether, tetrahydrofuran, dioxane, monoglyme, and diglyme, halogenated hydrocarbons such as dichloromethane, chloroform, carbon tetrachloride, 1,2-dichloroethane, and 1,1,2,2-tetrachloroethane, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, or solvents such as acetic acid and water in the presence or absence of bronsted acids such as acetic acid, trifluoroacetic acid, methanesulfonic acid, and p-toluenesulfonic acid, and Lewis acids such as a boron trifluoride-diethyl ether complex, aluminum chloride, iron chloride, zinc chloride, and titanium(IV) chloride.

### (Method B)

In a case where R⁴ is a C₁₋₆ alkyl group which may have a substituent, a C₁₋₁₂ acyl group which may have a substituent, a C₇₋₁₂ aralkyl group which may have a substituent, a C₃₋₁₀ cycloalkyl group which may have a substituent, a C₃₋₁₀ cycloalkyl C₁₋₆ alkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent, synthesis can be performed by the following method. 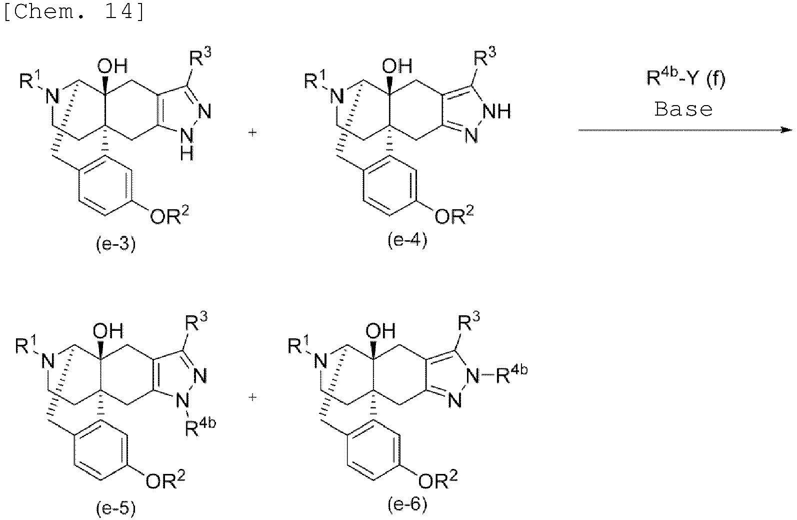 (in the formula, R^{4b} represents a C₁₋₆ alkyl group which may have a substituent, a C₁₋₁₂ acyl group which may have a substituent, a C₇₋₁₂ aralkyl group which may have a substituent, a C₃₋₁₀ cycloalkyl group which may have a substituent, a C₃₋₁₀ cycloalkyl C₁₋₆ alkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent, Y represents a leaving group such as a halogen atom, methanesulfonate, p-toluenesulfonate, or triflate, and R¹ to R³ are the same as described above, where excluding a case where R¹ is hydrogen and R³ is a hydroxy group.)

Each of inventive compounds (e-5) and (e-6) can be synthesized by reacting an equilibrium mixture of inventive compounds (e-3) and (e-4) in which R⁴ is hydrogen with a reagent (f) represented by R^{4b}-Y at 0°C to heating under flux for 1 to 24 hours in aromatic hydrocarbons such as benzene, toluene, and xylene, ethers such as diethyl ether, tetrahydrofuran, dioxane, monoglyme, and diglyme, halogenated hydrocarbons such as dichloromethane, chloroform, and carbon tetrachloride, aliphatic hydrocarbons such as pentane, hexane, heptane, and ligroin, aprotonic polar solvents such as acetonitrile, N,N-dimethylformamide, N-methylpyrrolidone, and dimethyl sulfoxide, solvents such as water, or a mixed solvent thereof in the presence of bases such as potassium bis(trimethylsilyl)amide (KHMDS) and lithium diisopropylamide (LDA), organic bases such as trimethylamine, triethylamine, tributylamine, N,N-diisopropylethylamine, pyridine, N,N-dimethylaniline, N,N-dimethylaminopyridine, N-methylpiperidine, N-methylmorpholine, and procaine, and inorganic bases lithium carbonate, sodium carbonate, potassium carbonate, sodium hydride, potassium hydride, and silver oxide.

### (Method C)

In a case where R⁴ is a C₆₋₁₀ aryl group which may have a substituent or a 5- to 10-membered heteroaryl group which may have a substituent, synthesis can be performed by the following method. 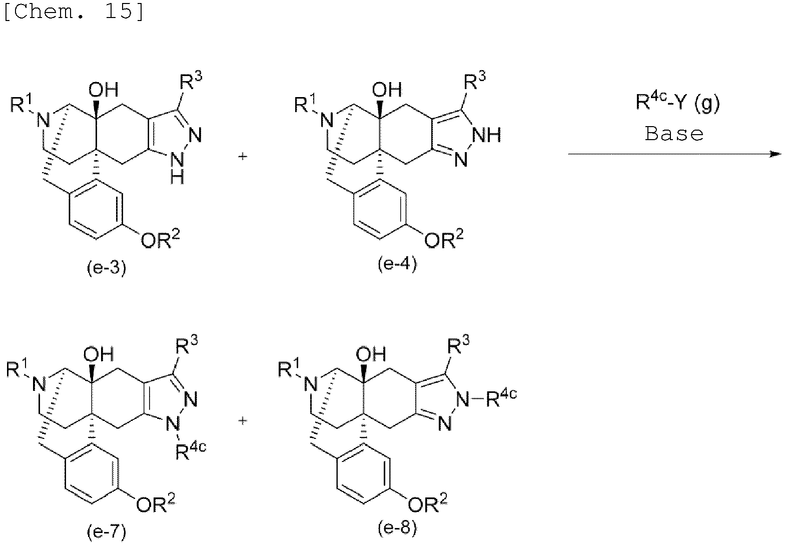 (in the formula, R^{4c} represents a C₆₋₁₀ aryl group which may have a substituent or a 5- to 10-membered heteroaryl group which may have a substituent, Y represents boronic acid, catecholborane, pinacolborane, trifluoroborate potassium salt, a halogen atom, triflate, or the like, and R¹ to R³ are the same as described above, where excluding a case where R¹ is hydrogen and R³ is a hydroxy group.)

Each of inventive compounds (e-7) and (e-8) can be synthesized by reacting an equilibrium mixture of the inventive compounds (e-3) and (e-4) in which R⁴ is hydrogen with a reagent (g) represented by R^{4c}-Y at room temperature to heating under flux for 1 to 72 hours in aromatic hydrocarbons such as benzene, toluene, and xylene, ethers such as diethyl ether, tetrahydrofuran, dioxane, monoglyme, and diglyme, halogenated hydrocarbons such as dichloromethane, chloroform, and carbon tetrachloride, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, solvents such as pyridine and water, or a mixed solvent thereof in the presence of copper catalysts such as copper powder and copper(I) iodide, palladium catalysts such as palladium(II) acetate, tris(dibenzylideneacetone)dipalladium(0), tetrakis(triphenylphosphine)palladium(0), and bis(triphenylphosphine)palladium(II) dichloride, ligands such as N,N'-dimethylethylenediamine, triphenylphosphine, 4,5-bis(diphenylphosphino)-9,9-dimethylxanthene, and 2-(dicyclohexylphosphino)-2',4',6'-triisopropyl-1,1'-biphenyl, organic bases such as sodium methoxide, sodium ethoxide, and potassium tert-butoxide, and inorganic bases such as lithium carbonate, sodium carbonate, potassium carbonate, sodium acetate, potassium acetate, and cesium carbonate.

(ii) Production method in a case where 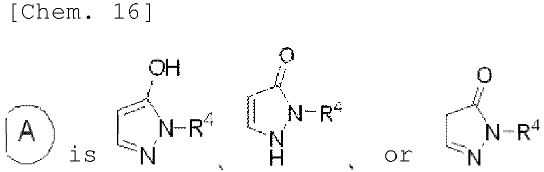 in general formula (I) (where excluding a case where R¹ is hydrogen.) 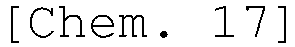 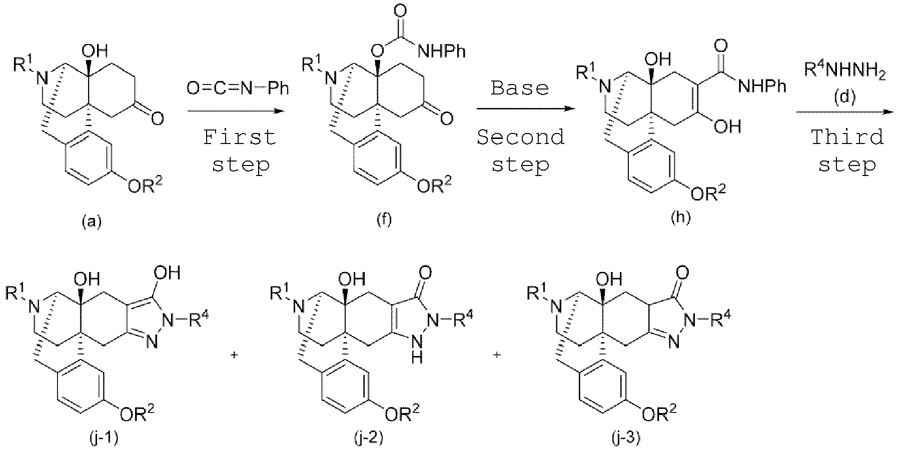 (in the formula, R¹, R², and R⁴ are the same as described above, where excluding a case where R¹ is hydrogen.)

### (First step)

A compound (f) can be synthesized by reacting the compound (a) with phenyl isocyanate at 0°C to heating under reflux for 1 to 24 hours in aromatic hydrocarbons such as benzene, toluene, and xylene, ethers such as diethyl ether, tetrahydrofuran, dioxane, monoglyme, and diglyme, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, esters such as methyl acetate and ethyl acetate, solvents such as water, or a mixed solvent thereof in the presence of copper(II) chloride, pyridine, triethylamine, sodium acetate, trifluoroacetic acid, a boron trifluoride-diethyl ether complex, hydrogen chloride, aluminum chloride, and lithium ethoxide.

### (Second step)

A compound (h) can be synthesized by reacting the compound (f) at 0°C to heating under reflux for 1 to 24 hours in aromatic hydrocarbons such as benzene, toluene, and xylene, ethers such as diethyl ether, tetrahydrofuran, dioxane, monoglyme, and diglyme, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, solvents such as water, or a mixed solvent thereof in the presence of bases such as potassium bis(trimethylsilyl)amide (KHMDS) and lithium diisopropylamide (LDA), organic bases such as trimethylamine, triethylamine, tributylamine, N,N-diisopropylethylamine, pyridine, N,N-dimethylaniline, N,N-dimethylaminopyridine, N-methylpiperidine, N-methylmorpholine, diethylamine, cyclohexylamine, procaine, sodium methoxide, sodium ethoxide, and potassium tert-butoxide, and inorganic bases such as lithium carbonate, sodium carbonate, potassium carbonate, sodium hydroxide, potassium hydroxide, sodium hydride, and potassium hydride.

### (Third step)

Each of inventive compounds (j-1), (j-2), and (j-3) can be synthesized by reacting the compound (h) with hydrazines (d) represented by R⁴NHNH₂ or hydrochlorides, hydrobromides, or sulfates thereof at room temperature to heating under reflux for 1 to 72 hours in aromatic hydrocarbons such as benzene, toluene, and xylene, ethers such as diethyl ether, tetrahydrofuran, dioxane, monoglyme, and diglyme, halogenated hydrocarbons such as dichloromethane, chloroform, and carbon tetrachloride, aliphatic hydrocarbons such as pentane, hexane, heptane, and ligroin, alcohols such as methanol, ethanol, and 2-propanol, aprotonic polar solvents such as acetonitrile, N,N-dimethylformamide, N-methylpyrrolidone, and dimethyl sulfoxide, or solvents such as acetic acid and water in the presence or absence of bronsted acids such as acetic acid, trifluoroacetic acid, methanesulfonic acid, and p-toluenesulfonic acid, and Lewis acids such as a boron trifluoride-diethyl ether complex, aluminum chloride, iron chloride, zinc chloride, and titanium(IV) chloride.

(iii) Production method in a case where R² is a hydrogen atom in general formula (I)

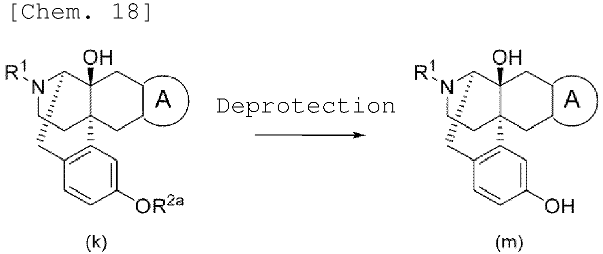

(in the formula, R^{2a} represents a hydroxy protecting group, and a cyclic structure represented by A and R¹ are the same as described above, where excluding a case where R¹ is hydrogen.)

The hydroxy protecting group of the inventive compound (k) obtained in (i) and (ii) above can be deprotected based on a known method (for example, a method described in Green's Protective Groups in Organic Synthesis, 5th ed.) and an inventive compound (m) can be synthesized.

(iv) Production method in a case where R¹ is a hydrogen atom in general formula (I) 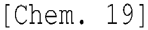 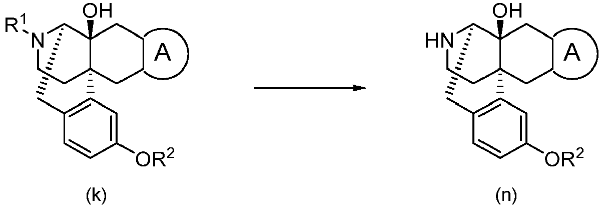 (in the formula, a cyclic structure represented by A, and R¹ and R² are the same as described above, where excluding a case where R¹ is hydrogen.)

In a case where R¹ of the inventive compound (k) obtained in (i) and (ii) above is not hydrogen, the compound can be converted to a compound in which R¹ is hydrogen, based on a known method (for example, a method described in Bioorg. Med. Chem. Lett., 2010, 20, 6302-6305 or WO 2013/035833 A).

### Examples

Next, the present invention will be described in more detail with reference to Reference Examples and Examples, but the present invention is not limited thereto.

Compounds in Examples and compounds in Reference Examples were named by converting a structural formula drawn using ChemDraw ver. 14 manufactured by Cambridge Software Corporation as an English name by a naming algorithm mounted on the software and then translating the English name into Japanese.

### Reference Example 1

### Synthesis of (4bR,8aS,9R)-11-(cyclopropylmethyl)-3-methoxy-6-oxo-5,6,7,8,9,10-hexahydro-8aH-9,4b-(epiminoethano)phenanthren-8a-yl acetate (Compound 1)

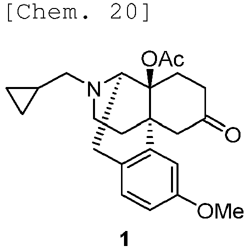

(4bR,8aS,9R)-11-(cyclopropylmethyl)-8a-hydroxy-3-methoxy-8,8a,9,10-tetrahydro-5H-9,4b-(epiminoethano)phenanthren-6(7H)-one (Compound 2) (synthesized by a method described in Chemical Biology & Drug Design 2009, 74, 335-342) (1.1 g, 3.2 mmol) was dissolved in acetic anhydride (5 mL), and stirring was performed at 85°C for 6.5 hours. After the reaction solution was concentrated under reduced pressure, a saturated sodium bicarbonate aqueous solution was added thereto, and extraction was performed with chloroform. After combined organic layers were washed with saturated brine and dried with anhydrous sodium sulfate, concentration was performed under reduced pressure. The obtained crude product was purified by silica gel column chromatography, and thus, the title compound 1 (1.16 g, 97%) as yellow-white amorphous was obtained.

¹H-NMR (400 MHz, CDCl₃) δ (ppm): 0.03 - 0.13 (m, 2H), 0.43 - 0.54 (m, 2H), 0.75 - 0.83 (m, 1H), 1.12 - 1.18 (m, 1H), 1.51 - 1.56 (m, 1H), 1.87 (ddd, J = 5.3, 14.0, 14.0 Hz, 1H), 2.08 (ddd, J = 3.3, 12.2, 12.2 Hz, 1H), 2.12 - 2.19 (m, 1H), 2.21 (s, 3H), 2.28 - 2.48 (m, 3H), 2.59 (ddd, J = 1.3, 5.0, 11.8 Hz, 1H), 2.66 (dd, J = 6.1, 18.5 Hz, 1H), 2.83 (ddd, J = 1.7, 6.8, 14.2 Hz, 1H), 2.90 (dd, J = 1.8, 14.8 Hz, 1H), 2, 96 (d, J = 14.8 Hz, 1H), 3.08 (d, J = 18.5 Hz, 1H), 3.77 (s, 3H), 4.37 (d, J = 6.1 Hz, 1H), 6.70 (dd, J = 2.6, 8.4 Hz, 1H), 6.79 (d, J = 2.6 Hz, 1H), 7.00 (d, J = 8.4 Hz, 1H).

### Reference Example 2

### Synthesis of 1-((4bR,8aS,9R)-11-(cyclopropylmethyl)-6,8a-dihydroxy-3-methoxy-8,8a,9,10-tetrahydro-5H-9,4b-(epiminoethano)phenanthren-7-yl)ethan-1-one (Compound 3)

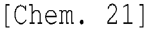

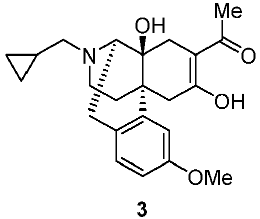

Under an argon atmosphere, a 1.5 M hexane solution of n-butyllithium (3.5 mL, 8.25 mmol) was added dropwise to a tetrahydrofuran (10.0 mL) solution of diisopropylamine (1.1 mL, 7.72 mmol) at -78°C, and stirring was performed at -78°C for 35 minutes. The temperature was raised to 0°C and stirring was performed for 50 minutes, and then cooling was performed to -78°C. A tetrahydrofuran (9.0 mL) solution of the compound 1 (2.3 g, 6.1 mmol) was added dropwise, and stirring was performed for 21.5 hours while slowly raising the temperature to room temperature. A saturated sodium bicarbonate aqueous solution was added to the reaction solution, and extraction was performed with ethyl acetate. After combined organic layers were washed with saturated brine and dried with anhydrous sodium sulfate, concentration was performed. The obtained crude product was purified by silica gel column chromatography and preparative thin layer chromatography, and thus, the title compound 3 (1.6 g, 69%) as yellow-white amorphous was obtained.

¹H-NMR (400 MHz, CDCl₃) δ (ppm): 0.10 - 0.19 (m, 2H), 0.50 - 0.60 (m, 2H), 0.82 - 0.94 (m, 1H), 1.26 (d, J = 12.4 Hz, 1H), 1.96 (s, 3H), 2.08 - 2.19 (m, 2H), 2.28 (d, J = 15.5 Hz, 1H), 2.36 - 2.43 (m, 3H), 2.58 - 2.64 (m, 1H), 2.84 (d, J = 18.0 Hz, 1H), 2.92 (dd, J = 6.9, 18.6 Hz, 1H), 2.94 (d, J = 18.0 Hz, d, 1H), 3.11 (d, J = 18.6 Hz, 1H), 3.19 (d, J = 6.9 Hz, 1H), 3.76 (s, 3H), 6.71 (dd, J = 2.6, 8.4 Hz, 1H), 6.78 (d, J = 2.6 Hz, 1H), 7.02 (d, J = 8.4 Hz, 1H), 15.9 (s, 1H), 1H (OH) was not observed.

### Example 1

### Synthesis of (6R,6aS,11aR)-14-(cyclopropylmethyl)-2-methoxy-8-methyl-9-phenyl-5,6,9,11-tetrahydro-6,11a-(epiminoethano)naphtho[2,1-f]indazol-6a(7H)-ol (Compound 4) and (6R,6aS,11aR)-14-(cyclopropylmethyl)-2-methoxy-8-methyl-10-phenyl-5,6,10,11-tetrahydro-6,11a-(epiminoethano)naphtho[2,1-f]indazol-6a(7H)-ol (Compound 5)

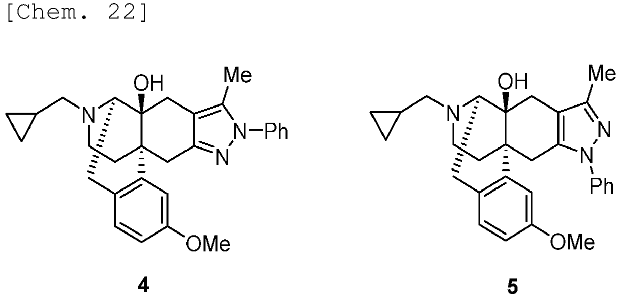

Under an argon atmosphere, phenylhydrazine hydrochloride (355.8 mg, 2.46 mmol) and methanesulfonic acid (283 µL, 4.36 mmol) were added to an ethanol (15 mL) solution of the compound 3 (699.8 mg, 1.83 mmol), and heating and reflux were performed for 2.5 hours. After the reaction solution was concentrated under reduced pressure, a saturated sodium bicarbonate aqueous solution was added thereto, and extraction was performed with ethyl acetate. After combined organic layers were washed with saturated brine and dried with anhydrous sodium sulfate, concentration was performed under reduced pressure. The obtained crude product was purified by silica gel column chromatography, and thus, the title compound 4 (97.4 mg, 11.7%) as yellow-white amorphous and the title compound 5 (620.8 mg, 74.7%) as yellow-white amorphous were obtained.

### Compound 4

¹H-NMR (400 MHz, CDCl₃) δ (ppm): 0.10 - 0.20 (m, 2H), 0.51 - 0.61 (m, 2H), 0.84 - 0.96 (m, 1H), 1.27 - 1.34 (m, 1H), 2.09 (s, 3H), 2.11 - 2.26 (m, 2H), 2.42 (d, J = 6.5 Hz, 2H), 2.50 (d, J = 16.6 Hz, 1H), 2.51 (d, J = 16.6 Hz, 1H), 2.62 - 2.68 (m, 1H), 2.96 (dd, J = 6.7, 18.5 Hz, 1H), 3.12 (br d, J = 17.8 Hz, 2H), 3.25 (d, J = 16.1 Hz, 1H), 3.27 (d, J = 6.7 Hz, 1H), 3.56 (s, 3H), 6.46 (d, J = 2.6 Hz, 1H), 6.63 (dd, J = 2.6, 8.4 Hz, 1H), 6.98 (d, J = 8.4 Hz, 1H), 7.28 - 7.34 (m, 1H), 7.43 - 7.54 (m, 4H), 1H (OH) was not observed.

### Compound 5

¹H-NMR (400 MHz, CDCl₃) δ (ppm): 0.11 - 0.20 (m, 2H), 0.51 - 0.60 (m, 2H), 0.84 - 0.96 (m, 1H), 1.34 - 1.40 (m, 1H), 2.09 (s, 3H), 2.14 - 2.28 (m, 2H), 2.42 (d, J = 6.5 Hz, 2H), 2.48 (d, J = 15.9 Hz, 1H), 2.56 (d, J = 15.9 Hz, 1H), 2.64 - 2.70 (m, 1H), 2.96 (dd, J = 6.8, 18.5 Hz, 1H), 3.07 (d, J = 16.5 Hz, 1H), 3.15 (d, J = 18.5 Hz, 1H), 2.24 (d, J = 6.8 Hz, 1H), 3.39 (d, 16.5 Hz, 1H), 3.71 (s, 3H), 6.68 (dd, J = 2.6, 8.4 Hz, 1H), 6.90 (d, J = 2.6 Hz, 1H), 7.00 (d, J = 8.4 Hz, 1H), 7.26 - 7.31 (m, 1H), 7.35 - 7.42 (m, 4H), 1H (OH) was not observed.

### Example 2

### Synthesis of (6R,6aS,11aR)-14-(cyclopropylmethyl)-9-isopropyl-2-methoxy-8-methyl-5,6,9,11-tetrahydro-6,11a-(epiminoethano)naphtho[2,1-f]indazol-6a(7H)-ol (Compound 6) and (6R,6aS,11aR)-14-(cyclopropylmethyl)-10-isopropyl-2-methoxy-8-methyl-5,6,10,11-tetrahydro-6,11a-(epiminoethano)naphtho[2,1-f]indazol-6a(7H)-ol (Compound 7)

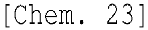

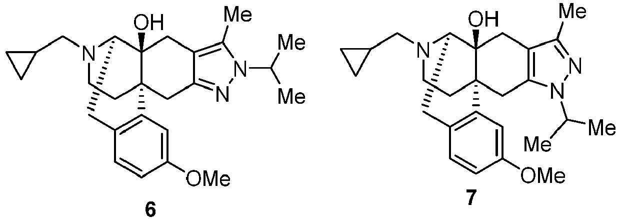

### Method 1

A reaction was carried out using the compound 3 (115.7 mg, 0.30 mmol), isopropylhydrazine (50.9 mg, 0.69 mmol), and methanesulfonic acid (47 µL, 0.72 mmol) in the same manner as that of Example 1, and thus, the title compound 6 (13.7 mg, 11%) as white oil and the title compound 7 (110.0 mg, 84%) as white oil were obtained.

### Method 2

Under an argon atmosphere, silver(I) oxide (489.7 mg, 2.11 mmol) and iodine isopropyl (0.21 mL, 2.11 mmol) were added to a toluene (10 mL) solution of an equilibrium mixture (401.0 mg, 1.06 mmol) of the compound 18 and the compound 19 at room temperature, and heating and reflux were performed for 2 hours. After cooling, the reaction solution was filtered through celite, and the filtrate was concentrated under reduced pressure. The obtained crude product was purified by silica gel column chromatography, and thus, the title compound 6 (121.4 mg, 27%) as colorless oil and the title compound 7 (13.0 mg, 9.9%) as colorless oil were obtained.

### Compound 6

¹H-NMR (400 MHz, CDCl₃) δ (ppm): 0.09 - 0.20 (m, 2H), 0.48 - 0.58 (m, 2H), 0.84 - 0.95 (m, 1H), 1.29 - 1.34 (m, 1H), 1.37 (d, J = 6.7 Hz, 3H), 1.39 (d, J = 6.7 Hz, 3H), 1.98 (s, 3H), 2.12 - 2.26 (m, 2H), 2.35 - 2.50 (m, 2H), 2.41 (d, J = 6.6 Hz, 2H), 2.61 - 2.68 (m, 1H), 2.92 (dd, J = 6.3, 18.4 Hz, 1H), 2.97 (d, J = 16.2 Hz, 1H), 3.12 (d, J = 18.4 Hz, 1H), 3.20 (d, J = 6.3 Hz, 1H), 3.31 (d, J = 16.2 Hz, 1H), 3.69 (s, 3H), 4.24 (sept, 1H), 6.65 (dd, J = 2.6, 8.4 Hz, 1H), 6.68 (d, J = 2.6 Hz, 1H), 6.97 (d, J = 8.4 Hz, 1H), 1H (OH) was not observed.

### Compound 7

¹H-NMR (400 MHz, CDCl₃) δ (ppm): 0.08 - 0.19 (m, 2H), 0.49 - 0.60 (m, 2H), 0.82 - 0.94 (m, 1H), 1.29 - 1.36 (m, 1H), 1.48 (d, J = 6.7 Hz, 3H), 1.50 (d, J = 6.7 Hz, 3H), 2.02 (s, 3H), 2.11 - 2.25 (m, 2H), 2.35 - 2.49 (m, 2H), 2.41 (d, J = 6.4 Hz, 2H), 2.62 - 2.68 (m, 1H), 2.89 (d, J = 16.2 Hz, 1H), 2.95 (dd, J = 6.6, 18.6 Hz, 1H), 3.10 (d, J = 18.6 Hz, 1H), 3.20 (d, J = 16.2 Hz, 1H), 3.21 (d, J = 6.6 Hz, 1H), 3.69 (s, 3H), 4.44 (sept, 1H), 6.65 (dd, J = 2.6, 8.3 Hz, 1H), 6.68 (d, J = 2.6 Hz, 1H), 6.99 (d, J = 8.3 Hz, 1H), 1H (OH) was not observed.

### Example 3

### Synthesis of (6R,6aS,11aR)-9-(tert-butyl)-14-(cyclopropylmethyl)-2-methoxy-8-methyl-5,6,9,11-tetrahydro-6,11a-(epiminoethano)naphtho[2,1-f]indazol-6a(7H)-ol (Compound 8) and (6R,6aS,11aR)-10-(tert-butyl)-14-(cyclopropylmethyl)-2-methoxy-8-methyl-5,6,10,11-tetrahydro-6,11a-(epiminoethano)naphtho[2,1-f]indazol-6a(7H)-ol (Compound 9)

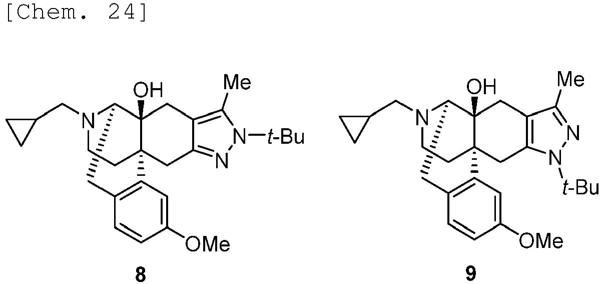

### Method 1

A reaction was carried out using the compound 3 (96.1 mg, 0.25 mmol), t-butylhydrazine hydrochloride (38.5 mg, 0.31 mmol), and methanesulfonic acid (39 µL, 0.60 mmol) in the same manner as that of Example 1, and thus, the title compound 8 (6.8 mg, 6%) as yellow oil and the title compound 9 (88.1 mg, 81%) as yellow oil were obtained.

### Method 2

A reaction was carried out using an equilibrium mixture (57.8 mg, 0.15 mmol) of the compound 18 and the compound 19, silver(I) oxide (38.8 mg, 0.17 mmol), and 2-bromo-2-methylpropane (26 µL, 0.23 mmol) in the same manner as that of Method 2 described in Example 2, and thus, the title compound 9 (8.0 mg, 12%) as colorless oil was obtained.

### Compound 8

¹H-NMR (400 MHz, CDCl₃) δ (ppm): 0.06 - 0.18 (m, 2H), 0.48 - 0.59 (m, 2H), 0.82 - 0.95 (m, 1H), 1.22 - 1.34 (m, 1H), 1.54 (s, 9H), 2.13 (s, 3H), 2.14 - 2.25 (m, 2H), 2.32 - 2.48 (m, 2H), 2.41 (d, J = 6.6 Hz, 2H), 2.59 - 2.68 (m, 1H), 2.92 (dd, J = 6.8, 18.4 Hz, 1H), 2.95 (d, J = 16.3 Hz, 1H), 3.12 (d, J = 18.4 Hz, 1H), 3.20 (d, J = 6.8 Hz, 1H), 3.30 (d, J = 16.3 Hz, 1H), 3.71 (s, 3H), 6.65 (dd, J = 2.6, 8.4 Hz, 1H), 6.87 (d, J = 2.4 Hz, 1H), 6.99 (d, J = 8.4 Hz, 1H), 1H (OH) was not observed.

### Compound 9

¹H-NMR (400 MHz, CDCl₃) δ (ppm): 0.09 - 0.18 (m, 2H), 0.49 - 0.60 (m, 2H), 0.82 - 0.94 (m, 1H), 1.28 - 1.36 (m, 1H), 1.68 (s, 9H), 1.99 (s, 3H), 2.10 - 2.24 (m, 2H), 2.34 - 2.49 (m, 2H), 2.40 (d, J = 6.5 Hz, 2H), 2.61 - 2.68 (m, 1H), 2.94 (dd, J = 6.8, 18.5 Hz, 1H), 3.02 - 3.13 (m, 2H), 3.20 (d, J = 6.6 Hz, 1H), 3.47 (d, J = 16.1 Hz, 1H), 3.68 (s, 3H), 6.65 (dd, J = 2.6, 8.4 Hz, 1H), 6.87 (d, J = 2.6 Hz, 1H), 6.98 (d, J = 8.4 Hz, 1H), 1H (OH) was not observed.

### Example 4

### Synthesis of (6R,6aS,11aR)-10-benzyl-14-(cyclopropylmethyl)-2-methoxy-8-methyl-5,6,10,11-tetrahydro-6,11a-(epiminoethano)naphtho[2,1-f]indazol-6a(7H)-ol (Compound 10) and (6R,6aS,11aR)-9-benzyl-14-(cyclopropylmethyl)-2-methoxy-8-methyl-5,6,9,11-tetrahydro-6,11a-(epiminoethano)naphtho[2,1-f]indazol-6a(7H)-ol (Compound 10a)

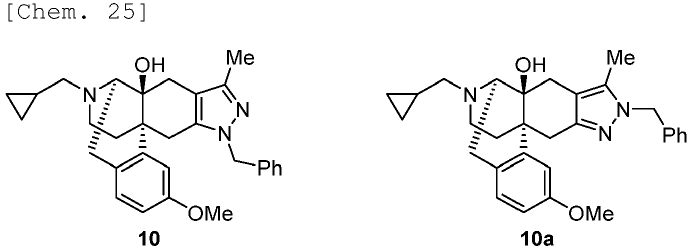

A reaction was carried out using the compound 3 (73.6 mg, 0.19 mmol), benzylhydrazine hydrochloride (45.7 mg, 0.29 mmol), and methanesulfonic acid (25 µL, 0.38 mmol) in the same manner as that of Example 1, and thus, the title compound 10 (85.1 mg, 94%) as yellow amorphous was obtained. The production of the position isomer 10a was also confirmed, but could not be isolated.

### Compound 10

¹H-NMR (400 MHz, CDCl₃) δ (ppm): 0.06 - 0.18 (m, 2H), 0.48 - 0.58 (m, 2H), 0.81 - 0.93 (m, 1H), 1.22 - 1.28 (m, 1H), 2.05 (s, 3H), 2.10 - 2.16 (m, 2H), 2.39 (d, J = 6.4 Hz, 2H), 2.41 - 2.52 (m, 2H), 2.56 - 2.63 (m, 1H), 2.80 (d, J = 16.2 Hz, 1H), 2.94 (dd, J = 6.7, 18.5 Hz, 1H), 3.07 (d, J = 16.2 Hz, 1H), 3.09 (d, J = 18.5 Hz, 1H), 3.21 (d, J = 6.7 Hz, 1H), 3.54 (s, 3H), 5.21 (d, J = 6.1 Hz, 1H), 5.29 (d, J = 6.1 Hz, 1H), 6.39 (d, J = 2.6 Hz, 1H), 6.64 (dd, J = 2.6, 8.4 Hz, 1H), 6.98 (d, J = 8.4 Hz, 1H), 7.20 - 7.35 (m, 5H), 1H (OH) was not observed.

### Example 5

### Synthesis of (6R,6aS,11aR)-14-(cyclopropylmethyl)-2-methoxy-8-methyl-10-(pyridin-2-yl)-5,6,10,11-tetrahydro-6,11a-(epiminoethano)naphtho[2,1-f]indazol-6a(7H)-ol (Compound 11) and (6R,6aS,11aR)-14-(cyclopropylmethyl)-2-methoxy-8-methyl-9-(pyridin-2-yl)-5,6,9,11-tetrahydro-6,11a-(epiminoethano)naphtho[2,1-f]indazol-6a(7H)-ol (Compound 11a)

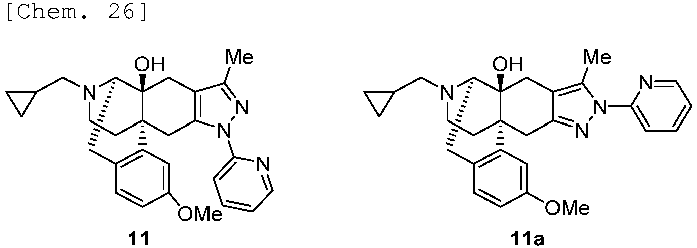

A reaction was carried out at 65°C using the compound 3 (76.7 mg, 0.20 mmol), 2-hydradinyl pyridine (43.6 mg, 0.40 mmol), and methanesulfonic acid (31 µL, 0.48 mmol) in the same manner as that of Example 1, and thus, the title compound 11 (85.4 mg, 93%) as yellow oil was obtained. The production of the position isomer 11a was also confirmed, but could not be isolated.

### Compound 11

¹H-NMR (400 MHz, CDCl₃) δ (ppm): 0.10 - 0.20 (m, 2H), 0.51 - 0.61 (m, 2H), 0.84 - 0.98 (m, 1H), 1.35 - 1.42 (m, 1H), 2.09 (s, 3H), 2.14 - 2.30 (m, 2H), 2.42 (d, J = 6.5 Hz, 1H), 2.40 - 2.52 (m, 2H), 2.63 - 2.70 (m, 1H), 2.95 (dd, J = 6.8, 18.6 Hz, 1H)3.14 (d, J = 18.6 Hz, 1H), 3.20 - 3.26 (m, 2H), 3.25 (d, J = 6.8 Hz, 1H), 3.61 (s, 3H), 4.39 (d, J = 17.4 Hz, 1H), 6.64 (dd, J = 2.6, 8.4 Hz, 1H), 6.88 (d, J = 2.6 Hz, 1H), 6.99 (d, J = 8.4 Hz, 1H), 7.09 (ddd, J = 1.0, 4.9, 7.5 Hz, 1H)7.73 (ddd, J = 1.9, 7.8, 9.7 Hz, 1H), 7.83 (br d, J = 8.3 Hz, 1H), 8.44 (ddd, J = 1.0, 1.9, 4.9 Hz, 1H), 1H (OH) was not observed.

### Example 6

### Synthesis of (6R,6aS,11aR)-14-(cyclopropylmethyl)-2-methoxy-8-methyl-10-(pyridin-4-yl)-5,6,10,11-tetrahydro-6,11a-(epiminoethano)naphtho[2,1-f]indazol-6a(7H)-ol (Compound 12) and (6R,6aS,11aR)-14-(cyclopropylmethyl)-2-methoxy-8-methyl-9-(pyridin-4-yl)-5,6,9,11-tetrahydro-6,11a-(epiminoethano)naphtho[2,1-f]indazol-6a(7H)-ol (Compound 12a)

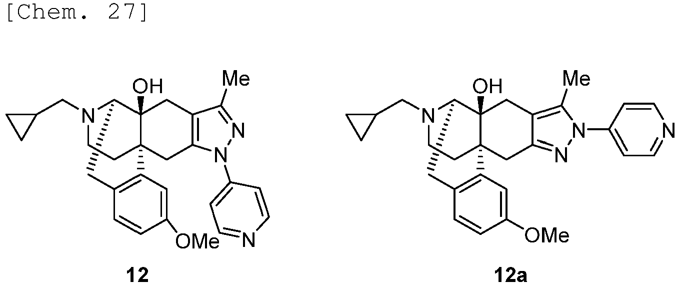

A reaction was carried out using the compound 3 (83.0 mg, 0.22 mmol), 4-hydradinyl pyridine (synthesized by a method described in Arch. Pharm. Chem. Life Sci. 2019, 352, e1800247) (38.0 mg, 0.35 mmol), and methanesulfonic acid (33 µL, 0.51 mmol) in the same manner as that of Example 1, and thus, the title compound 12 (60.3 mg, 61%) as yellow oil was obtained. The production of the position isomer 12a was also confirmed, but could not be isolated.

### Compound 12

¹H-NMR (400 MHz, CDCl₃) δ (ppm): 0.11 - 0.20 (m, 2H), 0.52 - 0.62 (m, 2H), 0.86 - 0.95 (m, 1H), 1.34 - 1.41 (m, 1H), 2.10 (s, 3H), 2.17 (ddd, J = 3.6, 11.8, 12.5 Hz, 1H), 2.26 (ddd, J = 3.6, 11.8, 12.5 Hz, 1H), 2.44 (dd, J = 1.8, 6.5 Hz, 2H), 2.46 - 2.52 (m, 2H), 2.64 - 2.71 (m, 1H), 2.96 (dd, J = 6.8, 18.6 Hz, 1H), 3.13 (d, J = 18.6 Hz, 1H)3.23 (d, J = 16.2 Hz, 1H), 3.28 (d, J = 6.8 Hz, 1H), 3.41 (d, J = 16.2 Hz, 1H)3.57 (s, 3H)6.45 (d, J = 2.6 Hz, 1H), 6.64 (dd, J = 2.6, 8.3 Hz, 1H), 7.00 (d, J = 8.3 Hz, 1H), 7.54 (dd, J = 1.6, 4.8 Hz, 2H), 8.66 (dd, J = 1.6, 4.8 Hz, 2H), 1H (OH) was not observed.

### Example 7

### Synthesis of (6R,6aS,11aR)-14-(cyclopropylmethyl)-2-methoxy-8-methyl-10-(pyrimidin-2-yl)-5,6,10,11-tetrahydro-6,11a-(epiminoethano)naphtho[2,1-f]indazol-6a(7H)-ol (Compound 13) and (6R,6aS,11aR)-14-(cyclopropylmethyl)-2-methoxy-8-methyl-9-(pyrimidin-2-yl)-5,6,9,11-tetrahydro-6,11a-(epiminoethano)naphtho[2,1-f]indazol-6a(7H)-ol (Compound 13a)

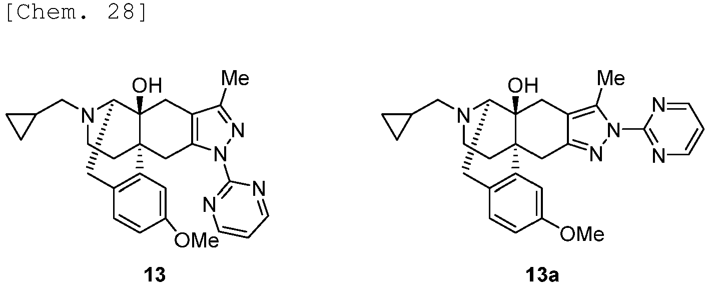

A reaction was carried out using the compound 3 (70.7 mg, 0.18 mmol), 2-hydradinyl pyridine (synthesized by a method described in Bioorg. Med. Chem. Lett. 2011, 21, 2887-2889) (31.1 mg, 0.18 mmol), and methanesulfonic acid (28 µL, 0.188 mmol) in the same manner as that of Example 1, and thus, the title compound 13 (57.8 mg, 69%) as white oil was obtained. The production of the position isomer 13a was also confirmed, but could not be isolated.

¹H-NMR (400 MHz, CDCl₃) δ (ppm): 0.09 - 0.20 (m, 2H), 0.49 - 0.61 (m, 2H), 0.84 - 0.96 (m, 1H), 1.36 - 1.43 (m, 1H), 2.14 (s, 3H), 2.15 - 2.31 (m, 2H), 2.37 - 2.53 (m, 4H), 2.63 - 2.70 (m, 1H), 2.95 (dd, J = 6.8, 18.7 Hz, 1H), 3.15 (d, J = 18.7 Hz, 1H), 3.17 - 3.22 (m, 1H), 3.26 (d, J = 6.8 Hz, 1H), 3.62 (s, 3H), 4.33 (d, J = 17.7 Hz, 1H), 6.64 (dd, J = 2.6, 8.4 Hz, 1H), 6.79 (d, J = 2.6 Hz, 1H), 7.00 (d, J = 8.4 Hz, 1H), 7.11 (dd, J = 4.8, 4.8 Hz, 1H), 8.75 (d, J = 4.8 Hz, 2H), 1H (OH) was not observed.

### Example 8

### Synthesis of a mixture (¹H-NMR ratio, Compound 14 : Compound 15 = 84 : 16) of 4-((6R,6aS,11aR)-14-(cyclopropylmethyl)-6a-hydroxy-2-methoxy-8-methyl-6,6a,7,11-tetrahydro-6,11a-(epiminoethano)naphtho[2,1-f]indazol-10(5H)-yl)pyridin-2(1H)-one (Compound 14) and 4-((6R,6aS,11aR)-14-(cyclopropylmethyl)-6a-hydroxy-2-methoxy-8-methyl-5,6,6a,7-tetrahydro-6,11a-(epiminoethano)naphtho[2,1-f]indazol-9(11H)-yl)pyridin-2(1H)-one (Compound 15)

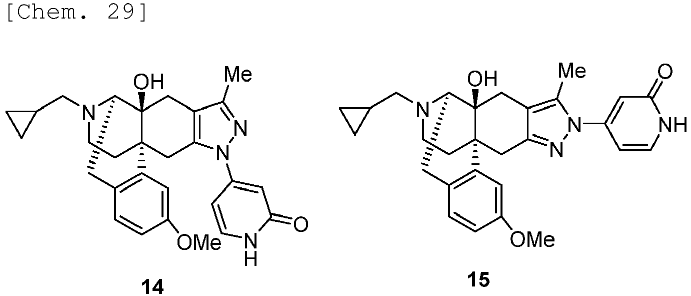

A reaction was carried out using the compound 3 (93.1 mg, 0.24 mmol), 4-hydradinylpyridin-2-(1H)-one (synthesized by a method described in Bioorg. Med. Chem. Lett. 2019, 29, 668-673) (48.1 mg, 0.36 mmol), and methanesulfonic acid (37 µL, 0.58 mmol) in the same manner as that of Example 1, and thus, a mixture (105.1 mg, 92%) of the title compound 14 and the title compound 15 as white amorphous was obtained.

¹H-NMR (400 MHz, CD₃OD) δ (ppm): 0.11 - 0.29 (m, 2H), 0.52 - 0.60 (m, 2H), 0.86 - 0.91 (m, 1H), 1.34 - 1.40 (m, 1H), 2.07 (s, 3H), 2.13 - 2.22 (m, 2H), 2.39 - 2.53 (m, 1H), 2.42 (d, J = 6.5 Hz, 2H), 2.62 - 2.72 (m, 1H), 3.10 - 3.30 (m, 3H), 3.45 (d, J = 17.2 Hz, 1H), 3.59 (s, 0.48H), 3.62 (s, 2.52H), 3.69 - 3.79 (m, 2H), 6.44 (d, J = 2.3 Hz, 0.16H), 6.53 (d, J = 2.3 Hz, 0.84H), 6.63 (d, J = 2.5 Hz, 0.84H), 6.65 (dd, J = 2.5 Hz, 8.6 Hz, 0.84H), 6.66 - 6.70 (m, 0.32H), 6.90 (dd, J = 2.1, 7.3 Hz, 0.84H), 6.96 - 7.23 (m, 1.16H), 7.30 (d, J = 7.3 Hz, 0.16H), 7.34 (d, J = 7.3 Hz, 0.84H), 2H (NH, OH) was not observed.

### Example 9

### Synthesis of (6R,6aS,11aR)-10-cyclohexyl-14-(cyclopropylmethyl)-2-methoxy-8-methyl-5,6,10,11-tetrahydro-6,11a-(epiminoethano)naphtho[2,1-f]indazol-6a(7H)-ol (Compound 16) and (6R,6aS,11aR)-9-cyclohexyl-14-(cyclopropylmethyl)-2-methoxy-8-methyl-5,6,9,11-tetrahydro-6,11a-(epiminoethano)naphtho[2,1-f]indazol-6a(7H)-ol (Compound 17)

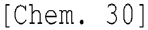

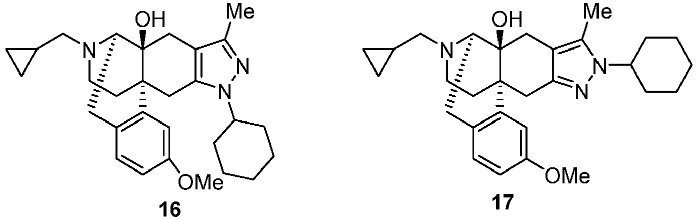

### Method 1

A reaction was carried out using the compound 3 (105.0 mg, 0.23 mmol), cyclohexylhydrazine hydrochloride (53.6 mg, 0.36 mmol), and methanesulfonic acid (42.7 µL, 0.66 mmol) in the same manner as that of Example 1, and thus, the title compound 16 (116.1 mg, 92%) as yellow oil and the title compound 17 (5.5 mg, 4%) as white oil were obtained.

### Method 2

A reaction was carried out using an equilibrium mixture (45.5 mg, 0.12 mmol) of the compound 18 and the compound 19, silver(I) oxide (32.4 mg, 0.14 mmol), and iodocyclohexane (24 µL, 0.19 mmol) in the same manner as that of Method 2 described in Example 2, and thus, the title compound 16 (9.5 mg, 17%) and the title compound 17 (12.9 mg, 23%) as colorless oil was obtained.

### Compound 16

¹H-NMR (400 MHz, CDCl₃) δ (ppm): 0.09 - 0.18 (m, 2H), 0.50 - 0.59 (m, 2H), 0.83 - 0.94 (m, 1H), 1.22 - 1.50 (m, 5H), 1.61 - 1.76 (m, 1H), 1.87 - 1.98 (m, 6H), 2.01 (s, 3H), 2.11 - 2.25 (m, 2H), 2.38 (d, J = 16.2 Hz, 1H), 2.41 (d, J = 6.6 Hz, 2H), 2.44 (d, J = 16.2 Hz, 1H), 2.62 - 2.78 (m, 1H), 2.88 (d, J = 15.9 Hz, 1H), 2.95 (dd, J = 6.8, 18.5 Hz, 1H), 3.10 (d, J = 18.5 Hz, 1H), 3.19 (d, J = 15.9 Hz, 1H), 3.22 (d, J = 6.8 Hz, 1H), 3.70 (s, 3H), 3.93 - 4.03 (m, 1H), 6.65 (dd, J = 2.5, 8.3 Hz, 1H), 6.65 (d, J = 2.5 Hz, 1H), 6.99 (d, J = 8.3 Hz, 1H).

### Compound 17

¹H-NMR (400 MHz, CDCl₃) δ (ppm): 0.08 - 0.20 (m, 2H), 0.48 - 0.59 (m, 2H), 0.82 - 0.95 (m, 1H), 1.19 - 1.31 (m, 5H), 1.63 - 1.89 (m, 1H), 1.74 - 1.93 (m, 6H), 2.01 (s, 3H), 2.12 - 2.25 (m, 2H), 2.40 (d, J = 15.7 Hz, 1H), 2.61 (d, J = 7.1 Hz, 2H), 2.46 (d, J = 15.7 Hz, 1H), 2.60 - 2.69 (m, 1H), 2.93 (dd, J = 6.4, 18.4 Hz, 1H), 2.98 (d, J = 16.2 Hz, 1H), 3.11 (d, J = 18.4 Hz, 1H), 3.20 (br d, J = 6.4 Hz, 1H), 3.31 (d, J = 16.2 Hz, 1H), 3.69 (s, 3H), 3.72 - 3.83 (m, 1H), 6.65 (dd, J = 2.6, 8.4 Hz, 1H), 6.85 (d, J = 2.6 Hz, 1H), 6.48 (d, J = 8.4 Hz, 1H).

### Example 10

### Synthesis of an equilibrium mixture of (6R,6aS,11aR)-14-(cyclopropylmethyl)-2-methoxy-8-methyl-5,6,9,11-tetrahydro-6,11a-(epiminoethano)naphtho[2,1-f]indazol-6a(7H)-ol (Compound 18) and (6R,6aS,11aR)-14-(cyclopropylmethyl)-2-methoxy-8-methyl-5,6,10,11-tetrahydro-6,11a-(epiminoethano)naphtho[2,1-f]indazol-6a(7H)-ol (Compound 19)

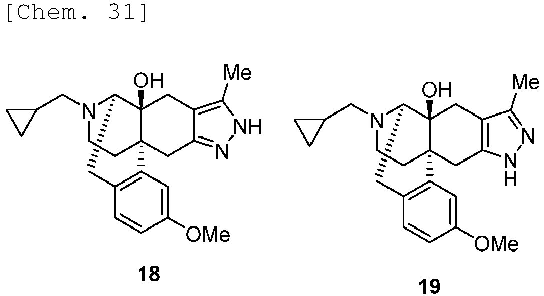

A reaction was carried out using the compound 3 (89.5 mg, 0.23 mmol), hydrazine hydrochloride (28.4 mg, 0.42 mmol), and methanesulfonic acid (36 µL, 0.55 mmol) in the same manner as that of Example 1, and thus, an equilibrium mixture (87.0 mg, 98%) of the title compounds 18 and 19 as white amorphous was obtained.

¹H-NMR (400 MHz, CDCl₃) δ (ppm): 0.09 - 0.20 (m, 2H), 0.50 - 0.60 (m, 2H), 0.83 - 0.94 (m, 1H), 1.31 - 1.37 (m, 1H), 2.03 (s, 3H), 2.14 - 2.24 (m, 2H), 2.37 - 2.52 (m, 2H), 2.41 (d, J = 6.5 Hz, 2H), 2.60 - 2.70 (m, 1H), 2.90 - 2.96 (m, 1H), 2.99 (d, J = 16.1 Hz, 1H), 3.12 (d, J = 18.5 Hz, 1H), 3.22 (d, J = 6.7 Hz, 1H), 3.26 (d, J = 16.1 Hz, 1H), 3.70 (s, 3H), 6.65 (dd, J = 2.3, 8.4 Hz, 1H), 6.78 (d, J = 2.3 Hz, 1H), 6.99 (d, J = 8.4 Hz, 1H), 2H (NH, OH) was not observed.

### Example 11

### Synthesis of (6R,6aS,11aR)-14-(cyclopropylmethyl)-8-methyl-9-phenyl-5,6,9,11-tetrahydro-6,11a-(epiminoethano)naphtho[2,1-f]indazole-2,6a(7H)-diol (Compound 20)

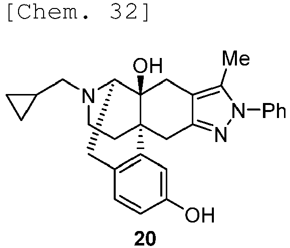

Under an argon atmosphere, a 1.0 M dichloromethane solution (0.54 mL, 0.54 mmol) of boron tribromide was added to a dichloromethane (3 mL) solution of the compound 4 (82.7 mg, 0.18 mmol), and stirring was performed at 0°C for 2 hours. Methanol (2 mL) was added to the reaction solution, stirring was performed at room temperature for 30 minutes, and then, concentration was performed under reduced pressure. A saturated sodium bicarbonate aqueous solution was added to the residue, and extraction was performed with a mixed solvent of chloroform and ethanol (chloroform : ethanol = 3 : 1). After combined organic layers were washed with saturated brine and dried with anhydrous sodium sulfate, concentration was performed under reduced pressure. The obtained crude product was purified by silica gel column chromatography, and thus, the title compound 20 (57.7 mg, 72%) as yellow-white amorphous was obtained.

¹H-NMR (400 MHz, CDCl₃) δ (ppm): 0.10 - 0.20 (m, 2H), 0.51 - 0.60 (m, 2H), 0.84 - 0.95 (m, 1H), 1.23 - 1.32 (m, 1H), 2.09 (s, 3H), 2.10 - 2.25 (m, 2H), 2.42 (d, J = 6.5 Hz, 2H), 2.47 (d, J = 16.1 Hz, 1H), 2.52 (d, J = 16.1 Hz, 1H), 2.62 - 2.68 (m, 1H), 2.94 (dd, J = 6.6, 18.5 Hz, 1H), 3.09 (br d, J = 17.9 Hz, 2H), 3.20 (d, J = 16.4 Hz, 1H), 3.27 (d, J = 6.6 Hz, 1H), 6.42 (d, J = 2.5 Hz, 1H), 6.52 (br d, J = 8.3 Hz, 1H), 6.90 (d, J = 8.1 Hz, 1H), 7.24 - 7.30 (m, 1H), 7.36 - 7.50 (m, 4H), 2H (OH × 2) was not observed.

### Example 12

### Synthesis of (6R,6aS,11aR)-14-(cyclopropylmethyl)-8-methyl-10-phenyl-5,6,10,11-tetrahydro-6,11a-(epiminoethano)naphtho[2,1-f]indazole-2,6a(7H)-diol (Compound 21)

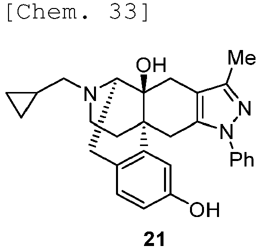

A reaction was carried out using the compound 5 (110 mg, 0.24 mmol), and a 1.0 M dichloromethane solution of boron tribromide (0.75 mL, 0.75 mmol) in the same manner as that of Example 11, and thus, the title compound 21 (78.1 mg, 73%) as yellow-white amorphous was obtained.

¹H-NMR (400 MHz, CDCl₃) δ (ppm): 0.12 - 0.20 (m, 2H), 0.52 - 0.60 (m, 2H), 0.84 - 0.96 (m, 1H), 1.32 - 1.40 (m, 1H), 2.05 (s, 3H), 2.14 - 2.26 (m, 2H), 2.42 (d, J = 6.5 Hz, 2H), 2.48 (d, J = 16.0 Hz, 1H), 2.56 (d, J = 15.9 Hz, 1H), 2.64 - 2.70 (m, 1H), 2.93 (dd, J = 6.7, 18.6 Hz, 1H), 3.09 (d, J = 16.5 Hz, 1H), 3.14 (d, J = 18.6 Hz, 1H), 3.23 (d, J = 6.7 Hz, 1H), 3.37 (d, 16.5 Hz, 1H), 6.51 (dd, J = 2.5, 8.2 Hz, 1H), 6.87 (d, J = 2.5 Hz, 1H), 6.91 (d, J = 8.2 Hz, 1H), 7.18 - 7.30 (m, 5H), 2H (OH × 2) was not observed.

### Example 13

### Synthesis of (6R,6aS,11aR)-14-(cyclopropylmethyl)-9-isopropyl-8-methyl-5,6,9,11-tetrahydro-6,11a-(epiminoethano)naphtho[2,1-f]indazole-2,6a(7H)-diol (Compound 22)

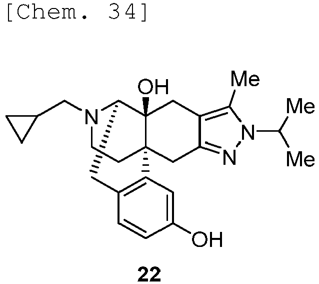

A reaction was carried out using the compound 6 (13.7 mg, 0.03 mmol), and a 1.0 M dichloromethane solution of boron tribromide (0.09 mL, 0.09 mmol) in the same manner as that of Example 11, and thus, the title compound 22 (10.0 mg, 76%) as white oil was obtained.

¹H-NMR (400 MHz, CD₃OD) δ (ppm): 0.10 - 0.18 (m, 2H), 0.50 - 0.58 (m, 2H), 0.84 - 0.91 (m, 1H), 1.20 - 1.28 (m, 1H), 1.30 (d, J = 6.7 Hz, 3H), 1.36 (d, J = 6.7 Hz, 3H), 1.99 (s, 3H), 2.09 - 2.68 (m, 2H), 3.57 (d, J = 15.9 Hz, 1H), 2.41 (d, J = 6.5 Hz, 2H), 2.47 (d, J = 15.9 Hz, 1H), 2.60 - 2.68 (m, 1H), 2.89 (dd, J = 6.6, 18.4 Hz, 1H), 2.96 (d, J = 16.2 Hz, 1H), 3.11 (d, J = 18.4 Hz, 1H), 3.19 (d, J = 6.7 Hz, 1H), 3.30 (d, J = 16.2 Hz, 1H), 4.24 (sept, 1H), 6.61 (dd, J = 2.5, 8.3 Hz, 1H), 6.84 (d, J = 2.5 Hz, 1H), 6.92 (d, J = 8.3 Hz, 1H), 2H (OH × 2) was not observed.

### Example 14

### Synthesis of (6R,6aS,11aR)-14-(cyclopropylmethyl)-10-isopropyl-8-methyl-5,6,10,11-tetrahydro-6,11a-(epiminoethano)naphtho[2,1-f]indazole-2,6a(7H)-diol (Compound 23)

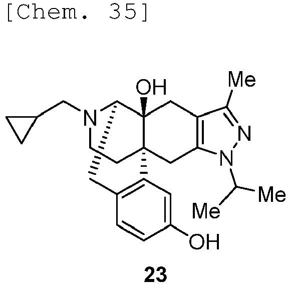

A reaction was carried out using the compound 7 (101 mg, 0.24 mmol), and a 1.0 M dichloromethane solution of boron tribromide (0.72 mL, 0.72 mmol) in the same manner as that of Example 11, and thus, the title compound 23 (73.2 mg, 75%) as a white solid was obtained.

¹H-NMR (400 MHz, CD₃OD) δ (ppm): 0.14 - 0.24 (m, 2H), 0.50 - 0.62 (m, 2H), 0.87 - 0.98 (m, 1H), 1.32 - 1.37 (m, 1H), 1.44 (d, J = 6.8 Hz, 3H), 1.47 (d, J = 6.8 Hz, 3H), 1.90 (s, 3H), 2.18 - 2.28 (m, 2H), 2.34 (d, J = 16.5 Hz, 1H), 2.45 - 2.52 (m, 2H), 2.49 (d, J = 16.5 Hz, 1H), 2.68

(d, J = 6.7 Hz, 1H), 2.80 (d, J = 16.2 Hz, 1H), 2.96 (dd, J = 6.7, 18.6 Hz, 1H), 3.14 (d, J = 18.6 Hz, 1H), 3.31 (d, J = 16.2 Hz, 1H), 4.54 (sept, 1H), 6.55 (dd, J = 2.5, 8.3 Hz, 1H), 6.63 (d, J = 2.5 Hz, 1H), 6.93 (d, J = 8.3 Hz, 1H), 3H (OH × 2, 6C-H) was not observed. The proton at the 6-position (6C-H) was not confirmed because it overlapped with the signal of the heavy solvent.

### Example 15

### Synthesis of (6R,6aS,11aR)-9-(tert-butyl)-14-(cyclopropylmethyl)-8-methyl-5,6,9,11-tetrahydro-6,11a-(epiminoethano)naphtho[2,1-f]indazole-2,6a(7H)-diol (Compound 24)

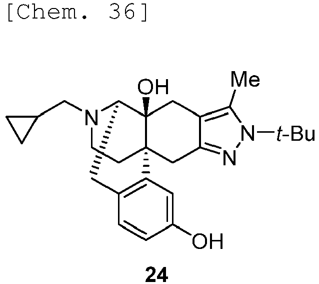

A reaction was carried out using the compound 8 (16.8 mg, 0.039 mmol), and a 1.0 M dichloromethane solution of boron tribromide (0.05 mL, 0.05 mmol) in the same manner as that of Example 11, and thus, the title compound 24 (10.7 mg, 66%) as yellow-white oil was obtained.

¹H-NMR (400 MHz, CDCl₃) δ (ppm): 0.09 - 0.20 (m, 2H), 0.49 - 0.60 (m, 2H), 0.80 - 0.94 (m, 1H), 1.23 - 1.32 (m, 1H), 1.52 (s, 9H), 2.14 (s, 3H), 2.14 - 2.23 (m, 2H), 2.35 (d, J = 15.8 Hz, 1H), 2.40 (d, J = 5.0 Hz, 2H), 2.44 (d, J = 15.8 Hz, 1H), 2.57 - 2.69 (m, 1H), 2.90 (dd, J = 6.6, 18.6 Hz, 1H), 2.93 (d, J = 16.3 Hz, 1H), 3.09 (d, J = 18.6 Hz, 1H), 3.19 (d, J = 6.6 Hz, 1H), 3.26 (d, J = 16.3 Hz, 1H), 6.56 (dd, J = 2.4, 8.2 Hz, 1H), 6.79 (d, J = 2.4 Hz, 1H), 6.90 (d, J = 8.2 Hz, 1H), 2H (OH × 2) was not observed.

### Example 16

### Synthesis of (6R,6aS,11aR)-10-(tert-butyl)-14-(cyclopropylmethyl)-8-methyl-5,6,10,11-tetrahydro-6,11a-(epiminoethano)naphtho[2,1-f]indazole-2,6a(7H)-diol (Compound 25)

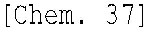

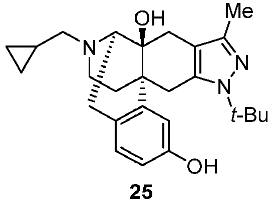

A reaction was carried out using the compound 9 (88.1 mg, 0.23 mmol), and a 1.0 M dichloromethane solution of boron tribromide (0.65 mL, 0.65 mmol) in the same manner as that of Example 11, and thus, the title compound 25 (24.8 mg, 29%) as a white solid was obtained.

¹H-NMR (400 MHz, CD₃OD) δ (ppm): 0.02 - 0.15 (m, 2H), 0.38 - 0.53 (m, 2H), 0.73 - 0.90 (m, 1H), 1.15 - 1.29 (m, 1H), 1.57 (s, 9H), 1.86 (s, 3H), 2.15 - 2.18 (m, 2H), 2.23 (d, J = 16.2 Hz, 1H), 2.36 (d, J = 6.9 Hz, 2H), 2.40 (d, J = 16.2 Hz, 1H), 2.57 (d, J = 6.9 Hz, 1H), 2.80 - 2.92 (m, 2H), 3.03 (d, J = 18.6 Hz, 1H), 3.46 (d, J = 16.4 Hz, 1H), 6.45 (dd, J = 2.4, 8.3 Hz, 1H), 6.57 (d, J = 2.4 Hz, 1H), 6.82 (d, J = 8.3 Hz, 1H), 3H (OH × 2, 6C-H) was not observed. The proton at the 6-position (6C-H) was not confirmed because it overlapped with the signal of the heavy solvent.

### Example 17

### Synthesis of (6R,6aS,11aR)-10-benzyl-14-(cyclopropylmethyl)-8-methyl-5,6,10,11-tetrahydro-6,11a-(epiminoethano)naphtho[2,1-f]indazole-2,6a(7H)-diol (Compound 26)

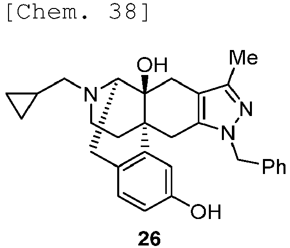

A reaction was carried out using the compound 10 (34.7 mg, 0.074 mmol), and a 1.0 M dichloromethane solution of boron tribromide (0.25 mL, 0.25 mmol) in the same manner as that of Example 11, and thus, the title compound 26 (21.8 mg, 65%) as yellow oil was obtained.

¹H-NMR (400 MHz, CDCl₃) δ (ppm): 0.06 - 0.17 (m, 2H), 0.48 - 0.58 (m, 2H), 0.80 - 0.95 (m, 1H), 1.12 - 1.20 (m, 1H), 2.05 (s, 3H), 2.07 - 2.13 (m, 2H), 2.37 (d, J = 6.5 Hz, 2H), 2.40 - 2.48 (m, 2H), 2.59 (d, J = 6.1 Hz, 1H), 2.79 (d, J = 16.2 Hz, 1H), 2.88 (dd, J = 6.6, 18.4 Hz, 1H), 2.92 (d, J = 16.2 Hz, 1H), 3.05 (d, J = 18.4 Hz, 1H), 3.19 (d, J = 6.6 Hz, 1H), 5.11 (d, J = 16.0 Hz, 1H), 5.30 (d, J = 16.0 Hz, 1H), 6.51 (dd, J = 2.4, 8.3 Hz, 1H), 6.86 (d, J = 8.3 Hz, 1H), 7.17 (br d, J = 7.4 Hz, 1H), 7.28 - 7.40 (m, 5H), 2H (OH × 2) was not observed.

### Example 18

### Synthesis of (6R,6aS,11aR)-14-(cyclopropylmethyl)-8-methyl-10-(pyridin-2-yl)-5,6,10,11-tetrahydro-6,11a-(epiminoethano)naphtho[2,1-f]indazole-2,6a(7H)-diol (Compound 27)

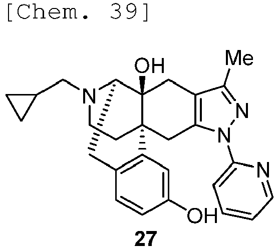

A reaction was carried out using the compound 11 (85.3 mg, 0.19 mmol), and a 1.0 M dichloromethane solution of boron tribromide (0.50 mL, 0.50 mmol) in the same manner as that of Example 11, and thus, the title compound 27 (55.8 mg, 67%) as white amorphous was obtained.

¹H-NMR (400 MHz, CDCl₃) δ (ppm): 0.10 - 0.20 (m, 2H), 0.50 - 0.61 (m, 2H), 0.84 - 0.96 (m, 1H), 1.33 - 1.40 (m, 1H), 2.08 (s, 3H), 2.12 - 2.28 (m, 2H), 2.42 (d, J = 6.6 Hz, 2H), 2.44 - 2.52 (m, 2H), 2.66 (d, J = 6.9 Hz, 1H), 2.93 (dd, J = 6.9, 18.6 Hz, 1H)3.12 (d, J = 18.6 Hz, 1H), 3.17 - 3.29 (m, 2H), 4.27 (d, J = 17.6 Hz, 1H), 6.57 (dd, J = 2.0, 8.2 Hz, 1H), 6.77 (d, J = 2.0 Hz, 1H), 6.93 (d, J = 8.2 Hz, 1H), 7.09 (br s, 1H), 7.72 (br d, J = 7.3 Hz, 1H), 7.80 (br d, J = 7.9 Hz, 1H), 8.42 (br s, 1H), 2H (OH × 2) was not observed.

### Example 19

### Synthesis of (6R,6aS,11aR)-14-(cyclopropylmethyl)-8-methyl-10-(pyridin-4-yl)-5,6,10,11-tetrahydro-6,11a-(epiminoethano)naphtho[2,1-f]indazole-2,6a(7H)-diol (Compound 28)

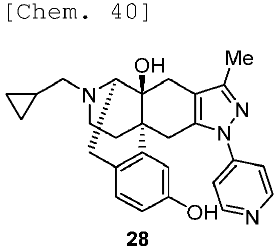

A reaction was carried out using the compound 12 (60.3 mg, 0.13 mmol), and a 1.0 M dichloromethane solution of boron tribromide (0.4 mL, 0.4 mmol) in the same manner as that of Example 11, and thus, the title compound 28 (9.8 mg, 14%) as a white solid was obtained.

¹H-NMR (400 MHz, CD₃OD) δ (ppm): 0.18 - 0.28 (m, 2H), 0.55 - 0.60 (m, 2H), 0.98 - 1.14 (m, 1H), 1.36 - 1.45 (m, 1H), 2.13 (s, 3H), 2.18 - 2.39 (m, 2H), 2.45 - 2.55 (m, 2H), 2.47 (dd, J = 1.2, 16.4 Hz, 1H), 2.68 - 2.76 (m, 1H), 2.64 (dd, J = 6.9, 18.6 Hz, 1H), 3.19 (d, J = 18.6 Hz, 1H), 3.26 (d, J = 16.6 Hz, 1H), 3.38 (d, J = 6.6 Hz, 1H), 3.50 (d, J = 16.6 Hz, 1H), 6.44 (d, J = 2.5 Hz, 1H), 6.97 (dd, J = 2.5, 8.3 Hz, 1H), 6.97 (d, J = 8.3 Hz, 1H), 7.75 (dd, J = 1.6, 4.8 Hz, 2H), 8.76 (dd, J = 1.5, 4, 9 Hz, 2H), 3H (OH × 2, 7C-H or 11C-H) was not observed. The proton at the 7-position (7C-H) or the proton at the 11-position (11C-H) were not confirmed because they overlapped with the signal of the heavy solvent.

### Example 20

### Synthesis of (6R,6aS,11aR)-14-(cyclopropylmethyl)-8-methyl-10-(pyrimidin-2-yl)-5,6,10,11-tetrahydro-6,11a-(epiminoethano)naphtho[2,1-f]indazole-2,6a(7H)-diol (Compound 29)

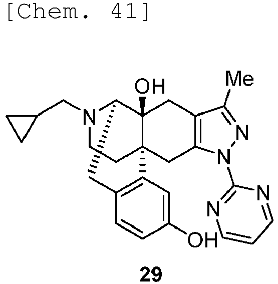

A reaction was carried out using the compound 13 (57.8 mg, 0.13 mmol), and a 1.0 M dichloromethane solution of boron tribromide (0.6 mL, 0.6 mmol) in the same manner as that of Example 11, and thus, the title compound 29 (19.9 mg, 36%) as white amorphous was obtained.

¹H-NMR (400 MHz, CD₃OD) δ (ppm): 0.04 - 0.12 (m, 2H), 0.40 - 0.52 (m, 2H), 0.76 - 0.89 (m, 1H), 1.22 - 1.30 (m, 1H), 2.01 (s, 3H), 2.18 - 2.22 (m, 2H), 2.31 (d, J = 16.5 Hz, 1H), 2.36 (d, J = 6.5 Hz, 2H), 2.47 (dd, J = 2.0, 16.5 Hz, 1H), 2.59 (d, J = 6.5 Hz, 1H), 2.86 (dd, J = 6.5, 18.6 Hz, 1H), 3.02 - 3.09 (m, 1H), 3.07 (d, J = 18.6 Hz, 1H), 3.22 - 3.26 (m, 1H), 4.17 (d, J = 17.9 Hz, 1H), 6.43 (dd, J = 2.5, 8.4 Hz, 1H), 6.55 (d, J = 2.5 Hz, 1H), 6.84 (d, J = 8.4 Hz, 1H), 7.22 (dd, J = 4.8, 4.8 Hz, 1H), 8.71 (d, J = 4.8 Hz, 2H), 2H (OH × 2) was not observed.

### Example 21

### Synthesis of 4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)pyridin-2(1H)-one (Compound 30) and 4-((6R,6aS,11aR)-14-(cyclopropylmethyl)-2,6a-dihydroxy-8-methyl-5,6,6a,7-tetrahydro-6,11a-(epiminoethano)naphtho[2,1-f]indazol-9(11H)-yl)pyridin-2(1H)-one (Compound 31)

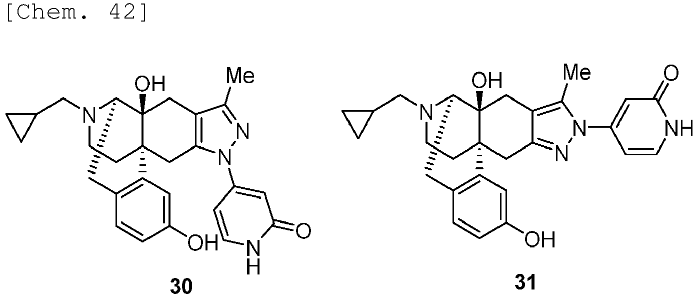

A reaction was carried out using a mixture (125.1 mg, 0.26 mmol) of the compound 14 and the compound 15 and a 1.0 M dichloromethane solution (0.80 mL, 0.80 mmol) of boron tribromide in the same manner as that of Example 11, and thus, a mixture of the title compounds 30 and 31 as a white solid (¹H-NMR ratio, compound 30 : compound 31 = 84 : 16) (34.0 mg, 28%) was obtained.

¹H-NMR (400 MHz, CD₃OD) δ (ppm): 0.12 - 0.24 (m, 2H), 0.50 - 0.62 (m, 2H), 0.86 - 0.98 (m, 1H), 1.33 - 1.39 (m, 1H), 2.01 (s, 0.48H), 2.52 (s, 2.52H), 2.14 - 2.33 (m, 2H), 2.39 - 2.50 (m, 3H), 2.52 - 2.59 (m, 1H), 2.62 - 2.72 (m, 1H), 2.90 - 2.96 (m, 1H), 2.95 (dd, J = 6.5, 18.3 Hz, 1H), 3.10 - 3.21 (m, 2H), 3.47 (d, J = 16.5 Hz, 1H), 6.46 (d, J = 2.4 Hz, 1H), 6.53 (dd, J = 2.4, 8.3 Hz, 1H), 6.66 (d, J = 2.1 Hz, 0.84H), 6.67 (d, J = 2.4 Hz, 0.16H), 6.73 (dd, J = 2.1, 7.2 Hz, 0.16H), 6.87 (dd, J = 2.1, 7.2 Hz, 0.84H), 6.94 (d, J = 8.3 Hz, 1H), 7.50 (d, J = 7.2 Hz, 0.16H), 7.56 (d, J = 7.2 Hz, 0.84H), 3H (NH, OH × 2) was not observed.

### Example 22

### Synthesis of (6R,6aS,11aR)-10-cyclohexyl-14-(cyclopropylmethyl)-8-methyl-5,6,10,11-tetrahydro-6,11a-(epiminoethano)naphtho[2,1-f]indazole-2,6a(7H)-diol (Compound 32)

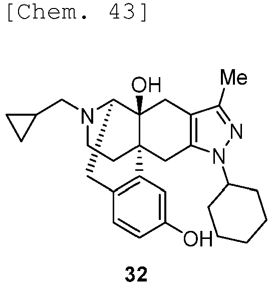

A reaction was carried out using the compound 16 (58.5 mg, 0.13 mmol), and a 1.0 M dichloromethane solution of boron tribromide (0.6 mL, 0.6 mmol) in the same manner as that of Example 11, and thus, the title compound 32 (36.0 mg, 64%) as pale yellow amorphous was obtained.

¹H-NMR (400 MHz, CDCl₃) δ (ppm): 0.08 - 0.19 (m, 2H), 0.48 - 0.60 (m, 2H), 0.81 - 0.92 (m, 1H), 1.17 - 1.49 (m, 5H), 1.63 - 1.71 (m, 1H), 1.82 - 1.95 (m, 6H), 2.00 (s, 3H), 2.11 - 2.25 (m, 2H), 2.38 (d, J = 15.9 Hz, 1H), 2.41 (d, J = 6.4 Hz, 2H), 2.45 (d, J = 15.9 Hz, 1H), 2.63 - 2.69 (m, 1H), 2.85 (d, J = 16.1 Hz, 1H), 2.93 (dd, J = 6.4, 18.5 Hz, 1H), 3.07 (d, J = 18.5 Hz, 1H), 3.15 (d, J = 16.1 Hz, 1H), 3.21 (d, J = 6.4 Hz, 1H), 3.90 - 4.00 (m, 1H), 6.54 (dd, J = 2.2, 8.2 Hz, 1H), 6.62 (d, J = 2.2 Hz, 1H), 6.90 (d, J = 8.2 Hz, 1H), 1H (OH) was not observed.

### Example 23

### Synthesis of (6R,6aS,11aR)-9-cyclohexyl-14-(cyclopropylmethyl)-8-methyl-5,6,9,11-tetrahydro-6,11a - (epiminoethano)naphtho[2,1-f]indazole-2,6a(7H)-diol (Compound 33)

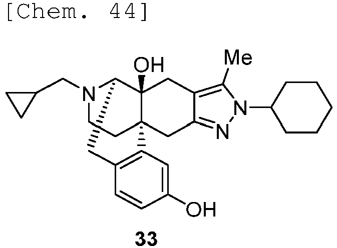

A reaction was carried out using the compound 17 (12.0 mg, 0.026 mmol), and a 1.0 M dichloromethane solution of boron tribromide (0.2 mL, 0.2 mmol) in the same manner as that of Example 11, and thus, the title compound 33 (10.0 mg, 86%) as pale yellow oil was obtained.

¹H-NMR (400 MHz, CD₃OD) δ (ppm): 0.15 - 0.25 (m, 2H), 0.41 - 0.63 (m, 2H), 0.84 - 0.99 (m, 1H), 1.23 - 1.51 (m, 5H), 1.68 - 1.76 (m, 1H), 1.77 - 1.92 (m, 6H), 2.04 (s, 3H), 2.17 - 2.31 (m, 2H), 2.37 (d, J = 16.1 Hz, 1H), 2.45 - 2.56 (m, 3H), 2.65 - 2.73 (m, 1H), 2.85 (d, J = 16.2 Hz, 1H), 2.96 (dd, J = 6.5, 18.6 Hz, 1H), 3.16 (d, J = 18.6 Hz, 1H), 3.24 (d, J = 16.2 Hz, 1H), 3.89 - 3.99 (m, 1H), 6.53 (dd, J = 2.4, 8.3 Hz, 1H), 6.67 (d, J = 2.4 Hz, 1H), 6.92 (d, J = 8.3 Hz, 1H), 2H (OH, 6C-H) was not observed. The proton at the 6-position (6C-H) was not confirmed because it overlapped with the signal of the heavy solvent.

### Example 24

### Synthesis of an equilibrium mixture of (6R,6aS,11aR)-14-(cyclopropylmethyl)-8-methyl-5,6,9,11-tetrahydro-6,11a-(epiminoethano)naphtho[2,1-f]indazole-2,6a(7H)-diol (Compound 34) and (6R,6aS,11aR)-14-(cyclopropylmethyl)-8-methyl-5,6,10,11-tetrahydro-6,11a-(epiminoethano)naphtho[2,1-f]indazole-2,6a(7H)-diol (Compound 35)

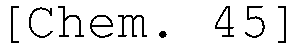

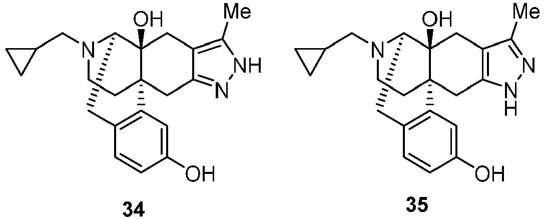

A reaction was carried out using an equilibrium mixture (72.2 mg, 0.19 mmol) of the compound 18 and the compound 19, and a 1.0 M dichloromethane solution of boron tribromide (0.2 mL, 0.2 mmol) in the same manner as that of Example 11, and thus, an equilibrium mixture (52.2 mg, 75%) of the title compounds 34 and 35 as a white solid was obtained.

¹H-NMR (400 MHz, CD₃OD) δ (ppm): 0.11 - 0.22 (m, 2H), 0.47 - 0.58 (m, 2H), 0.86 - 0.91 (m, 1H), 1.22 - 1.32 (m, 1H), 1.97 (s, 3H), 2.14 - 2.24 (m, 2H), 2.32 (d, J = 15.8 Hz, 1H), 2.42 - 2.50 (m, 3H), 2.62 - 2.68 (m, 1H), 2.86 (d, J = 16.0 Hz, 1H), 2.95 (dd, J = 6.8, 18.6 Hz, 1H), 3.14 (d, J = 18.6 Hz, 1H), 3.16 - 3.22 (m, 2H), 6.55 (dd, J = 2.6, 8.2 Hz, 1H), 6.73 (d, J = 2.6 Hz, 1H), 6.91 (d, J = 8.2 Hz, 1H), 3H (NH, OH × 2) was not observed.

### Example 25

### Synthesis of ((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)(phenyl)methanone (Compound 36)

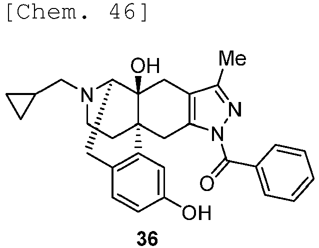

Under an argon atmosphere, pyridine (14.8 µL, 0.18 mmol) and benzoyl chloride (19.7 µL, 0.17 mmol) were added to a dichloromethane (1.5 mL) solution of an equilibrium mixture (52.2 mg, 0.14 mmol) of the compound 34 and the compound 35 at 0°C, and stirring was performed at room temperature for 30 minutes. A saturated sodium bicarbonate aqueous solution was added to the reaction solution, and extraction was performed with ethyl acetate. After an organic layer was washed with saturated brine and dried with anhydrous sodium sulfate, concentration was performed under reduced pressure. The obtained crude product was purified by silica gel column chromatography, and thus, the title compound 36 (27.6 mg, 41%) as yellow oil was obtained.

¹H-NMR (400 MHz, CDCl₃) δ (ppm): 0.10 - 0.23 (m, 2H), 0.50 - 0.64 (m, 2H), 0.90 - 1.04 (m, 1H), 1.31 - 1.39 (m, 1H), 2.03 (s, 3H), 2.18 - 2.35 (m, 3H), 2.44 - 2.57 (m, 3H), 2.74 - 2.84 (m, 1H), 3.00 (dd, J = 6.3, 18.8 Hz, 1H), 3.06 - 3.14 (m, 1H), 3.17 (d, J = 18.0 Hz, 1H), 3.47 (d, J = 6.3 Hz, 1H), 4.08 (d, J = 17.9 Hz, 1H), 6.01 (dd, J = 2.5, 8.3 Hz, 1H), 6.81 (d, J = 2.5 Hz, 1H), 6.93 (d, J = 8.3 Hz, 1H), 7.35 - 7.56 (m, 5H), 2H (OH × 2) was not observed.

### Example 26

### Synthesis of (6R,6aS,11aR)-9-(cyclohexylmethyl)-14-(cyclopropylmethyl)-2-methoxy-8-methyl-5,6,9,11-tetrahydro-6,11a-(epiminoethano)naphtho[2,1-f]indazol-6a(7H)-ol (Compound 37) and (6R,6aS,11aR)-10-(cyclohexylmethyl)-14-(cyclopropylmethyl)-2-methoxy-8-methyl-5,6,10,11-tetrahydro-6,11a-(epiminoethano)naphtho[2,1-f]indazol-6a(7H)-ol (Compound 38)

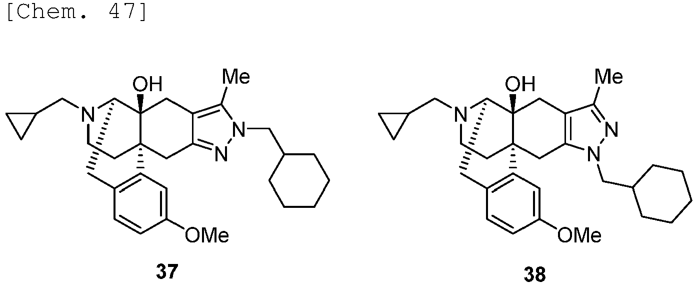

Under an argon atmosphere, an N,N-dimethylformamide (1.5 mL) solution of the equilibrium mixture (81.0 mg, 0.21 mmol) of the compound 18 and the compound 19 was added dropwise to an N,N-dimethylformamide (1.0 mL) suspension of sodium hydride (55% in oil, 63.3 mg, 1.45 mmol) at 0°C, and stirring was performed for 30 minutes. An N,N-dimethylformamide (1.0 mL) solution of bromomethylcyclohexane (147 µL, 1.06 mmol) was added at 0°C, stirring was performed at room temperature for 21 hours, heating was performed at 80°C, and stirring was performed for 24 hours. After cooling, a sodium bicarbonate aqueous solution was added, and extraction was performed with diethyl ether. After an organic layer was washed with saturated brine and dried with anhydrous sodium sulfate, concentration was performed under reduced pressure. The obtained crude product was purified by silica gel column chromatography, and thus, the title compound 37 (17.8 mg, 18%) as yellow-white oil and the title compound 38 (25.1 mg, 25%) as yellow-white oil were obtained.

### Compound 37

¹H-NMR (400 MHz, CDCl₃) δ (ppm): 0.10 - 0.20 (m, 2H), 0.49 - 0.59 (m, 2H), 0.82 - 0.96 (m, 3H), 1.08 - 1.38 (m, 4H), 1.51 - 1.84 (m, 6H), 1.96 (s, 3H), 2.11 - 2.45 (m, 2H), 2.37 - 2.49 (m, 2H), 2.41 (d, J = 6.3 Hz, 2H), 2.59 - 2.69 (m, 1H), 2.91 (dd, J = 6.7, 18.5 Hz, 1H), 2.98 (d, J = 16.2 Hz, 1H), 3.12 (d, J = 18.5 Hz, 1H), 3.20 (d, J = 6.7 Hz, 1H), 3.26 (d, J = 16.2 Hz, 1H), 3.62 - 3.74 (m, 2H), 3.68 (s, 3H), 6.64 (dd, J = 2.4, 8.4 Hz, 1H), 6.82 (d, J = 2.4 Hz, 1H), 6.96 (d, J = 8.4 Hz, 1H), 1H (OH) was not observed.

### Compound 38

¹H-NMR (400 MHz, CDCl₃) δ (ppm): 0.08 - 0.19 (m, 2H), 0.47 - 0.60 (m, 2H), 0.82 - 1.09 (m, 3H), 1.12 - 1.38 (m, 4H), 1.54 - 1.79 (m, 5H), 1.87 - 1.99 (m, 1H), 2.01 (s, 3H), 2.10 - 2.26 (m, 2H), 2.34 - 2.49 (m, 2H), 2.40 (d, J = 6.3 Hz, 2H), 2.61 - 2.68 (m, 1H), 2.85 (d, J = 16.0 Hz, 1H), 2.95 (dd, J = 6.7, 18.2 Hz, 1H), 3.10 (d, J = 18.2 Hz, 1H), 3.14 (d, J = 16.0 Hz, 1H), 3.21 (d, J = 6.7 Hz, 1H), 3.70 (s, 3H), 3.79 (dd, J = 1.3, 7.3 Hz, 2H), 6.65 (dd, J = 2.5, 8.4 Hz, 1H), 6.69 (d, J = 2.5 Hz, 1H), 6.99 (d, J = 8.4 Hz, 1H), 1H (OH) was not observed.

### Example 27

### Synthesis of (6R,6aS,11aR)-14-(cyclopropylmethyl)-2-methoxy-10-(2-methoxyethyl)-8-methyl-5,6,10,11-tetrahydro-6,11a-(epiminoethano)naphtho[2,1-f]indazol-6a(7H)-ol (Compound 39)

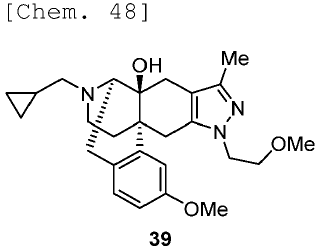

A reaction was carried out using the equilibrium mixture (88.4 mg, 0.23 mmol) of the compound 18 and the compound 19, sodium hydride (55% in oil, 65.9 mg, 1.5 mmol), and 1-bromo-2-methoxyethane (110 µL, 1.17 mmol) in the same manner as that of Example 26, and thus, the title compound 39 (72.6 mg, 71%) as yellow-white oil was obtained.

¹H-NMR (400 MHz, CDCl₃) δ (ppm): 0.08 - 0.19 (m, 2H), 0.45 - 0.58 (m, 2H), 0.84 - 0.94 (m, 1H), 1.16 - 1.24 (m, 1H), 2.03 (s, 3H), 2.05 - 2.12 (m, 1H), 2.19 (d, J = 16.3 Hz, 1H), 2.23 (dd, J = 6.9, 12.4 Hz, 1H), 2.41 - 2.53 (m, 2H), 2.57 - 2.65 (m, 2H), 2.73 (dd, J = 6.1, 18.1 Hz, 1H), 3.04 (d, J = 16.2 Hz, 1H), 3.15 (d, J = 18.1 Hz, 1H), 3.19 (d, J = 16.2 Hz, 1H), 3.27 (s, 3H), 3.42 - 3.51 (m, 4H), 3.70 (s, 3H), 3.82 - 3.90 (m, 1H), 6.64 (dd, J = 2.5, 8.4 Hz, 1H), 6.78 (d, J = 2.5 Hz, 1H), 6.97 (d, J = 8.4 Hz, 1H), 1H (OH) was not observed.

### Example 28

### Synthesis of (6R,6aS,11aR)-10,14-bis(cyclopropylmethyl)-2-methoxy-8-methyl-5,6,10,11-tetrahydro-6,11a-(epiminoethano)naphtho[2,1-f]indazol-6a(7H)-ol (Compound 40) and (6R,6aS,11aR)-9,14-bis(cyclopropylmethyl)-2-methoxy-8-methyl-5,6,9,11-tetrahydro-6,11a-(epiminoethano)naphtho[2,1-f]indazol-6a(7H)-ol (Compound 41)

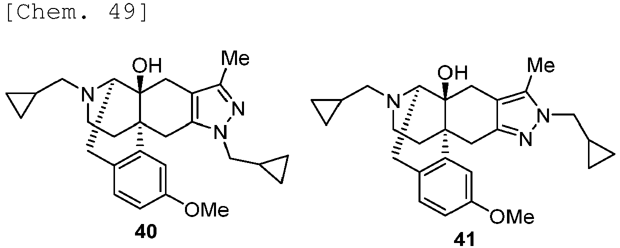

A reaction was carried out using the equilibrium mixture (80.4 mg, 0.21 mmol) of the compound 18 and the compound 19, sodium hydride (55% in oil, 25.4 mg, 0.58 mmol), and chloromethyl cyclopropane (140 µL, 1.51 mmol) in the same manner as that of Example 26, and thus, the title compound 40 (29.6 mg, 32%) as yellow oil and the title compound 41 (21.6 mg, 24%) as pale yellow oil were obtained.

### Compound 40

¹H-NMR (400 MHz, CDCl₃) δ (ppm): 0.08 - 0.19 (m, 2H), 0.33 - 0.44 (m, 2H), 0.48 - 0.64 (m, 4H), 0, 80 - 0.98 (m, 2H), 1.31 - 1.37 (m, 1H), 2.02 (s, 3H), 2.10 - 2.26 (m, 2H), 2.36 - 2.50 (m, 2H), 2.41 (d, J = 6.4 Hz, 2H), 2.59 - 2.69 (m, 1H), 2.90 (d, J = 16.1 Hz, 1H), 2.92 - 3.00 (m, 1H), 3.11 (d, J = 18.5 Hz, 1H), 3.18 (d, J = 16.1 Hz, 1H), 3.23 (d, J = 6.6 Hz, 1H), 3.69 (s, 3H), 3.86 (dd, J = 6.7, 14.4 Hz, 1H), 3.93 (dd, J = 6.7, 14.4 Hz, 1H), 6.65 (dd, J = 2.6, 8.3 Hz, 1H), 6.68 (d, J = 2.6 Hz, 1H), 6.99 (d, J = 8.3 Hz, 1H), 1H (OH) was not observed.

### Compound 41

¹H-NMR (400 MHz, CDCl₃) δ (ppm): 0.10 - 0.20 (m, 2H), 0.22 - 0.34 (m, 2H), 0.43 - 0.60 (m, 4H), 0.82 - 0.98 (m, 1H), 1.08 - 1.18 (m, 1H), 1.29 - 1.38 (m, 1H), 2.00 (s, 3H), 2.12 - 2.28 (m, 2H), 2.36 - 2.52 (m, 2H), 2.43 (d, J = 6.4 Hz, 2H), 2.63 - 2.71 (m, 1H), 2.89 - 2.96 (m, 1H), 2.99 (d, J = 16.2 Hz, 1H), 3.12 (d, J = 18.5 Hz, 1H), 3.19 - 3.26 (m, 1H), 3.28 (d, J = 16.2 Hz, 1H), 3.70 (s, 3H), 3.77 (dd, J = 4.0, 13.2 Hz, 1H), 3.78 (dd, J = 4.0, 13.2 Hz, 1H), 6.65 (dd, J = 2.6, 8.4 Hz, 1H), 6.85 (d, J = 2.6 Hz, 1H), 6.97 (d, J = 8.4 Hz, 1H), 1H (OH) was not observed.

### Example 29

### Synthesis of (6R,6aS,11aR)-9-(cyclohexylmethyl)-14-(cyclopropylmethyl)-8-methyl-5,6,9,11-tetrahydro-6,11a-(epiminoethano)naphtho[2,1-f]indazole-2,6a(7H)-diol (Compound 42)

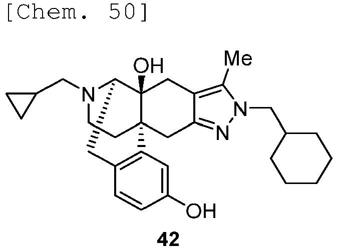

A reaction was carried out using the compound 37 (17.8 mg, 0.03 mmol), and a 1.0 M dichloromethane solution of boron tribromide (0.11 mL, 0.11 mmol) in the same manner as that of Example 11, and thus, the title compound 42 (16.8 mg, 97%) as a white solid was obtained.

¹H-NMR (400 MHz, CD₃OD) δ (ppm): 0.13 - 0.24 (m, 2H), 0.49 - 0.62 (m, 2H), 0.84 - 1.00 (m, 3H), 1.12 - 1.25 (m, 3H), 1.26 - 1.34 (m, 1H), 1.46 - 1.55 (m, 2H), 1.61 - 1.81 (m, 5H), 2.15 - 2.29 (m, 2H), 2.01 (s, 3H), 2.36 (d, J = 16.2 Hz, 1H), 2.47 (d, J = 6.5 Hz, 2H), 2.51 (d, J = 16.2 Hz, 1H), 2.64 - 2.71 (m, 1H), 2.87 (d, J = 16.1 Hz, 1H), 2.94 (dd, J = 6.8, 18.5 Hz, 1H), 3.14 (d, J = 18.5 Hz, 1H), 3.18 (d, J = 16.1 Hz, 1H), 3.74 (d, J = 7.4 Hz, 2H), 6.51 (dd, J = 2.5, 8.1 Hz, 1H), 6.64 (d, J = 2.5 Hz, 1H), 6.91 (d, J = 8.1 Hz, 1H), 2H (OH × 2) was not observed.

### Example 30

### Synthesis of (6R,6aS,11aR)-10-(cyclohexylmethyl)-14-(cyclopropylmethyl)-8-methyl-5,6,10,11-tetrahydro-6,11a-(epiminoethano)naphtho[2,1-f]indazole-2,6a(7H)-diol (Compound 43)

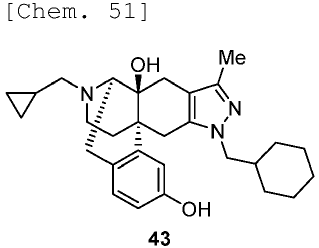

A reaction was carried out using the compound 38 (28.1 mg, 0.059 mmol), and a 1.0 M dichloromethane solution of boron tribromide (0.18 mL, 0.18 mmol) in the same manner as that of Example 11, and thus, the title compound 43 (18.6 mg, 68%) as a white solid was obtained.

¹H-NMR (400 MHz, CD₃OD) δ (ppm): 0.02 - 0.22 (m, 2H), 0.49 - 0.61 (m, 2H), 0.87 - 1.40 (m, 7H), 1.56 - 1.93 (m, 6H), 1.98 (s, 3H), 2.13 - 2.26 (m, 2H), 2.34 (d, J = 16.1 Hz, 1H), 2.45 (d, J = 6.5 Hz, 2H), 2.50 (d, J = 16.1 Hz, 1H), 2.61 - 2.71 (m, 1H), 2.77 (d, J = 16.3 Hz, 1H), 2.95 (dd, J = 6.1, 18.7 Hz, 1H), 3.13 (d, J = 18.7 Hz, 1H), 3.25 (d, J = 16.3 Hz, 1H), 3.27 (d, J = 6.1 Hz, 1H), 3.78 - 3.79 (m, 2H), 6.54 (dd, J = 2.5, 8.3 Hz, 1H), 6.63 (d, J = 2.5 Hz, 1H), 6.92 (d, J = 8.3 Hz, 1H), 2H (OH × 2) was not observed.

### Example 31

### Synthesis of (6R,6aS,11aR)-14-(cyclopropylmethyl)-10-(2-hydroxyethyl)-8-methyl-5,6,10,11-tetrahydro-6,11a-(epiminoethano)naphtho[2,1-f]indazole-2,6a(7H)-diol (Compound 44)

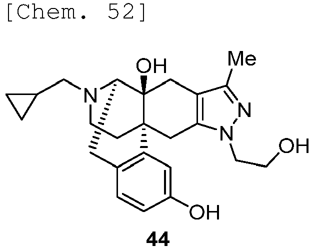

A reaction was carried out using the compound 39 (72.6 mg, 0.17 mmol), and a 1.0 M dichloromethane solution of boron tribromide (1.0 mL, 1.0 mmol) in the same manner as that of Example 11, and thus, the title compound 44 (18.6 mg, 61%) as a white solid was obtained.

¹H-NMR (400 MHz, CD₃OD) δ (ppm): 0.16 - 0.30 (m, 2H), 0.51 - 0.66 (m, 2H), 0.94 - 1.04 (m, 1H), 1.26 - 1.38 (m, 1H), 2.03 (s, 3H), 2.30 (d, J = 16.7 Hz, 1H), 2.53 (ddd, J = 5.0, 13.0, 13.0 Hz, 1H), 2.63 - 2.74 (m, 2H), 2.86 - 3.06 (m, 2H), 2.98 (d, J = 16.3 Hz, 1H), 3.21 (d, J = 7.0 Hz, 1H), 3.24 - 3.34 (m, 3H), 3.38 - 3.44 (m, 1H), 3.54 - 3.61 (m, 1H), 3.66 - 3.86 (m, 3H), 6.55 (dd, J = 2.2, 8.3 Hz, 1H), 6.70 (d, J = 2.2 Hz, 1H), 6.95 (d, J = 8.3 Hz, 1H), 3H (OH × 3) was not observed.

### Example 32

### Synthesis of (6R,6aS,11aR)-10,14-bis(cyclopropylmethyl)-8-methyl-5,6,10,11-tetrahydro-6,11a-(epiminoethano)naphtho[2,1-f]indazole-2,6a(7H)-diol (Compound 45)

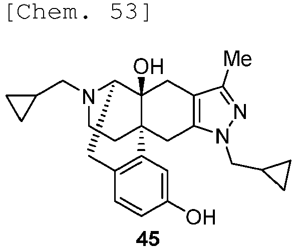

A reaction was carried out using the compound 40 (29.6 mg, 0.068 mmol), and a 1.0 M dichloromethane solution of boron tribromide (0.2 mL, 0.2 mmol) in the same manner as that of Example 11, and thus, the title compound 45 (13.5 mg, 47%) as white oil was obtained.

¹H-NMR (400 MHz, CDCl₃) δ (ppm): 0.11 - 0.19 (m, 2H), 0.20 - 0.33 (m, 2H), 0.43 - 0.61 (m, 4H), 0.80 - 0.97 (m, 2H), 1.08 - 1.20 (m, 1H), 1, 27 - 1.37 (m, 1H), 2.00 (s, 3H), 2.16 - 2.24 (m, 2H), 2.40 (d, J = 15.9 Hz, 1H), 2.42 (d, J = 7.4 Hz, 2H), 2.47 (d, J = 15.9 Hz, 1H), 2.66 - 2.70 (m, 1H), 2.85 (d, J = 16.0 Hz, 1H), 2.94 (dd, J = 6.5, 18.6 Hz, 1H), 3.08 (d, J = 18.6 Hz, 1H), 3.09 (d, J = 16.0 Hz, 1H), 3.24 (d, J = 6.5 Hz, 1H), 3.74 (dd, J = 6.7, 14.4 Hz, 1H), 3.82 (dd, J = 6.8, 14.4 Hz, 1H), 6.55 (dd, J = 2.4, 8.2 Hz, 1H), 6.64 (d, J = 2.4 Hz, 1H), 6.90 (d, J = 8.2 Hz, 1H).1H (OH) was not observed.

### Example 33

### Synthesis of (6R,6aS,11aR)-9,14-bis(cyclopropylmethyl)-8-methyl-5,6,9,11-tetrahydro-6,11a-(epiminoethano)naphtho[2,1-f]indazole-2,6a(7H)-diol (Compound 46)

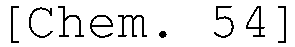

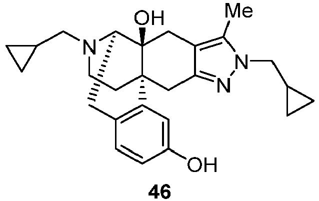

A reaction was carried out using the compound 41 (21.6 mg, 0.050 mmol), and a 1.0 M dichloromethane solution of boron tribromide (0.2 mL, 0.2 mmol) in the same manner as that of Example 11, and thus, the title compound 46 (15.8 mg, 76%) as white oil was obtained.

¹H-NMR (400 MHz, CDCl₃) δ (ppm): 0.12 - 0.24 (m, 3H), 0.27 - 0.33 (m, 1H), 0.36 - 0.47 (m, 2H), 0.50 - 0.60 (m, 2H), 0.81 - 0.96 (m, 2H), 1.04 - 1.16 (m, 1H), 1.30 - 1.38 (m, 1H), 2.01 (s, 3H), 2.13 - 2.30 (m, 2H), 2.41 (d, J = 15.9 Hz, 1H), 2.44 (d, J = 6, 5 Hz, 2H), 2, 51 (d, J = 15.9 Hz, 1H), 2.68 (br d, J = 6.3 Hz, 1H), 2.92 (dd, J = 6.7, 18.4 Hz, 1H), 3.01 (d, J = 16.1 Hz, 1H), 3.12 (d, J = 18.4 Hz, 1H), 3.24 (br d, J = 6.7 Hz, 1H), 3.38 (d, J = 16.1 Hz, 1H), 3.74 (dd, J = 6.8, 14.6 Hz, 1H), 3.77 (dd, J = 6.8, 14.6 Hz, 1H), 6.63 (dd, J = 2.2, 8.3 Hz, 1H), 6.94 (d, J = 8.3 Hz, 1H), 6.96 (d, J = 2.2 Hz, 1H), 1H (OH) was not observed.

### Example 34

### Synthesis of (6R,6aS,11aR)-14-(cyclopropylmethyl)-2-methoxy-8-methyl-10-(pyridin-3-yl)-5,6,10,11-tetrahydro-6,11a-(epiminoethano)naphtho[2,1-f]indazol-6a(7H)-ol (Compound 47) and (6R,6aS,11aR)-14-(cyclopropylmethyl)-2-methoxy-8-methyl-9-(pyridin-3-yl)-5,6,9,11-tetrahydro-6,11a-(epiminoethano)naphtho[2,1-f]indazol-6a(7H)-ol (Compound 48)

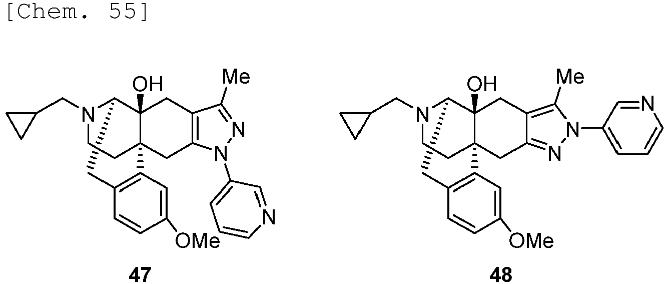

Under an argon atmosphere, copper(I) iodide (22.5 mg, 0.12 mmol), N,N'-dimethylethylenediamine (23 µL, 0.21 mmol), potassium carbonate (59.5 mg, 0.43 mmol), and 3-bromopyridine (21.0 µL, 0.22 mmol) were added to an N,N-dimethylformamide (0.7 mL) solution of the equilibrium mixture (85.8 mg, 0.22 mmol) of the compound 18 and the compound 19, and heating and reflux were performed for 24 hours. After cooling, the produced insoluble matters were removed by filtration through celite, a sodium carbonate aqueous solution was added to the filtrate, and extraction was performed with diethyl ether. After an organic layer was washed with saturated brine and dried with anhydrous sodium sulfate, concentration was performed under reduced pressure. The obtained crude product was purified by silica gel column chromatography, and thus, the title compound 47 (7.4 mg, 7%) as yellow oil and the title compound 48 (5.1 mg, 5%) as yellow oil were obtained.

### Compound 47

¹H-NMR (400 MHz, CDCl₃) δ (ppm): 0.11 - 0.20 (m, 2H), 0.52 - 0.60 (m, 2H), 0.84 - 0.95 (m, 1H), 1.30 - 1.36 (m, 1H), 2.10 (s, 3H), 2.14 - 2.28 (m, 2H), 2.43 (d, J = 6.4 Hz, 2H), 2.46 - 2.56 (m, 2H), 2.64 - 2.70 (m, 1H), 2.97 (dd, J = 6.4, 18.6 Hz, 1H), 3.12 - 3.16 (m, 1H), 3.12 (d, J = 18.6 Hz, 1H)3.24 - 3.31 (m, 2H), 3.58 (s, 3H)6.45 (d, J = 2.6 Hz, 1H), 6.64 (dd, J = 2.6, 8.4 Hz, 1H), 7.00 (d, J = 8.4 Hz, 1H), 7.43 (br dd, J = 5.1, 7.8 Hz, 1H), 7.90 (ddd, J = 1.5, 2.5, 8.3 Hz, 1H), 8.57 (br s, 1H), 8.86 (br s, 1H), 1H (OH) was not observed.

### Compound 48

¹H-NMR (400 MHz, CDCl₃) δ (ppm): 0.10 - 0.21 (m, 2H), 0.51 - 0.62 (m, 2H), 0.82 - 0.97 (m, 1H), 1.36 - 1.42 (m, 1H), 2.08 - 2.30 (m, 3H), 2.13 (s, 3H), 2.40 - 2.48 (m, 2H), 2.50 - 2.60 (m, 1H), 2.53 (d, J = 18.0 Hz, 1H), 2.04 - 2.72 (m, 1H), 2.97 (dd, J = 6.7, 18.6 Hz, 1H), 3.08 (d, J = 16.6 Hz, 1H)3.16 (d, J = 12.5 Hz, 1H), 3.22 - 3.31 (m, 1H), 3.39 (d, J = 16.6 Hz, 1H), 3.72 (s, 3H)6.68 (dd, J = 2.6, 8.4 Hz, 1H), 6.88 (d, J = 2.6 Hz, 1H), 7.02 (d, J = 8.4 Hz, 1H), 7.36 (dd, J = 5.0, 7.0 Hz, 1H), 8.66 (ddd, J = 1.3, 2.1, 8.1 Hz, 1H), 8.54 (br s, 1H), 8.68 (br s, 1H).

### Example 35

### Synthesis of (6R,6aS,11aR)-14-(cyclopropylmethyl)-8-methyl-10-(pyridin-3-yl)-5,6,10,11-tetrahydro-6,11a-(epiminoethano)naphtho[2,1-f]indazole-2,6a(7H)-diol (Compound 49)

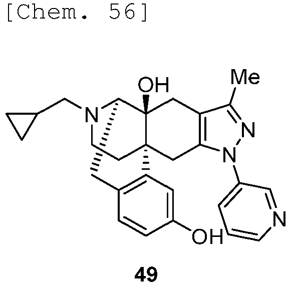

A reaction was carried out using the compound 47 (7.4 mg, 0.016 mmol), and a 1.0 M dichloromethane solution of boron tribromide (0.1 mL, 0.1 mmol) in the same manner as that of Example 11, and thus, the title compound 49 (3.8 mg, 53%) as yellow-white amorphous was obtained.

¹H-NMR (400 MHz, CDCl₃) δ (ppm): 0.12 - 0.20 (m, 2H), 0.52 - 0.60 (m, 2H), 0.82 - 0.95 (m, 1H), 1.31 - 1.36 (m, 1H), 2.10 (s, 3H), 2.14 - 2.24 (m, 2H), 2.44 (d, J = 6.4 Hz, 1H), 2.48 (d, J = 16.2 Hz, 1H), 2.56 (d, J = 16.2 Hz, 1H), 2.62 - 2.72 (m, 2H), 2.96 (dd, J = 6.4, 18.6 Hz, 1H), 3.07 - 3.13 (m, 1H), 3.12 (d, J = 18.6 Hz, 1H), 3.23 (d, J = 16.3 Hz, 1H), 3.26 - 3.33 (m, 1H), 6.53 (d, J = 2.4 Hz, 1H), 6.61 (dd, J = 2.4, 8.2 Hz, 1H), 6.95 (d, J = 8.2 Hz, 1H), 7.40 (br s, 1H), 7.94 (br d, J = 8.0 Hz, 1H), 8.42 (br s, 1H), 8.79 (br s, 1H), 2H (OH × 2) was not observed.

### Example 36

### Synthesis of (6R,6aS,11aR)-14-(cyclopropylmethyl)-2-methoxy-8-methyl-10-(thiazol-2-yl)-5,6,10,11-tetrahydro-6,11a-(epiminoethano)naphtho[2,1-f]indazol-6a(7H)-ol (Compound 50) and (6R,6aS,11aR)-14-(cyclopropylmethyl)-2-methoxy-8-methyl-9-(thiazol-2-yl)-5,6,9,11-tetrahydro-6,11a-(epiminoethano)naphtho[2,1-f]indazol-6a(7H)-ol (Compound 50a)

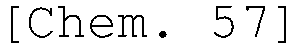

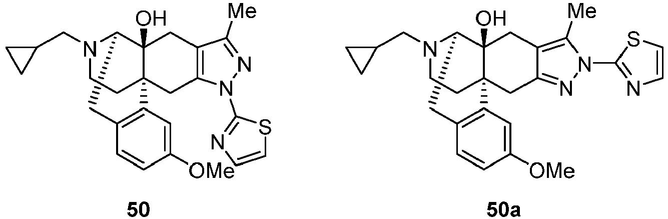

Under an argon atmosphere, a tetrahydrofuran (1.5 mL) solution of the equilibrium mixture (42.7 mg, 0.10 mmol) of the compound 18 and the compound 19 was added dropwise to a tetrahydrofuran (1.0 mL) suspension of sodium hydride (55% in oil, 26.7 mg, 0.61 mmol) at 0°C, and stirring was performed at room temperature for 1.5 hours. 2-Chlorothiazole (43 µL, 0.50 mmol) was added dropwise, and heating and reflux were performed for 15.5 hours. After cooling, a saturated sodium bicarbonate aqueous solution was added to the reaction solution, and extraction was performed with ethyl acetate. After an organic layer was washed with saturated brine and dried with anhydrous sodium sulfate, concentration was performed under reduced pressure. The obtained crude product was purified by silica gel column chromatography, and thus, the title compound 50 (7.1 mg, 14%) as yellow oil was obtained. The production of the position isomer 50a was also confirmed, but could not be isolated.

¹H-NMR (400 MHz, CDCl₃) δ (ppm): 0.10 - 0.20 (m, 2H), 0.50 - 0.61 (m, 2H), 0.83 - 0.95 (m, 1H), 1.39 - 1.46 (m, 1H), 2.08 (s, 3H), 2.13 - 2.32 (m, 2H), 2.39 - 2.52 (m, 4H), 2.63 - 2.73 (m, 1H), 2.95 (dd, J = 6.8, 18.6 Hz, 1H), 3.14 (d, J = 18.6 Hz, 1H), 3.16 (d, J = 17.6 Hz, 1H), 3.25 (d, J = 6.6 Hz, 1H), 3.62 (s, 3H), 4.27 (d, J = 17.6 Hz, 1H), 6.65 (dd, J = 2.6, 8.4 Hz, 1H), 6.82 (d, J = 2.6 Hz, 1H), 6.96 - 7.02 (m, 2H), 7.54 (d, 1H), 1H (OH) was not observed.

### Example 37

### Synthesis of (6R,6aS,11aR)-14-(cyclopropylmethyl)-8-methyl-10-(thiazol-2-yl)-5,6,10,11-tetrahydro-6,11a-(epiminoethano)naphtho[2,1-f]indazole-2,6a(7H)-diol (Compound 51)

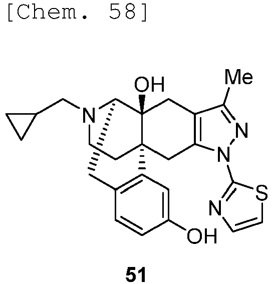

A reaction was carried out using the compound 50 (7.1 mg, 0.015 mmol), and a 1.0 M dichloromethane solution of boron tribromide (0.08 mL, 0.08 mmol) in the same manner as that of Example 11, and thus, the title compound 51 (5.0 mg, 73%) as a white solid was obtained.

¹H-NMR (400 MHz, CD₃OD) δ (ppm): 0.15 - 0.26 (m, 2H), 0.50 - 0.62 (m, 2H), 0.88 - 1.00 (m, 1H), 1.38 - 1.42 (m, 1H), 2.07 (s, 3H), 2.24 - 2.32 (m, 2H), 2.41 (d, J = 16.4 Hz, 1H), 2.51 (d, J = 6.4 Hz, 2H), 2.56 (dd, J = 1.7, 16.4 Hz, 1H), 2.68 - 2.74 (m, 1H), 2.99 (dd, J = 6.6, 18.6 Hz, 1H), 3.06 (d, J = 17.8 Hz, 1H), 3.18 (d, J = 18.6 Hz, 1H), 3.37 (d, J = 6.6 Hz, 1H), 4.18 (d, J = 17.8 Hz, 1H), 6.55 (dd, J = 2.4, 8.3 Hz, 1H), 6.66 (d, J = 2.4 Hz, 1H), 6.95 (d, J = 8.3 Hz, 1H), 7.24 (dd, J = 1.9, 3.6 Hz, 1H), 7.60 (dd, J = 1.1, 3.6 Hz, 1H), 2H (OH × 2) was not observed.

### Reference Example 3

### Synthesis of (4bR,8aS,9R)-3-(benzyloxy)-11-(cyclopropylmethyl)-8a-hydroxy-8,8a,9,10-tetrahydro-5H-9,4b-(epiminoethano)phenanthren-6(7H)-one (Compound 52)

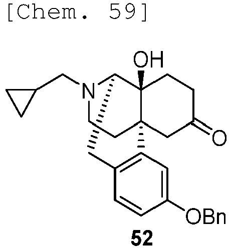

Under an argon atmosphere, benzyl bromide (267 mg, 1.58 mmol) was added to an acetonitrile (10 mL) suspension of (4bR,8aS,9R) - 11 - (cyclopropylmethyl) - 3,8a - dihydroxy - 8,8a, 9,10 - tetrahydro - 5H - 9,4b - (epiminoethano)phenanthren - 6(7H) - one (Compound 53) (synthesized by a method described in Bioorganic & Medicinal Chemistry 2016, 24, 2199-2205) (344 mg, 1.05 mmol) and potassium carbonate (363 mg, 2.63 mmol), and stirring was performed at room temperature for 45 hours. Water was added to the reaction solution, and extraction was performed with ethyl acetate. After an organic layer was washed with saturated brine and dried with sodium sulfate, concentration was performed under reduced pressure. The obtained crude product was purified by silica gel chromatography, and thus the title compound 52 (392 mg, 89%) as colorless amorphous was obtained.

¹H-NMR (400 MHz, CDCl₃) δ (ppm): 0.10 - 0.20 (m, 2H), 0.49 - 0.59 (m, 2H), 0.82 - 0.92 (m, 1H), 1.11 - 1.21 (m, 1H), 1.80 (m, 1H), 1.87 (ddd, J = 5.1, 13.2, 13.2 Hz, 1H), 2.05 - 2.20 (m, 3H), 2.39 (d, J = 6.5 Hz, 12.8 Hz, 1H), 2.40 (dd, J = 6.7, 12.8 Hz, 1H), 2.54 - 2.62 (m, 1H), 2.70 - 2.85 (m, 3H), 3.05 (d, J = 13.9 Hz, 1H), 3.06 (d, J = 18.7 Hz, 1H), 3.13 (d, J = 6.4 Hz, 1H), 4.67 - 4.84 (br s, 1H), 4.98 (d, J = 11.5 Hz, 1H), 5.02 (d, J = 11.5 Hz, 1H), 6.75 (dd, J = 2.6, 8.4 Hz, 1H), 6.90 (d, J = 2.6 Hz, 1H), 6.98 (d, J = 8.4 Hz, 1H), 7.28 - 7.34 (m, 1H), 7.35 - 7.40 (m, 2H), 7.41 - 7.46 (m, 2H).

### Reference Example 4

### Synthesis of (4bR,8aS,9R)-3-(benzyloxy)-11-(cyclopropylmethyl)-6-oxo-5,6,7,8,9,10-hexahydro-8aH-9,4b-(epiminoethano)phenanthren-8a-yl acetate (Compound 54)

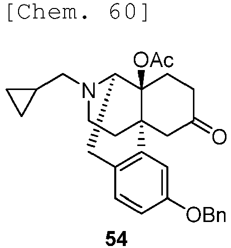

A reaction was carried out using the compound 52 (649.7 mg, 1.6 mmol) in the same manner as that of Reference Example 1, and thus, the title compound 54 (637.4 mg, 89%) as white oil was obtained.

¹H-NMR (400 MHz, CDCl₃) δ (ppm): 0.03 - 0.14 (m, 2H), 0.43 - 0.55 (m, 2H), 0.73 - 0.85 (m, 1H), 1.10 - 1.19 (m, 1H), 1.88 (ddd, J = 5.3, 14.0, 14.0 Hz, 1H), 2.08 (ddd, J = 3.3, 12.2, 12.2 Hz, 1H), 2.12 - 2.25 (m, 1H), 2.20 (s, 3H), 2.28 - 2.39 (m, 3H), 2.44 (ddd, J = 7.0, 14.4, 14.4 Hz, 1H), 2.59 (dd, J = 3.7, 11.9 Hz, 1H), 2.66 (dd, J = 6.3, 18.4 Hz, 1H), 2.83 (ddd, J = 1.6, 7.0, 7.0 Hz, 1H), 2.86 - 2.93 (m, 1H), 2.98 (d, J = 14.8 Hz, 1H), 3.08 (d, J = 18.4 Hz, 1H), 3.00 (d, J = 6.0 Hz, 1H), 5.00 (dd, J = 1.5, 11, 5 Hz, 2H), 6.77 (dd, J = 2.5, 8.4 Hz, 1H), 6.89 (d, J = 2.5 Hz, 1H), 7.00 (d, J = 8.4 Hz, 1H), 7.29 - 7.48 (m, 5H).

### Reference Example 5

### Synthesis of 1-((4bR,8aS,9R)-3-(benzyloxy)-11-(cyclopropylmethyl)-6,8a-dihydroxy-8,8a,9,10-tetrahydro-5H-9,4b-(epiminoethano)phenanthren-7-yl)ethan-1-one (Compound 55)

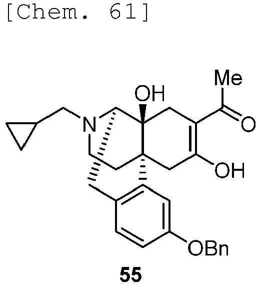

A reaction was carried out using diisopropylamine (260 µL, 1.8 mmol), a 1.5 M hexane solution of n-butyllithium (1.26 mL, 1.9 mmol), and the compound 54 (637.4 mg, 1.39 mmol) in the same manner as that of Reference Example 2, and thus, the title compound 55 (253.4 mg, 45%) as yellow-white amorphous was obtained.

¹H-NMR (400 MHz, CDCl₃) δ (ppm): 0.08 - 0.19 (m, 2H), 0.48 - 0.60 (m, 2H), 0.81 - 0.92 (m, 1H), 1.20 - 1.27 (m, 1H), 1.96 (s, 3H), 2.00 - 2.14 (m, 2H), 2.28 (d, J = 15.5 Hz, 1H), 2.34 - 2.41 (m, 3H), 2.57 - 2.64 (m, 1H), 2.81 (d, J = 18.1 Hz, 1H), 2.91 (dd, J = 6.9, 18.1 Hz, 1H), 2.94 (d, J = 18.1 Hz, 1H), 3.01 (d, J = 18.6 Hz, 1H), 3.19 (d, J = 6.7 Hz, 1H), 4.99 (s, 2H), 6.78 (dd, J = 2.5, 8.4 Hz, 1H), 6.87 (d, J = 2.5 Hz, 1H), 7.02 (d, J = 8.4 Hz, 1H), 7.28 - 7.46 (m, 5H), 15.9 (s, 1H), 1H (OH) was not observed.

### Example 38

### Synthesis of an equilibrium mixture of (6R,6aS,11aR)-2-(benzyloxy)-14-(cyclopropylmethyl)-8-methyl-5,6,9,11-tetrahydro-6,11a-(epiminoethano)naphtho[2,1-f]indazol-6a(7H)-ol (Compound 56) and (6R,6aS,11aR)-2-(benzyloxy)-14-(cyclopropylmethyl)-8-methyl-5,6,10,11-tetrahydro-6,11a-(epiminoethano)naphtho[2,1-f]indazol-6a(7H)-ol (Compound 57)

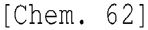

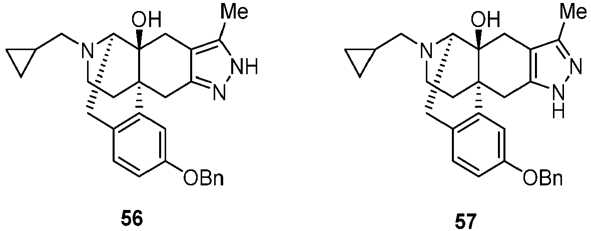

A reaction was carried out using the compound 55 (253.4 mg, 0.55 mmol), hydrazine hydrochloride (58.6 mg, 0.86 mmol), and methanesulfonic acid (195 µL, 3.0 mmol) in the same manner as that of Example 1, and thus, an equilibrium mixture (231.3 mg, 92%) of the title compounds 56 and 57 as yellow amorphous was obtained.

¹H-NMR (400 MHz, CDCl₃) δ (ppm): 0.06 - 0.20 (m, 2H), 0.48 - 0.60 (m, 2H), 0.82 - 0.91 (m, 1H), 1.28 - 1.36 (m, 1H), 2.02 (s, 3H), 2.14 - 2.20 (m, 2H), 2.40 (d, J = 6.5 Hz, 1H), 2.42 - 2.52 (m, 2H), 2.59 - 2.69 (m, 1H), 2.90 - 2.98 (m, 1H), 2.97 (d, J = 15.5 Hz, 1H), 3.12 (d, J = 18.5 Hz, 1H), 3.19 - 3.22 (m, 1H), 3.23 (d, J = 8.2 Hz, 1H), 4.92 (s, 2H), 6.71 (dd, J = 2.5, 8.4 Hz, 1H), 6.86 (d, J = 2.5 Hz, 1H), 6.98 (d, J = 8.4 Hz, 1H), 7.27 - 7.40 (m, 5H), 15.9 (s, 1H), 2H (NH, OH) was not observed.

### Example 39

### Synthesis of (6R,6aS,11aR)-2-(benzyloxy)-14-(cyclopropylmethyl)-10-(2-methoxyethyl)-8-methyl-5,6,10,11-tetrahydro-6,11a-(epiminoethano)naphtho[2,1-f]indazol-6a(7H)-ol (Compound 58)

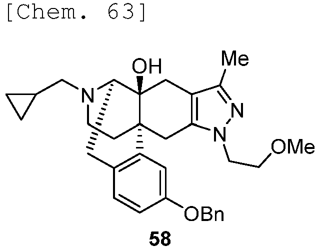

A reaction was carried out using the equilibrium mixture (215.0 mg, 0.47 mmol) of the compound 56 and the compound 57, sodium hydride (55% in oil, 98.7 mg, 2.26 mmol), and 1-bromo-2-methoxyethane (310 µL, 3.3 mmol) in the same manner as that of Example 26, and thus, the title compound 58 (84.4 mg, 35%) as yellow-white amorphous was obtained.

¹H-NMR (400 MHz, CDCl₃) δ (ppm): 0.09 - 0.20 (m, 2H), 0.44 - 0.60 (m, 2H), 0.80 - 0.96 (m, 1H), 1.15 - 1.23 (m, 1H), 2.04 (s, 3H), 2.06 - 2.24 (m, 3H), 2.28 - 2.56 (m, 4H), 2.68 (dd, J = 6.2, 18.3 Hz, 1H), 3.05 (d, J = 16.2 Hz, 1H), 3.11 - 3.20 (m, 2H), 3.27 (s, 3H), 3.42 - 3.60 (m, 4H), 3.82 - 3.91 (m, 1H), 4.93 (s, 2H), 6.71 (dd, J = 2.5, 8.4 Hz, 1H), 6.87 (d, J = 2.5 Hz, 1H), 6.97 (d, J = 8.4 Hz, 1H), 7.27 - 7.41 (m, 5H), 1H (OH) was not observed.

### Example 40

### Synthesis of (6R,6aS,11aR)-14-(cyclopropylmethyl)-10-(2-methoxyethyl)-8-methyl-5,6,10,11-tetrahydro-6,11a-(epiminoethano)naphtho[2,1-f]indazole-2,6a(7H)-diol (Compound 59)

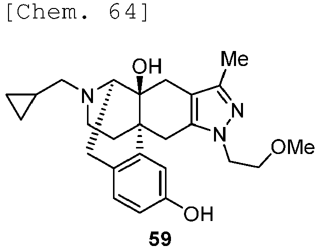

Acetic acid (5 µL, 0.0087 mmol) and 10% palladium/carbon (17.2 mg) were added to a methanol (2 mL) solution of the compound 58 (34.6 mg, 0.067 mmol), and stirring was performed under a hydrogen atmosphere at room temperature for 16 hours. After filtration through celite, the filtrate was concentrated under reduced pressure, the obtained crude product was produced by silica gel column chromatography, and thus, the title compound 59 (24.9 mg, 87%) as a white solid was obtained.

¹H-NMR (400 MHz, CD₃OD) δ (ppm): 0.12 - 0.26 (m, 2H), 0.47 - 0.63 (m, 2H), 0.86 - 0.96 (m, 1H), 1.16 - 1.24 (m, 1H), 2.02 (s, 3H), 2.12 - 2.24 (m, 1H), 2.21 (d, J = 16.3 Hz, 1H), 2.34 - 2.66 (m, 4H), 2.71 (d, J = 16.3 Hz, 1H), 2.79 (dd, J = 6.3, 18.3 Hz, 1H), 2.93 (d, J = 16.2 Hz, 1H), 3.16 (d, J = 16.2 Hz, 1H), 3.18 (d, J = 18.3 Hz, 1H), 3.24 (s, 3H), 3.46 - 3.64 (m, 3H), 3.68 - 3.76 (m, 1H), 3.81 - 3.88 (m, 1H), 6.52 (dd, J = 2.2, 8.3 Hz, 1H), 6.67 (d, J = 2.2 Hz, 1H), 6.91 (d, J = 8.3 Hz, 1H), 2H (OH × 2) was not observed.

### Example 41

### Synthesis of (6R,6aS,11aR)-2-methoxy-8,14-dimethyl-10-phenyl-5,6,10,11-tetrahydro-6,11a-(epiminoethano)naphtho[2,1-f]indazol-6a(7H)-ol (Compound 60)

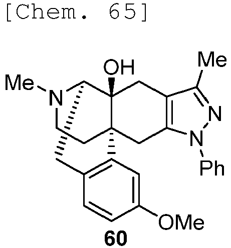

Under an argon atmosphere, 2,2,2-trichloroethyl chloroformate (115 µL, 0.84 mmol) and potassium carbonate (91.3 mg, 0.66 mmol) were added to a 1,1,2,2-tetrachloroethane (2.5 mL) solution of the compound 5 (123.3 mg, 0.27 mmol), and heating and reflux were performed for 12 hours. After cooling, a saturated sodium bicarbonate aqueous solution was added to the reaction solution, and extraction was performed with chloroform. After an organic layer was washed with saturated brine and dried with anhydrous sodium sulfate, concentration was performed under reduced pressure. The obtained crude product was crudely purified by silica gel chromatography and was used as it was for the next reaction. Under an argon atmosphere, a tetrahydrofuran solution of the crude product (52.6 mg) was added dropwise to a tetrahydrofuran (3.0 mL) suspension of lithium aluminum hydride (15.0 mg, 0.4 mmol) at 0°C, and stirring was performed at room temperature for 1 hour. Sodium sulfate decahydrate was added under ice cooling, stirring was performed for 16 hours, and then, filtration through celite was performed. After the filtrate was concentrated under reduced pressure, the obtained crude product was purified by preparative TLC, and thus, the title compound 60 (24.4 mg, 66%) as yellow oil was obtained.

¹H-NMR (400 MHz, CDCl₃) δ (ppm): 1.23 - 1.32 (m, 1H), 2.09 (s, 3H), 2.14 - 2.26 (m, 2H), 2.37 - 2.56 (m, 3H), 2.42 (s, 3H), 2.91 - 3.00 (m, 2H), 3.10 (d, J = 15.4 Hz, 1H), 3.16 - 3.28 (m, 2H), 3.57 (s, 3H), 6.46 (d, J = 2.6 Hz, 1H), 6.64 (dd, J = 2.6, 8.4 Hz, 1H), 7.00 (d, J = 8.4 Hz, 1H), 7.28 - 7.35 (m, 1H), 7.43 - 7.53 (m, 4H), 1H (OH) was not observed.

### Example 42

### Synthesis of (6R,6aS,11aR)-8,14-dimethyl-10-phenyl-5,6,10,11-tetrahydro-6,11a-(epiminoethano)naphtho[2,1-f]indazole-2,6a(7H)-diol (Compound 61)

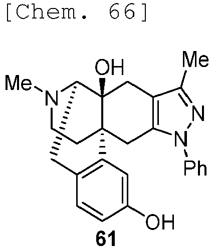

A reaction was carried out using the compound 60 (24.8 mg, 0.06 mmol), and a 1.0 M dichloromethane solution of boron tribromide (0.2 mL, 0.2 mmol) in the same manner as that of Example 11, and thus, the title compound 61 (9.2 mg, 38%) as a white solid was obtained.

¹H-NMR (400 MHz, CDCl₃) δ (ppm): 1.24 - 1.32 (m, 1H), 2.08 (s, 3H), 2.13 - 2.25 (m, 2H), 2.41 (s, 3H), 2.36 - 2.58 (m, 3H), 2.89 - 2.98 (m, 2H), 3.09 (d, J = 16.4 Hz, 1H), 3.14 - 3.22 (m, 1H), 3.22 (d, J = 16.4 Hz, 1H), 6.41 (d, J = 2.5 Hz, 1H), 6.44 (dd, J = 2.5, 8.2 Hz, 1H), 6.94 (J = 8.2 Hz, 1H), 7.27 - 7.32 (m, 1H), 7.47 - 7.53 (m, 4H), 2H (OH × 2) was not observed.

### Reference Example 6

### Synthesis of (4bR,8aS,9R)-3-methoxy-11-methyl-6-oxo-5,6,7,8,9,10-hexahydro-8aH-9,4b-(epiminoethano)phenanthren-8a-yl acetate (Compound 62)

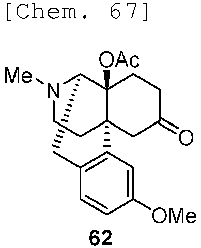

A reaction was carried out using (4bR,8aS,9R)-8a-hydroxy-3-methoxy-11-methyl-8,8a,9,10-tetrahydro-5H-9,4b-(epiminoethano)phenanthren-6(7H)-one (Compound 63) (synthesized by a method described in Bioorganic & Medicinal Chemistry Letters 2010, 20, 6302-6305) (267.3 mg, 0.89 mmol) in the same manner as that of Reference Example 1, and thus, the title compound 62 (238.5 mg, 78 %) as yellow oil was obtained.

¹H-NMR (400 MHz, CDCl₃) δ (ppm): 1.11 - 1.19 (m, 1H), 1.88 (ddd, J = 5.3, 14.0, 14.0 Hz, 1H), 2.03 - 2.12 (m, 2H), 2.20 (s, 3H), 2.28 - 2.45 (m, 3H), 2.33 (s, 3H), 6.64 (dd, J = 6.0, 18.5 Hz, 1H), 2.82 (ddd, J = 1.7, 6.8, 14.8 Hz, 1H), 2.89 (dd, J = 1.7, 14.8 Hz, 1H), 2.97 (d, J = 14.8 Hz, 1H), 3.19 (d, J = 18.5 Hz, 1H), 3.77 (s, 3H), 4.09 (d, J = 6.0 Hz, 1H), 6.71 (dd, J = 2.6, 8.4 Hz, 1H), 6.78 (d, J = 2.6 Hz, 1H), 7.02 (d, J = 8.4 Hz, 1H).

### Reference Example 7

### Synthesis of 1-((4bR,8aS,9R)-6,8a-dihydroxy-3-methoxy-11-methyl-8,8a,9,10-tetrahydro-5H-9,4b-(epiminoethano)phenanthren-7-yl)ethan-1-one (Compound 64)

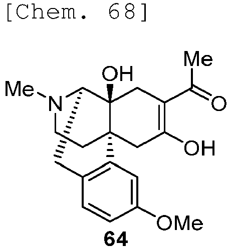

A reaction was carried out using diisopropylamine (85 µL, 0.60 mmol), a 2.6 M hexane solution of n-butyllithium (220 µL, 0.57 mmol), and the compound 62 (186 mmg, 0.54 mmol) in the same manner as that of Reference Example 2, and thus, the title compound 64 (121.7 mg, 66%) as yellow amorphous was obtained.

¹H-NMR (400 MHz, CDCl₃) δ (ppm): 1.22 - 1.28 (m, 1H), 1.95 (s, 3H), 2.04 (ddd, J = 5.1, 12.6, 12.6 Hz, 1H), 2.10 - 2.19 (m, 2H), 2.27 (d, J = 18.2 Hz, 1H), 2.34 - 2.37 (m, 1H), 2.39 (m, 3H), 2.84 - 2.95 (m, 4H), 3.20 (d, J = 17.0 Hz, 1H), 3.76 (s, 3H), 6.72 (dd, J = 2.6, 8.4 Hz, 1H), 6.78 (d, J = 2.6 Hz, 1H), 7.04 (d, J = 8.4 Hz, 1H), 15.9 (s, 1H), 1H (OH) was not observed.

### Example 43

### Synthesis of (6R,6aS,11aR)-10-benzyl-2-methoxy-8,14-dimethyl-5,6,10,11-tetrahydro-6,11a-(epiminoethano)naphtho[2,1-f]indazol-6a(7H)-ol (Compound 65) and (6R,6aS,11aR)-9-benzyl-2-methoxy-8,14-dimethyl-5,6,9,11-tetrahydro-6,11a-(epiminoethano)naphtho[2,1-f]indazol-6a(7H)-ol (Compound 65a)

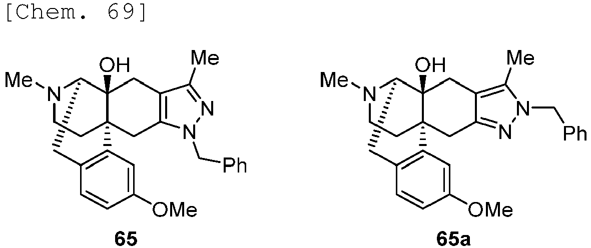

A reaction was carried out using the compound 64 (58.5 mg, 0.17 mmol), benzylhydrazine hydrochloride (38.1 mg, 0.35 mmol), and methanesulfonic acid (22 µL, 0.34 mmol) in the same manner as that of Example 1, and thus, the title compound 65 (15.0 mg, 21%) as a yellow-white solid was obtained. The production of the position isomer 65a was also confirmed, but could not be isolated.

### Compound 65

¹H-NMR (400 MHz, CDCl₃) δ (ppm): 1.21 - 1.27 (m, 1H), 2.05 (s, 3H), 2.10 - 2.19 (m, 2H), 2.28 - 2.43 (m, 2H), 2.39 (s, 3H), 2.49 (dd, J = 1.1, 16.0 Hz, 1H), 2.79 (d, J = 16.2 Hz, 1H), 2.88 - 2.97 (m, 2H), 3.07 (d, J = 16.2 Hz, 1H), 3.14 - 3.22 (m, 1H), 3.54 (s, 3H), 5.20 (d, J = 16.2 Hz, 1H), 5.28 (d, J = 16.1 Hz, 1H), 6.38 (d, J = 2.6 Hz, 1H), 6.64 (dd, J = 2.6, 8.4 Hz, 1H), 7.00 (d, J = 8.4 Hz, 1H), 7.10 - 7.16 (m, 2H), 7.25 - 7.34 (m, 3H), 1H (OH) was not observed.

### Example 44

### Synthesis of (6R,6aS,11aR)-10-benzyl-8,14-dimethyl-5,6,10,11-tetrahydro-6,11a-(epiminoethano)naphtho[2,1-f]indazole-2,6a(7H)-diol (Compound 66)

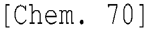

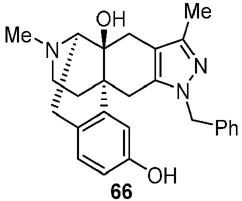

A reaction was carried out using the compound 65 (15.0 mg, 0.035 mmol), and a 1.0 M dichloromethane solution of boron tribromide (0.1 mL, 0.1 mmol) in the same manner as that of Example 11, and thus, the title compound 66 (4.4 mg, 30%) as colorless oil was obtained.

¹H-NMR (400 MHz, CDCl₃) δ (ppm): 1.10 - 1.19 (m, 1H), 2.05 (s, 3H), 2.07 - 2.20 (m, 2H), 2.32 - 2.46 (m, 3H), 2.39 (s, 3H), 2.80 (d, J = 16.6 Hz, 1H), 2.84 - 2.89 (m, 1H), 1.90 - 2.94 (m, 2H), 3.14 (d, J = 18.0 Hz, 1H), 5.12 (d, J = 15.9 Hz, 1H), 5.36 (d, J = 15.9 Hz, 1H), 5.58 (d, J = 2.3 Hz, 1H), 6.53 (dd, J = 2.3, 8.3 Hz, 1H), 6.90 (d, J = 8.3 Hz, 1H), 7.18 - 7.23 (m, 2H), 7.31 - 7.41 (m, 3H), 2H (OH × 2) was not observed.

### Example 45

### Synthesis of (6R,6aS,11aR)-2-(benzyloxy)-14-(cyclopropylmethyl)-6a-hydroxy-9-phenyl-6,6a,7,7a,9,11-hexahydro-6,11a-(epiminoethano)naphtho[2,1-f]indazol-8(5H)-one (Compound 67) and (6R,6aS,11aR)-14-(cyclopropylmethyl)-2,6a-dihydroxy-9-phenyl-6,6a,7,9,10,11-hexahydro-6,11a-(epiminoethano)naphtho[2,1-f]indazol-8(5H)-one (Compound 68)

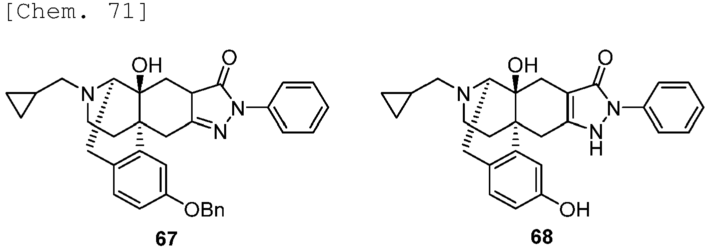

Under an argon atmosphere, a 5.1 M copper(II) chloride aqueous solution (190 µL) was added to an ethyl acetate (5 mL) solution of the compound 52 (411.6 mg, 0.99 mmol) and phenyl isocyanate (140 mg, 1.18 mmol), and stirring was performed at room temperature for 10 hours. A saturated sodium carbonate aqueous solution was added to the reaction solution, and extraction was performed with ethyl acetate. After an organic layer was washed with saturated brine and dried with anhydrous sodium sulfate, concentration was performed under reduced pressure, and thus, a crude product (584.4 mg) was obtained. A 6 M potassium hydroxide aqueous solution (700 µL) was added to a 2-propanol (10 mL) solution of the obtained crude purified product, and stirring was performed under an argon atmosphere at 80°C for 7 hours. Water was added to the reaction solution, and extraction was performed with ethyl acetate. After an organic layer was washed with saturated brine and dried with anhydrous sodium sulfate, concentration was performed under reduced pressure. The obtained crude product was crudely purified by silica gel chromatography, and thus, a crude product (473.3 mg) was obtained. The obtained crude product and phenylhydrazine hydrochloride (255.1 mg, 1.764 mmol) were suspended in acetic acid (8 mL), and heating and reflux were performed under an argon atmosphere for 5 hours. After the reaction solution was concentrated under reduced pressure, a saturated sodium carbonate aqueous solution was added thereto, and extraction was performed with ethyl acetate. After an organic layer was washed with saturated brine and dried with anhydrous sodium sulfate, concentration was performed under reduced pressure. The obtained crude product was purified by silica gel chromatography, and thus, the title compound 67 (337.8 mg, 64%) and the title compound 68 (14.8 mg, 3.4%) were obtained.

### Compound 67

¹H-NMR (400 MHz, CDCl₃) δ (ppm): 0.1 - 0.16 (m, 2H), 0.5 - 0.57 (m, 2H), 0.81 - 0.92 (m, 1H), 1.26 - 1.34 (m, 1H), 1.63 (dd, J = 12.6, 12.6 Hz, 1H), 2.09 - 2.26 (m, 3H), 2.38 (dd, J = 6.5, 12.6 Hz, 1H), 2.40 (dd, J = 6.5, 12.6 Hz, 1H), 2.59 - 2.69 (m, 1H), 2.69 (dd, J = 6.5, 18.7 Hz, 1H), 3.00 (d, J = 14.1 Hz, 1H), 3.05 (d, J = 18.7 Hz, 1H), 3.13 (d, J = 6.4 Hz, 1H), 3.16 (d, J = 14.1 Hz, 1H), 3.75 (dd, J = 7.8, 12.4 Hz, 1H), 5.02 (s, 2H), 6.72 (dd, J = 2.5, 8.4 Hz, 1H), 6.95 (d, J = 8.4 Hz, 1H), 7.02 (d, J = 2.5 Hz, 1H), 7.06 - 7.16 (m, 1H), 7.27 - 7.36 (m, 5H), 7.38 - 7.44 (m, 2H), 7.80 - 7.86 (m, 2H), 1H (OH) was not observed.

### Compound 68

¹H-NMR (400 MHz, CD₃OD) δ (ppm): 0.34 - 0.48 (m, 2H), 0.66 - 0.82 (m, 2H), 1.02 - 1.14 (m, 1H), 1.40 - 1.50 (m, 1H), 2.38 - 2.51 (m, 1H), 2.41 (d, J = 16.4 Hz, 1H), 2.52 (d, J = 16.4 Hz, 1H), 2.58 - 2.72 (m, 1H), 2.78 - 2.88 (m, 1H), 2.92 (d, J = 16.6 Hz, 1H), 2.92 - 3.00 (m, 1H), 3.05 - 3.12 (m, 1H), 3.19 (d, J = 16.6 Hz, 1H), 3.26 - 3.33 (m, 2H), 3.86 (br s, 1H), 6.51 (dd, J = 2.5, 8.4 Hz, 1H), 6.77 (d, J = 2.4 Hz, 1H), 7.01 (d, J = 8.4 Hz, 1H), 7.12 - 7.20 (m, 1H), 7.32 - 7.41 (m, 1H), 3H (OHx2, NH) was not observed.

### Example 46

### Synthesis of (6R,6aS,11aR)-14-(cyclopropylmethyl)-2,6a-dihydroxy-9-phenyl-6,6a,7,9,10,11-hexahydro-6,11a-(epiminoethano)naphtho[2,1-f]indazol-8(5H)-one (Compound 68)

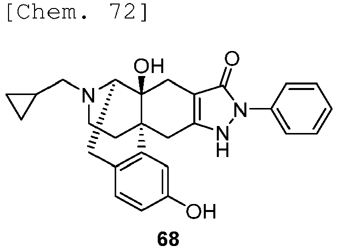

10% palladium/carbon (33.8 mg) was added to methanol (5 mL) of the compound 67 (337.8 mg, 0.63 mmol), and stirring was performed under a hydrogen atmosphere at room temperature for 19 hours. The reaction solution was filtered through celite, and the filtrate was concentrated under reduced pressure. The obtained residue was purified by silica gel chromatography, and thus, the title compound 68 (254.6 mg, 91%) was obtained.

NMR data identical to that of the compound 68 described in Example 45 was shown.

### Example 47

### Synthesis of (6R,6aS,11aR)-14-(cyclopropylmethyl)-2,6a-dihydroxy-9-isopropyl-6,6a,7,9,10,11-hexahydro-6,11a-(epiminoethano)naphtho[2,1-f]indazol-8(5H)-one (Compound 69)

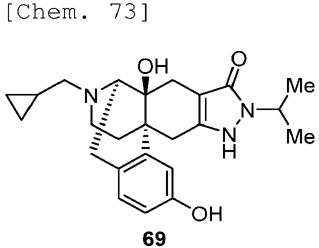

A reaction was carried out using the compound 52 (1.16 g, 2.78 mmol), phenyl isocyanate (0.41 g, 3.35 mmol), a 5.1 M copper(II) chloride aqueous solution (0.55 mL), and a 6 M potassium hydroxide aqueous solution (2 mL) in the same manner as that of Example 45, the obtained crude product was crudely purified by silica gel column chromatography, and thus, a crudely purified product (1.16 g) was obtained. A reaction was carried out using 308.8 mg of the crudely purified product (1.16 g) and isopropylhydrazine hydrochloride (130 mg, 1.15 mmol), the obtained crude product was crudely purified by silica gel column chromatography, and thus, the title compound 69 (107 mg, 35%) was obtained in the same manner as that of Example 46.

¹H-NMR (400 MHz, CD₃OD) δ (ppm): 0.19 - 0.29 (m, 2H), 0.53 - 0.67 (m, 2H), 0.89 - 1.40 (m, 1H), 1.26 (d, J = 6.8 Hz, 3H), 1.30 (d, J = 6.8 Hz, 3H), 1.30 - 1.37 (m, 1H), 2.19 - 2.30 (m, 1H), 2.26 - 2.36 (m, 1H), 2.29 (d, J = 16.5 Hz, 1H), 2.39 (d, J = 16.5 Hz, 1H), 2.55 (dd, J = 6.6, 12.9 Hz, 1H), 2.59 (dd, J = 6.6, 12.9 Hz, 1H), 2.69 - 2.77 (m, 1H), 2.82 (d, J = 16.7 Hz, 1H), 3.39 (dd, J = 6.7, 18.9 Hz, 1H), 3.10 (d, J = 16.7 Hz, 1H), 3.15 (d, J = 18.9 Hz, 1H), 3.43 (d, J = 6.7 Hz, 1H), 4.50 (sept, J = 6.8 Hz, 1H), 6.57 (dd, J = 2.4, 8.3 Hz, 1H), 6.94 (d, J = 2.4 Hz, 1H), 3H (OHx2, NH) was not observed.

### Example 48

### Synthesis of (6R,6aS,11aR)-10-cyclopropyl-14-(cyclopropylmethyl)-2-methoxy-8-methyl-5,6,10,11-tetrahydro-6,11a-(epiminoethano)naphtho[2,1-f]indazol-6a(7H)-ol (Compound 70) and (6R,6aS,11aR)-9-cyclopropyl-14-(cyclopropylmethyl)-2-methoxy-8-methyl-5,6,9,11-tetrahydro-6,11a-(epiminoethano)naphtho[2,1-f]indazol-6a(7H)-ol (Compound 71)

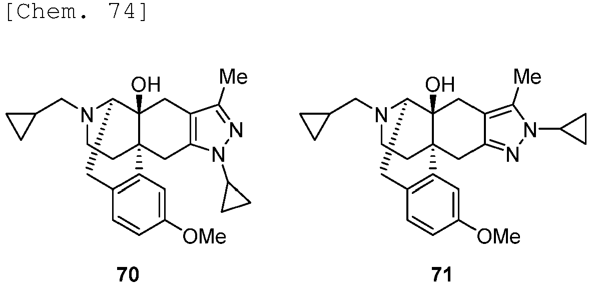

A reaction was carried out using the compound 3 (101.7 mg, 0.27 mmol), cyclohexylhydrazine hydrochloride (57.6 mg, 0.53 mmol), and methanesulfonic acid (42 µL, 0.64 mmol) in the same manner as that of Example 1, and thus, the title compound 70 (45.9 mg, 41%) as yellow-white amorphous and the title compound 71 (11.5 mg, 10%) as yellow-white amorphous were obtained.

### Compound 70

¹H-NMR (400 MHz, CDCl₃) δ (ppm): 0.09 - 0.18 (m, 2H), 0.50 - 0.59 (m, 2H), 0.83 - 0.94 (m, 1H), 0.97 - 1.11 (m, 3H), 1.14 - 1.22 (m, 1H), 1.33 - 1.38 (m, 1H), 1.99 (s, 3H), 2.11 - 2.26 (m, 2H), 2.37 (d, J = 15.9 Hz, 1H), 2.41 (d, J = 6.4 Hz, 2H), 2.42 (d, J = 15.9 Hz, 1H), 2.62 - 2.68 (m, 1H), 2.89 (d, J = 16.4 Hz, 1H), 2.94 (dd, J = 6.7, 18.5 Hz, 1H), 3.10 (d, J = 18.5 Hz, 1H), 3.21 (d, J = 6.7 Hz, 1H), 3.26 - 3.33 (m, 1H), 3.36 (d, J = 16.4 Hz, 1H), 3.71 (s, 3H), 4.68 (br s, 1H), 6.65 (dd, J = 2.6, 8.4 Hz, 1H), 6.70 (d, J = 2.6 Hz, 1H), 6.99 (d, J = 8.4 Hz, 1H).

### Compound 71

¹H-NMR (400 MHz, CDCl₃) δ (ppm): 0.09 - 0.20 (m, 2H), 0.50 - 0.58 (m, 2H), 0.83 - 0.99 (m, 4H), 1.08 - 1.15 (m, 1H), 1.29 - 1.35 (m, 1H), 2.07 (s, 3H), 2.11 - 2.23 (m, 2H), 2.36 (d, J = 15.8 Hz, 1H), 2.40 (d, J = 6.5 Hz, 2H), 2.46 (d, J = 15.8 Hz, 1H), 2.59 - 2.69 (m, 1H), 2.92 (dd, J = 6.8, 18.5 Hz, 1H), 2.93 (d, J = 16.4 Hz, 1H), 3.10 - 3.22 (m, 1H), 3.11 (d, J = 18.5 Hz, 1H), 3.19 (d, J = 6.8 Hz, 1H), 3.28 (d, J = 16.4 Hz, 1H), 3.70 (s, 3H), 6.65 (dd, J = 2.6, 8.4 Hz, 1H), 6.84 (d, J = 2.6 Hz, 1H), 6.97 (d, J = 8.4 Hz, 1H), 1H (OH) was not observed.

### Example 49

### Synthesis of (6R,6aS,11aR)-10-cyclobutyl-14-(cyclopropylmethyl)-2-methoxy-8-methyl-5,6,10,11-tetrahydro-6,11a-(epiminoethano)naphtho[2,1-f]indazol-6a(7H)-ol (Compound 72) and (6R,6aS,11aR)-9-cyclobutyl-14-(cyclopropylmethyl)-2-methoxy-8-methyl-5,6,9,11-tetrahydro-6,11a-(epiminoethano)naphtho[2,1-f]indazol-6a(7H)-ol (Compound 73)

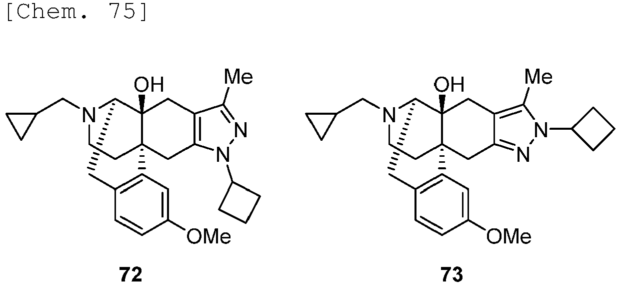

A reaction was carried out using the compound 3 (97.2 mg, 0.25 mmol), cyclobutylhydrazine hydrochloride (61.5 mg, 0.38 mmol), and methanesulfonic acid (40 µL, 0.61 mmol) in the same manner as that of Example 1, and thus, the title compound 72 (78.7 mg, 72%) as yellow-white amorphous and the title compound 73 (13.9 mg, 13%) as yellow-white amorphous were obtained.

### Compound 72

¹H-NMR (400 MHz, CDCl₃) δ (ppm): 0.09 - 0.18 (m, 2H), 0.50 - 0.59 (m, 2H), 0.83 - 0.94 (m, 1H), 1.30 - 1.36 (m, 1H), 1.72 - 1.82 (m, 2H), 2.03 (s, 3H), 2.11 - 2.24 (m, 2H), 2.32 - 2.47 (m, 6H), 2.60 - 2.67 (m, 3H), 2.85 (d, J = 16.2 Hz, 1H), 2.94 (dd, J = 6.8, 18.4 Hz, 1H), 3.10 (d, J = 18.4 Hz, 1H), 3.18 (d, J = 16.2 Hz, 1H), 3.21 (d, J = 6.8 Hz, 1H), 3.69 (s, 3H), 4.63 - 4.73 (m, 1H), 6.62 - 6.66 (m, 2H), 6.98 (d, J = 9.1 Hz, 1H), 1H (OH) was not observed.

### Compound 73

¹H-NMR (400 MHz, CDCl₃) δ (ppm): 0.10 - 0.17 (m, 2H), 0.50 - 0.57 (m, 2H), 0.83 - 0.94 (m, 1H), 1.30 - 1.36 (m, 1H), 1.77 - 1.88 (m, 2H), 2.58 (s, 3H), 2.10 - 2.33 (m, 4H), 2.38 (d, J = 15.7 Hz, 1H), 2.40 (d, J = 6.6 Hz, 2H), 2.46 (d, J = 15.7 Hz, 1H), 2.57 - 2.61 (m, 3H), 2.92 (dd, J = 6.7, 18.4 Hz, 1H), 2.99 (d, J = 16.3 Hz, 1H), 3.11 (d, J = 18.4 Hz, 1H), 3.19 (d, J = 6.7 Hz, 1H), 3.35 (d, J = 16.3 Hz, 1H), 3.70 (s, 3H), 4.45 - 4.55 (m, 1H), 6.64 (dd, J = 2.6, 8.4 Hz, 1H), 6.87 (d, J = 2.6 Hz, 1H), 6.97 (d, J = 8.4 Hz, 1H), 1H (OH) was not observed.

### Example 50

### Synthesis of (6R,6aS,11aR)-10-cyclopentyl-14-(cyclopropylmethyl)-2-methoxy-8-methyl-5,6,10,11-tetrahydro-6,11a-(epiminoethano)naphtho[2,1-f]indazol-6a(7H)-ol (Compound 74) and (6R,6aS,11aR)-9-cyclopentyl-14-(cyclopropylmethyl)-2-methoxy-8-methyl-5,6,9,11-tetrahydro-6,11a-(epiminoethano)naphtho[2,1-f]indazol-6a(7H)-ol (Compound 75)

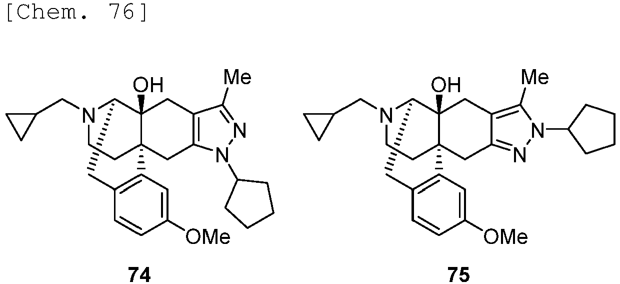

A reaction was carried out using the compound 3 (128.3 mg, 0.33 mmol), cyclopentylhydrazine hydrochloride (81.0 mg, 0.47 mmol), and methanesulfonic acid (52 µL, 0.77 mmol) in the same manner as that of Example 1, and thus, the title compound 74 (86.1 mg, 58%) as white amorphous and the title compound 75 (19.9 mg, 13%) as yellow oil were obtained.

### Compound 74

¹H-NMR (400 MHz, CDCl₃) δ (ppm): 0.10 - 0.18 (m, 2H), 0.50 - 0.59 (m, 2H), 0.83 - 0.94 (m, 1H), 1.30 - 1.36 (m, 1H), 1.60 - 1.72 (m, 2H), 1.85 - 1.98 (m, 2H), 1.99 - 2.25 (m, 6H), 2.01 (s, 3H), 2.39 (d, J = 16.5 Hz, 1H), 2.40 (d, J = 6.7 Hz, 2H), 2.43 (d, J = 16.5 Hz, 1H), 2.61 - 2.68 (m, 1H), 2.88 (d, J = 16.0 Hz, 1H), 2.97 (dd, J = 6.7, 18.4 Hz, 1H), 3.10 (d, J = 18.4 Hz, 1H), 3.212 (d, J = 16.0 Hz, 1H), 3.214 (d, J = 6.7 Hz, 1H), 3.70 (s, 3H), 4.55 (dddd, J = 8.0, 8.0, 8.0, 8.0 Hz, 1H), 4.66 (br s, 1H), 6.45 (dd, J = 2.6, 8.3 Hz, 1H), 6.67 (d, J = 2.6 Hz, 1H), 6.98 (d, J = 8.3 Hz, 1H).

### Compound 75

¹H-NMR (400 MHz, CDCl₃) δ (ppm): 0.08 - 0.19 (m, 2H), 0.48 - 0.58 (m, 2H), 0.83 - 0.95 (m, 1H), 1.28 - 1.36 (m, 1H), 1.53 - 1.66 (m, 2H), 1.82 - 2.08 (m, 6H), 1.99 (s, 3H), 2.11 - 2.25 (m, 2H), 2.39 (d, J = 15.6 Hz, 1H), 2.40 (d, J = 6.4 Hz, 2H), 2.47 (d, J = 15.6 Hz, 1H), 2.60 - 2.67 (m, 1H), 2.92 (dd, J = 6.7, 18.5 Hz, 1H), 2.97 (d, J = 16.1 Hz, 1H), 3.11 (d, J = 18.5 Hz, 1H), 3.19 (d, J = 6.7 Hz, 1H), 3.31 (d, J = 16.1 Hz, 1H), 3.69 (s, 3H), 4.35 (dddd, J = 7.8, 7.8, 7.8, 7.8 Hz, 1H), 6.64 (dd, J = 2.6, 8.4 Hz, 1H), 6.85 (d, J = 2.6 Hz, 1H), 6.97 (d, J = 8.4 Hz, 1H), 1H (OH) was not observed.

### Example 51

### Synthesis of (6R,6aS,11aR)-14-(cyclopropylmethyl)-2-methoxy-8-methyl-10-(tetrahydro-2H-pyran-4-yl)-5,6,10,11-tetrahydro-6,11a-(epiminoethano)naphtho[2,1-f]indazol-6a(7H)-ol (Compound 76) and (6R,6aS,11aR)-14-(cyclopropylmethyl)-2-methoxy-8-methyl-9-(tetrahydro-2H-pyran-4-yl)-5,6,9,11-tetrahydro-6,11a-(epiminoethano)naphtho[2,1-f]indazol-6a(7H)-ol (Compound 77)

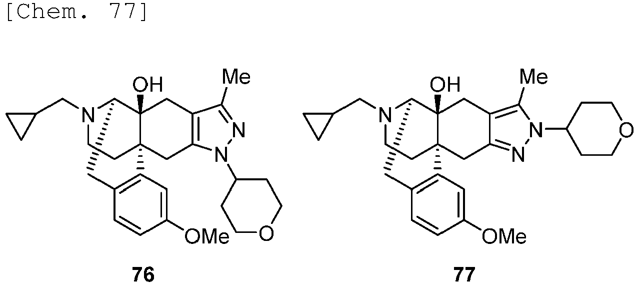

Under an argon atmosphere, tert-butyl 2-(tetrahydro-2H-pyran-4-yl)hydrazine-1-carboxylate (synthesized by a method described in J. Med. Chem., 2007, 50, 4789-4792) (116.7 mg, 0.54 mmol) and methanesulfonic acid (60 µL, 0.89 mmol) were added to an ethanol (4.5 mL) solution of the compound 3 (112.6 mg, 0.54 mmol), and heating and reflux were performed for 14.5 hours. After cooling, the reaction solution was concentrated under reduced pressure, a saturated sodium bicarbonate aqueous solution was added thereto, and extraction was performed with chloroform. After combined organic layers were washed with saturated brine and dried with anhydrous sodium sulfate, concentration was performed under reduced pressure. The obtained crude product was purified by silica gel column chromatography, and thus, the title compound 76 (77.2 mg, 57%) as yellow-white amorphous and the title compound 77 (18.3 mg, 13%) as yellow-white amorphous were obtained.

### Compound 76

¹H-NMR (400 MHz, CDCl₃) δ (ppm): 0.09 - 0.18 (m, 2H), 0.50 - 0.50 (m, 2H), 0.83 - 0.94 (m, 1H), 1.31 - 1.37 (m, 1H), 1.83 - 1.92 (m, 2H), 2.01 (s, 3H), 2.11 - 2.40 (m, 5H), 2.42 (d, J = 6.6 Hz, 2H), 2.44 (d, J = 16.3 Hz, 1H), 2.61 - 2.68 (m, 1H), 2.90 (d, J = 15.7 Hz, 1H), 2.95 (dd, J = 6.8, 18.6 Hz, 1H), 3.11 (d, J = 18.6 Hz, 1H), 3.215 (d, J = 15.7 Hz, 1H), 3.217 (d, J = 6.8 Hz, 1H), 3.50 - 3.61 (m, 2H), 3.12 (s, 3H), 4.14 (br d, J = 11.9 Hz, 2H), 4.23 (dddd, J = 4.1, 4.1, 11.8, 11. 8 Hz, 1H), 4.68 (br s, 1H), 6.652 (dd, J = 2.6, 9.1 Hz, 1H), 6.654 (d, J = 2.6, 1H), 7.00 (d, J = 9.1 Hz, 1H).

### Compound 77

¹H-NMR (400 MHz, CDCl₃) δ (ppm): 0.09 - 0.19 (m, 2H), 0.49 - 0.59 (m, 2H), 0.83 - 0.93 (m, 1H), 1.29 - 1.36 (m, 1H), 1.66 - 1.79 (m, 2H), 2.01 (s, 3H), 2.10 - 2.31 (m, 4H), 2.40 (d, J = 15.7 Hz, 1H), 2.41 (d, J = 6.4 Hz, 2H), 2.47 (d, J = 15.7 Hz, 1H), 2.61 - 2.67 (m, 1H), 2.92 (dd, J = 6.7, 18.5 Hz, 1H), 2.97 (d, J = 16.2 Hz, 1H), 3.12 (d, J = 18.5 Hz, 1H), 3.20 (d, J = 6.7 Hz, 1H), 3.31 (d, J = 16.2 Hz, 1H), 3.41 - 3.52 (m, 2H), 3.69 (s, 3H), 3.98 - 4.11 (m, 3H), 6.65 (d, J = 2.6, 8.4 Hz, 1H), 6.85 (d, J = 2.6 Hz, 1H), 6.98 (d, J = 8.4 Hz, 1H), 1H (OH) was not observed.

### Example 52

### Synthesis of (6R,6aS,11aR)-14-(cyclopropylmethyl)-2-methoxy-8-methyl-10-(tetrahydro-2H-thiopyran-4-yl)-5,6,10,11-tetrahydro-6,11a-(epiminoethano)naphtho[2,1-f]indazol-6a(7H)-ol (Compound 78) and (6R,6aS,11aR)-14-(cyclopropylmethyl)-2-methoxy-8-methyl-9-(tetrahydro-2H-thiopyran-4-yl)-5,6,9, 11-tetrahydro-6,11a-(epiminoethano)naphtho[2,1-f]indazol-6a(7H)-ol (Compound 79)

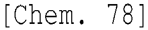

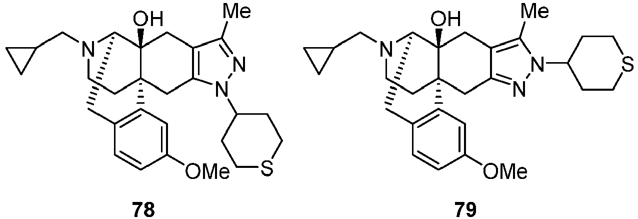

A reaction was carried out using ethanol (7.0 mL) of the compound 3 (202.1 mg, 0.53 mmol), tert-butyl 2-(tetrahydro-2H-thiopyran-4-yl)hydrazin-1-carboxylate (synthesized by a method described in J. Med. Chem., 2004, 47, 2180-2193) (183.7 mg, 0.79 mmol), and methanesulfonic acid (103 µL, 1.52 mmol) in the same manner as that of Example 51, and thus, the title compound 78 (110.1 mg, 43.6%) as white amorphous and the title compound 79 (51.0 mg, 20.2%) as white amorphous were obtained.

### Compound 78

¹H-NMR (400 MHz, CDCl₃) δ (ppm): 0.10 - 0.18 (m, 2H), 0.51 - 0.60 (m, 2H), 0.83 - 0.94 (m, 1H), 1.31 - 1.36 (m, 1H), 2.00 (s, 3H), 2.11 - 2.41 (m, 6H), 2.37 (d, J = 16.5 Hz, 1H), 2.41 (d, J = 6.5 Hz, 2H), 2.44 (d, J = 16.5 Hz, 1H), 2.62 - 2.68 (m, 1H), 2.76 - 2.99 (m, 6H), 3.10 (d, J = 18.5 Hz, 1H), 3.20 (d, J = 15.9 Hz, 1H), 3.21 (d, J = 6.3 Hz, 1H), 3.70 (s, 3H), 4.00 (dddd, J = 3.7, 3.7, 11.6, 11.6 Hz, 1H), 4.67 (br s, 1H), 6.63 - 6.67 (m, 2H), 7.00 (d, J = 9.0 Hz, 1H).

### Compound 79

¹H-NMR (400 MHz, CDCl₃) δ (ppm): 0.11 - 0.17 (m, 2H), 0.51 - 0.57 (m, 2H), 0.83 - 0.92 (m, 1H), 1.29 - 1.35 (m, 1H), 1, 98 (s, 3H), 2.02 - 2.34 (m, 6H), 2.38 (d, J = 15.8 Hz, 1H), 2.40 (d, J = 6.4 Hz, 2H), 2.45 (d, J = 15.8 Hz, 1H), 2.61 - 2.66 (m, 1H), 2.69 - 2.84 (m, 4H), 2.91 (dd, J = 6.7, 18.4 Hz, 1H), 2.96 (d, J = 16.2 Hz, 1H), 3.12 (d, J = 18.4 Hz, 1H), 3.19 (d, J = 6.7 Hz, 1H), 3.30 (d, J = 16.2 Hz, 1H), 3.70 (s, 3H), 3.79 (dddd, J = 3.4, 3.4, 11.6, 11.6 Hz, 1H), 6.65 (dd, J = 2.6, 8.4 Hz, 1H), 6.84 (d, J = 2.6 Hz, 1H), 6.97 (d, J = 8.4 Hz, 1H), 1H (OH) was not observed.

### Example 53

### Synthesis of a mixture of (6R,6aS,11aR)-14-(cyclopropylmethyl)-2-methoxy-8-methyl-10-(1-methylpiperidin-4-yl)-5,6,10,11-tetrahydro-6,11a-(epiminoethano)naphtho[2,1-f]indazol-6a(7H)-ol (Compound 80) and (6R,6aS,11aR)-14-(cyclopropylmethyl)-2-methoxy-8-methyl-9-(1-methylpiperidin-4-yl)-5,6,9,11-tetrahydro-6,11a-(epiminoethano)naphtho[2,1-f]indazol-6a(7H)-ol (Compound 81)

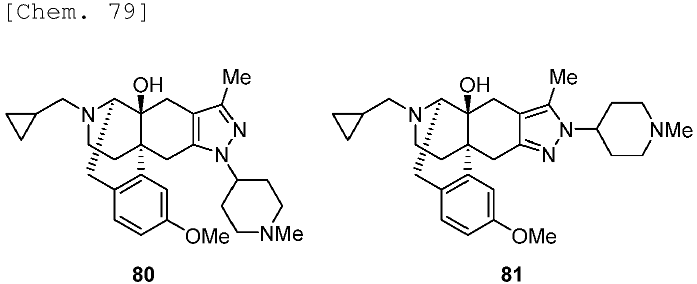

A reaction was carried out using the compound 3 (116.2 mg, 0.30 mmol), tert-butyl 2-(1-methylpiperidin-4-yl)hydrazine-1-carboxylate (synthesis by a method described in WO 2016/44666 A1) (108.5 mg, 0.47 mmol), and methanesulfonic acid (90 µL, 1.3 mmol) in the same manner as that of Example 51, and thus, a mixture (60.8 mg, 42%) of the title compound 80 and the title compound 81 as white amorphous was obtained.

### Mixture of compound 80 and compound 81

¹H-NMR (400 MHz, CDCl₃) δ (ppm): 0.08 - 0.18 (m, 2H), 0.48 - 0.60 (m, 2H), 0.82 - 0.94 (m, 1H), 1.28 - 1.35 (m, 1H), 1.63 - 2.50 (m, 12H), 1.99 (s, 3H), 2.39 (s, 1.8H), 2.41 (s, 1.2H), 2.59 - 2.69 (m, 1H), 2.87 - 3.10 (m, 4H), 3.10 (d, J = 18.4 Hz, 0.6H), 3.13 (d, J = 18.5 Hz, 0.4H), 3.19 (d, J = 7.8 Hz, 0.6H), 3.21 (d, J = 7.2 Hz, 0.4H), 3.27 (d, J = 16.1 Hz, 0.4H), 3.29 (d, J = 16.2 Hz, 0.6H), 3.69 (s, 3H), 3.78 - 3.88 (m, 0.4H), 4.01 - 4.12 (m, 0.6H), 4.67 (br s, 1H), 6.62 - 9.68 (m, 1.6H), 6.85 (d, J = 2.6 Hz, 0.4H), 6.97 (d, J = 8.1 Hz, 0.6H), 6.99 (d, J = 7.9 Hz, 0.4H).

### Example 54

### Synthesis of (6R,6aS,11aR)-10-cyclopropyl-14-(cyclopropylmethyl)-8-methyl-5,6,10,11-tetrahydro-6,11a-(epiminoethano)naphtho[2,1-f]indazole-2,6a(7H)-diol (Compound 82)

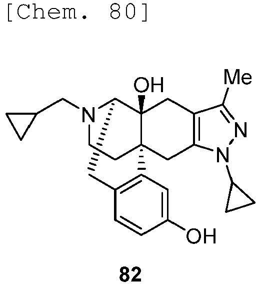

A reaction was carried out using the compound 70 (45.9 mg, 0.11 mmol), and a 1.0 M dichloromethane solution of boron tribromide (0.33 mL, 0.33 mmol) in the same manner as that of Example 11, and thus, the title compound 82 (11.5 mg, 26%) as white amorphous was obtained.

### Compound 82

¹H-NMR (400 MHz, CDCl₃) δ (ppm): 0.09 - 0.18 (m, 2H), 0.49 - 0.60 (m, 3H), 0.68 - 0.96 (m, 4H), 1.26 - 1.33 (m, 1H), 1.97 (s, 3H), 2.11 - 2.23 (m, 2H), 2.34 (d, J = 15.9 Hz, 1H), 2.40 (d, J = 6.5 Hz, 2H), 2.45 (d, J = 15.9 Hz, 1H), 2.59 - 2.68 (m, 1H), 2.76 (d, J = 16.2 Hz, 1H), 2.90 (dd, J = 6.8, 18.5 Hz, 1H), 2.95 - 3.33 (m, 1H), 3.07 (d, J = 18.5 Hz, 1H), 3.19 (d, J = 6.8 Hz, 1H), 3.22 (d, J = 16.2 Hz, 1H), 6.51 (dd, J = 2.4, 8.3 Hz, 1H), 6.72 (d, J = 2.4 Hz, 1H), 6.86 (d, J = 8.3 Hz, 1H), 2H (OHX2) was not observed.

### Example 55

### Synthesis of (6R,6aS,11aR)-9-cyclopropyl-14-(cyclopropylmethyl)-8-methyl-5,6,9,11-tetrahydro-6,11a-(epiminoethano)naphtho[2,1-f]indazole-2,6a(7H)-diol (Compound 83)

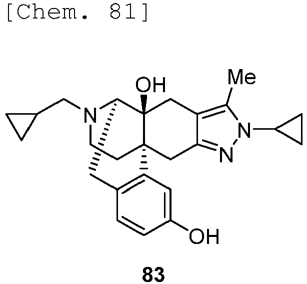

A reaction was carried out using the compound 71 (11.3 mg, 0.027 mmol), and a 1.0 M dichloromethane solution of boron tribromide (81 µL, 0.081 mmol) in the same manner as that of Example 11, and thus, the title compound 83 (4.9 mg, 45%) as yellow oil was obtained.

### Compound 83

¹H-NMR (400 MHz, CDCl₃) δ (ppm): 0.10 - 0.18 (m, 2H), 0.49 - 0.58 (m, 2H), 0.83 - 0.95 (m, 4H), 1.04 - 1.11 (m, 1H), 1.29 - 1.34 (m, 1H), 2, 07 (s, 3H), 2.11 - 2.26 (m, 2H), 2.37 (d, J = 15.9 Hz, 1H), 2.41 (d, J = 6.5 Hz, 2H), 2.46 (d, J = 15.9 Hz, 1H), 2.62 - 2.69 (m, 1H), 2.90 (dd, J = 6.7, 18.3 Hz, 1H), 2.94 (d, J = 16.2 Hz, 1H), 3.09 - 3.17 (m, 1H), 3.11 (d, J = 18.3 Hz, 1H), 3.19 (d, J = 6.7 Hz, 1H), 3.28 (d, J = 16.2 Hz, 1H), 6.62 (dd, J = 2.5, 8.2 Hz, 1H), 6.86 (d, J = 2.5 Hz, 1H), 6.93 (d, J = 8.2 Hz, 1H), 2H (OHX2) was not observed.

### Example 56

### Synthesis of (6R,6aS,11aR)-10-cyclobutyl-14-(cyclopropylmethyl)-8-methyl-5,6,10,11-tetrahydro-6,11a-(epiminoethano)naphtho[2,1-f]indazole-2,6a(7H)-diol (Compound 84)

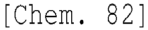

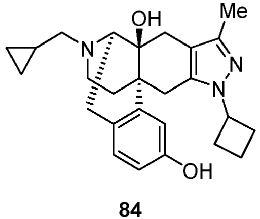

A reaction was carried out using the compound 72 (78.7 mg, 0.18 mmol), and a 1.0 M dichloromethane solution of boron tribromide (0.55 mL, 0.55 mmol) in the same manner as that of Example 11, and thus, the title compound 84 (54.3 mg, 71%) as white amorphous was obtained.

### Compound 84

¹H-NMR (400 MHz, CDCl₃) δ (ppm): 0.08 - 0.20 (m, 2H), 0.48 - 0.60 (m, 2H), 0.81 - 0.95 (m, 1H), 1.27 - 1.35 (m, 1H), 1.56 - 1.77 (m, 2H), 1.99 (s, 3H), 2.12 - 2.33 (m, 4H), 2.34 - 2.70 (m, 7H), 2.79 (d, J = 16.1 Hz, 1H), 2.91 (dd, J = 6.5, 18.5 Hz, 1H), 3.07 (d, J = 18.5 Hz, 1H), 3.11 (d, J = 16.1 Hz, 1H), 3.26 (d, J = 6.5 Hz, 1H), 4.55 (dddd, J = 8.4, 8.4, 8.4, 8.4 Hz, 1H), 6.52 (dd, J = 2.1, 8.2 Hz, 1H), 6.61 (d, J = 2.1 Hz, 1H), 6.86 (d, J = 8.2 Hz, 1H), 2H (OHX2) was not observed.

### Example 57

### Synthesis of (6R,6aS,11aR)-9-cyclobutyl-14-(cyclopropylmethyl)-8-methyl-5,6,9,11-tetrahydro-6,11a-(epiminoethano)naphtho[2,1-f]indazole-2,6a(7H)-diol (Compound 85)

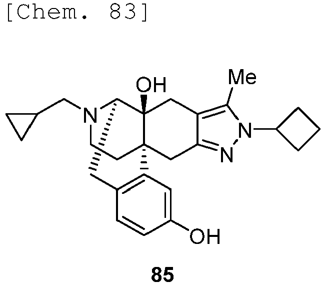

A reaction was carried out using the compound 73 (13.9 mg, 0.032 mmol), and a 1.0 M dichloromethane solution of boron tribromide (96 µL, 0.096 mmol) in the same manner as that of Example 11, and thus, the title compound 85 (9.7 mg, 72%) as yellow oil was obtained.

### Compound 85

¹H-NMR (400 MHz, CDCl₃) δ (ppm): 0.10 - 0.20 (m, 2H), 0.49 - 0.60 (m, 2H), 0.83 - 0.98 (m, 1H), 1.23 - 1.33 (m, 1H), 1.60 - 1.77 (m, 2H), 1.96 (s, 3H), 2.08 - 2.30 (m, 4H), 2.38 (d, J = 15.8 Hz, 1H), 2.44 (d, J = 6.7 Hz, 2H), 2.45 (d, J = 15.9 Hz, 1H), 2.46 - 2.71 (m, 3H), 2.91 (dd, J = 6.5, 18.5 Hz, 1H), 2.97 (d, J = 16.3 Hz, 1H), 3.10 (d, J = 18.5 Hz, 1H), 3.23 (d, J = 6.5 Hz, 1H), 3.30 (d, J = 16.3 Hz, 1H), 4.49 (dddd, J = 8.4, 8.4, 8.4, 8.4 Hz, 1H), 6.61 (dd, J = 2.4, 8.2 Hz, 1H), 6.81 (d, J = 2.4 Hz, 1H), 6.92 (d, J = 8.2 Hz, 1H), 2H (OHX2) was not observed.

### Example 58

### Synthesis of (6R,6aS,11aR)-10-cyclopentyl-14-(cyclopropylmethyl)-8-methyl-5,6,10,11-tetrahydro-6,11a-(epiminoethano)naphtho[2,1-f]indazole-2,6a(7H)-diol (Compound 86)

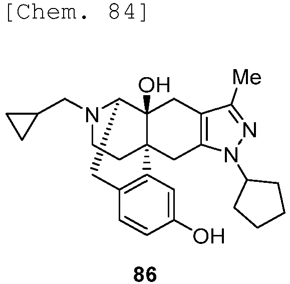

A reaction was carried out using the compound 74 (78.4 mg, 0.18 mmol), and a 1.0 M dichloromethane solution of boron tribromide (0.53 mL, 0.53 mmol) in the same manner as that of Example 11, and thus, the title compound 86 (48.8 mg, 64%) as white amorphous was obtained.

### Compound 86

¹H-NMR (400 MHz, CDCl₃) δ (ppm): 0.08 - 0.19 (m, 2H), 0.48 - 0.59 (m, 2H), 0.81 - 0.94 (m, 1H), 1.27 - 1.38 (m, 1H), 1.42 - 1.59 (m, 2H), 1.69 - 1.83 (m, 2H), 1.88 - 2.05 (m, 4H), 1.97 (s, 3H), 2.11 - 2.22 (m, 2H), 2.37 (d, J = 15.8 Hz, 1H), 2.40 (d, J = 6.6 Hz, 2H), 2.46 (d, J = 15.8 Hz, 1H), 2.58 - 2.69 (m, 1H), 2.82 (d, J = 16.1 Hz, 1H), 2.91 (dd, J = 6.6, 18.5 Hz, 1H), 3.07 (d, J = 18.5 Hz, 1H), 3.15 (d, J = 16.1 Hz, 1H), 3.20 (d, J = 6.6 Hz, 1H), 4.45 (dddd, J = 8.0, 8.0, 8.0, 8.0 Hz, 1H), 6.50 (dd, J = 2.3, 8.3 Hz, 1H), 6.63 (d, J = 2.3 Hz, 1H), 6.85 (d, J = 8.3 Hz, 1H), 2H (OHX2) was not observed.

### Example 59

### Synthesis of (6R,6aS,11aR)-9-cyclopentyl-14-(cyclopropylmethyl)-8-methyl-5,6,9,11-tetrahydro-6,11a-(epiminoethano)naphtho[2,1-f]indazole-2,6a(7H)-diol (Compound 87)

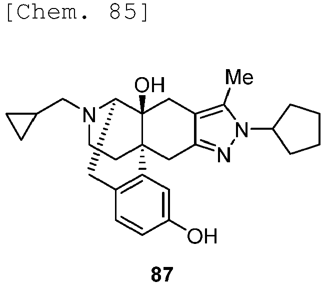

A reaction was carried out using the compound 75 (98.1 mg, 0.22 mmol), and a 1.0 M dichloromethane solution of boron tribromide (0.66 mL, 0.66 mmol) in the same manner as that of Example 11, and thus, the title compound 87 (51.9 mg, 55%) as white amorphous was obtained.

### Compound 87

¹H-NMR (400 MHz, CDCl₃) δ (ppm): 0.10 - 0.20 (m, 2H), 0.49 - 0.58 (m, 2H), 0.83 - 0.95 (m, 1H), 1.25 - 1.32 (m, 1H), 1.38 - 1.58 (m, 2H), 1.58 - 2.01 (m, 6H), 1.99 (s, 3H), 2.10 - 2.25 (m, 2H), 2.38 (d, J = 15.8 Hz, 1H), 2.40 (d, J = 6.6 Hz, 2H), 2.46 (d, J = 15.8 Hz, 1H), 2.61 - 2.68 (m, 1H), 2.88 (dd, J = 6.8, 18.5 Hz, 1H), 2.97 (d, J = 16.2 Hz, 1H), 3.11 (d, J = 18.5 Hz, 1H), 3.18 (d, J = 6.8 Hz, 1H), 3.30 (d, J = 16.2 Hz, 1H), 4.31 (dddd, J = 8.1, 8.1, 8.1, 8.1 Hz, 1H), 6.61 (dd, J = 2.5, 8.2 Hz, 1H), 6.83 (d, J = 2.5 Hz, 1H), 6.92 (d, J = 8.2 Hz, 1H), 2H (OHX2) was not observed.

### Example 60

### Synthesis of (6R,6aS,11aR)-14-(cyclopropylmethyl)-8-methyl-10-(tetrahydro-2H-pyran-4-yl)-5,6,10,11-tetrahydro-6,11a-(epiminoethano)naphtho[2,1-f]indazole-2,6a(7H)-diol (Compound 88)

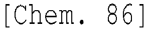

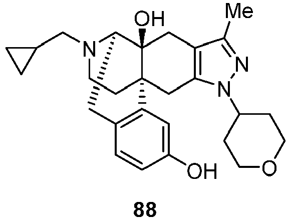

A reaction was carried out using the compound 76 (77.2 mg, 0.17 mmol), and a 1.0 M dichloromethane solution of boron tribromide (0.60 mL, 0.60 mmol) in the same manner as that of Example 11, and thus, the title compound 88 (64.1 mg, 86%) as white amorphous was obtained.

### Compound 88

¹H-NMR (400 MHz, CDCl₃) δ (ppm): 0.09 - 0.19 (m, 2H), 0.50 - 0.59 (m, 2H), 0.82 - 0.93 (m, 1H), 1.31 - 1.37 (m, 1H), 1.79 - 1.89 (m, 2H), 2.00 (s, 3H), 2.12 - 2.35 (m, 4H), 2.38 (d, J = 16.2 Hz, 1H), 2.40 (d, J = 6.8 Hz, 2H), 2.45 (d, J = 16.2 Hz, 1H), 2.62 - 2.68 (m, 1H), 2.88 (d, J = 16.1 Hz, 1H), 2.93 (dd, J = 6.7, 18.4 Hz, 1H), 3.09 (d, J = 18.4 Hz, 1H), 3.18 (d, J = 16.1 Hz, 1H), 3.21 (d, J = 6.7 Hz, 1H), 3.45 - 3.57 (m, 2H), 4.04 - 4.14 (m, 2H), 4.15 - 4.25 (m, 1H), 6.54 (dd, J = 2.5, 8.2 Hz, 1H), 6.61 (d, J = 2.5 Hz, 1H), 6.92)d, J = 8.2 Hz, 1H), 2H (OHX2) was not observed.

### Example 61

### Synthesis of (6R,6aS,11aR)-14-(cyclopropylmethyl)-8-methyl-10-(tetrahydro-2H-thiopyran-4-yl)-5,6,10,11-tetrahydro-6,11a-(epiminoethano)naphtho[2,1-f]indazole-2,6a(7H)-diol (Compound 89)

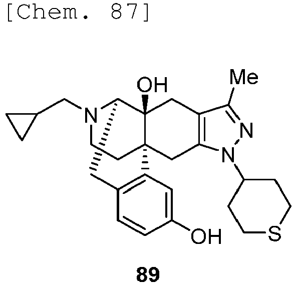

A reaction was carried out using the compound 78 (87.2 mg, 0.18 mmol), and a 1.0 M dichloromethane solution of boron tribromide (0.55 mL, 0.55 mmol) in the same manner as that of Example 11, and thus, the title compound 89 (66.6 mg, 79%) as pale yellow amorphous was obtained.

### Compound 89

¹H-NMR (400 MHz, CDCl₃) δ (ppm): 0.09 - 0.18 (m, 2H), 0.50 - 0.59 (m, 2H), 0.82 - 0.93 (m, 1H), 1.30 - 1.38 (m, 1H), 1.98 (s, 3H), 2.10 - 2.32 (m, 6H), 2.37 (d, J = 15.9 Hz, 1H), 2.40 (d, J = 6.5 Hz, 2H), 2.45 (d, J = 15.9 Hz, 1H), 2.61 - 2.81 (m, 5H), 2.85 (d, J = 16.0 Hz, 1H), 2.92 (dd, J = 6.7, 18.6 Hz, 1H), 3.08 (d, J = 18.6 Hz, 1H), 3.16 (d, J = 16.0 Hz, 1H), 3.20 (d, J = 6.7 Hz, 1H), 3.94 (dddd, J = 3.5, 3.5, 11.6, 11.6 Hz, 1H), 6.52 (dd, J = 2.4, 8.2 Hz, 1H), 6.61 (d, J = 2.4 Hz, 1H), 6.88 (d, J = 8.2 Hz, 1H), 2H (OHX2) was not observed.

### Example 62

### Synthesis of (6R,6aS,11aR)-14-(cyclopropylmethyl)-8-methyl-9-(tetrahydro-2H-thiopyran-4-yl)-5,6,9,11-tetrahydro-6,11a-(epiminoethano)naphtho[2,1-f]indazole-2,6a(7H)-diol (Compound 90)

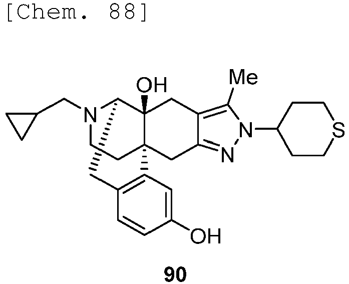

A reaction was carried out using the compound 79 (34.9 mg, 0.073 mmol), and a 1.0 M dichloromethane solution of boron tribromide (0.22 mL, 0.22 mmol) in the same manner as that of Example 11, and thus, the title compound 90 (21.3 mg, 63%) as pale yellow amorphous was obtained.

### Compound 90

¹H-NMR (400 MHz, DMSO - d₆) δ (ppm): 0.06 - 0.14 (m, 2H), 0.41 - 0.51 (m, 2H), 0.80 - 0.88 (m, 1H), 1.13 (br d, J = 11.1 Hz, 1H), 1.93 - 2.10 (m, 7H), 1.95 (s, 3H), 2.21 (d, J = 15.7 Hz, 1H), 2.29 - 2.38 (m, 3H), 2.52 - 2.58 (m, 1H), 2.60 - 2.69 (m, 3H), 2.71 - 2.85 (m, 3H), 2.99 (d, J = 18.7 Hz, 1H), 3.04 (d, J = 16.1 Hz, 1H), 3.06 - 3.12 (m, 1H), 3.90 - 3.99 (m, 1H), 4.45 (br s, 1H), 6.43 (dd, J = 2.4, 8.2 Hz, 1H), 6.60 (d, J = 2.4 Hz, 1H), 6.84 (d, J = 8.2 Hz, 1H).

### Example 63

### Synthesis of (6R,6aS,11aR)-14-(cyclopropylmethyl)-8-methyl-10-(1-methylpiperidin-4-yl)-5,6,10,11-tetrahydro-6,11a-(epiminoethano)naphtho[2,1-f]indazole-2,6a(7H)-diol (Compound 91) and (6R,6aS,11aR)-14-(cyclopropylmethyl)-8-methyl-9-(1-methylpiperidin-4-yl)-5,6,9,11-tetrahydro-6,11a-(epiminoethano)naphtho[2,1-f]indazole-2,6a(7H)-diol (Compound 92)

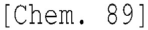

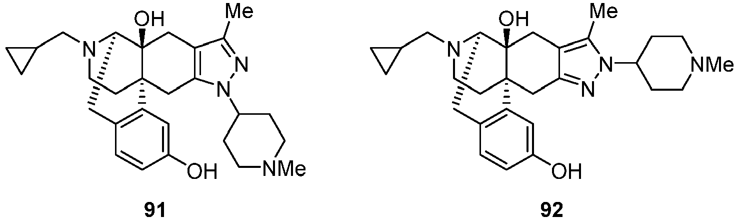

A reaction was carried out using a mixture (60.8 mg, 0.064 mmol) of the compounds 80 and 81, and a 1.0 M dichloromethane solution of boron tribromide (0.45 mL, 0.45 mmol) in the same manner as that of Example 11, and thus, the title compound 91 (17.4 mg, 30%) as yellow-white amorphous and the title compound 92 (13.1 mg, 22%) as yellow-white amorphous were obtained.

### Compound 91

¹H-NMR (400 MHz, CDCl₃) δ (ppm): 0.09 - 0.18 (m, 2H), 0.48 - 0.58 (m, 2H), 0.82 - 0.93 (m, 1H), 1.24 - 1.33 (m, 1H), 1.60 - 1.80 (m, 2H), 1.79 (s, 3H), 1.98 - 2.23 (m, 6H), 2.30 (s, 3H), 2.34 (d, J = 15.8 Hz, 1H), 2.39 (d, J = 6.5 Hz, 2H), 2.43 (d, J = 15.8 Hz, 1H), 2.60 - 2.67 (m, 1H), 2.88 (dd, J = 6.8, 18.4 Hz, 1H), 2.91 - 3.02 (m, 2H), 2.94 (d, J = 16.3 Hz, 1H), 3.10 (d, J = 18.4 Hz, 1H), 3.16 (d, J = 6.8 Hz, 1H), 3.25 (d, J = 16.3 Hz, 1H), 3.80 - 3.89 (m, 1H), 4.69 (br s, 1H), 6.60 (dd, J = 2.5, 8.2 Hz, 1H), 6.77 (d, J = 2.5 Hz, 1H), 6.91 (d, J = 8.2 Hz, 1H), 1H (OH) was not observed.

### Compound 92

¹H-NMR (400 MHz, CDCl₃) δ (ppm): 0.08 - 0.17 (m, 2H), 0.48 - 0.58 (m, 2H), 0.80 - 0.91 (m, 1H), 1.08 - 1.14 (m, 1H), 1.89 - 2.00 (m, 2H), 1.97 (s, 3H), 2.04 - 2.20 (m, 4H), 2.23 - 2.42 (m, 5H), 2.34 (s, 3H), 2.45 (d, J = 15.9 Hz, 1H), 2.53 - 2.60 (m, 1H), 2.81 (d, J = 16.1 Hz, 1H), 2.91 (dd, J = 6.6, 18.5 Hz, 1H), 2.95 - 3.04 (m, 2H), 3.06 (d, J = 18.5 Hz, 1H), 3.17 (d, J = 6.6 Hz, 1H), 3.25 (d, J = 16.1 HZ, 1H), 4.08 (dddd, J = 4.2, 4.2, 11.8, 11.8 Hz, 1H), 4.62 (br s, 1H), 6.52 (dd, J = 1.9, 8.2 Hz, 1H), 6.76 (br d, J = 1.9 Hz, 1H), 6.90 (d, J = 8.2 Hz, 1H), 1H (OH) was not observed.

### Example 64

### Synthesis of a mixture of (1S,4S)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 93) and (1S,4R)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 94)

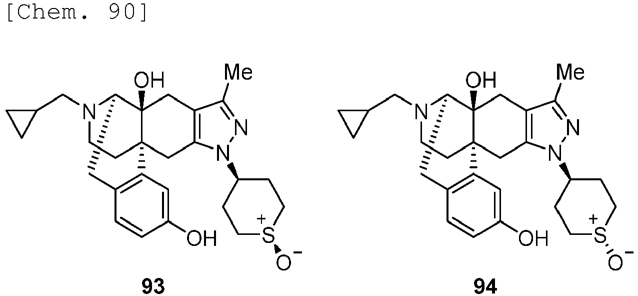

Under an argon atmosphere, m-chloroperbenzoic acid (77%, 16.0 mg, 0.071 mmol) was added to a dichloromethane (3.0 mL) solution of the compound 89 (29.2 mg, 0.063 mmol) at room temperature, and stirring was performed at room temperature for 3 hours. A saturated thiosulfate aqueous sodium was added to the reaction solution under ice cooling, and stirring was performed. After confirming the disappearance of m-chloroperbenzoic acid on potassium iodide starch paper, a saturated sodium bicarbonate aqueous solution was added, and extraction was performed with chloroform. After an organic layer was washed with saturated brine and dried with anhydrous sodium sulfate, concentration was performed under reduced pressure. The obtained crude product was purified by silica gel column chromatography, and thus, a mixture (18.8 mg, 62%) of the title compound 93 and the title compound 94 as colorless oil was obtained.

### Mixture of compound 93 and compound 94

¹H-NMR (400 MHz, acetone - d₆) δ (ppm): 0.13 - 0.23 (m, 2H), 0.48 - 0.59 (m, 2H), 0.85 - 0.97 (m, 1H), 1.25 - 1.33 (m, 1H), 1.91 (s, 1.8H), 1.92 (s, 1.2H), 2.12 - 2.25 (m, 2.6H), 2.31 (d, J = 15.9 Hz, 1H), 2.36 - 2.52 (m, 3.6H), 2.62 - 2.72 (m, 1H), 2.75 - 3.12 (m, 7.8H), 3.14 (d, J = 18.5 Hz, 1H), 3.23 (d, J = 6.6 Hz, 1H), 3.34 (d, J = 16.1 Hz, 0.6H), 3.36 (d, J = 16.0 Hz, 0.4H), 3.39 - 3.48 (m, 1H), 3.58 - 3.66 (m, 1H), 4.44 - 4.53 (m, 0.4H), 4.58 - 4.66 (m, 0.6H), 6.57 (dd, J = 2.4, 8.2 Hz, 0.4H), 6.58 (dd, J = 2.4, 8.2 Hz, 0.6H), 6.79 (d, J = 2.4 Hz, 0.6H), 6.82 (d, J = 2.4 Hz, 0.4H), 6.92 (d, J = 8.2 Hz, 1H), 1H (OH) was not observed.

### Example 65

### Opioid receptor function test

Functional activity of the compound provided by the present invention on µ, δ, and κ opioid receptors was examined.

Method: Using a Lance Ultra cAMP kit (Perkin Elmer), the functional activity was examined by a predetermined method. In evaluation of agonist activity, CHO cells expressing each of human opioid receptors (δ, µ, and κ: accession numbers and catalog numbers are described below) were reacted with a test compound in an assay buffer (1 × HBSS, 1 M HEPES, pH 7.4, 250 mM isobutylmethylxanthine (IBMX), 7.5% BSA) in the presence of 10 µM forskolin for 30 minutes. Subsequently, a cAMP detection reagent in the kit was added. One hour later, time-resolved fluorescence measurement was performed using an EnVision plate reader (Perkin Elmer). The test compound and each control drug (δ: SNC 80, µ: DAMGO, κ: U-69593) were evaluated in a concentration range of 10⁻¹² to 10⁻⁵ M. A dose reaction curve of the test compound was determined from a fluorescence value at 665 nm, and an EC₅₀ value and an Eₘₐₓ value were calculated. The Eₘₐₓ value was determined as a ratio of a maximum reaction of the test compound when a maximum reaction of each control drug was taken as 100%.
SNC80: (+)-4-[(αR)-α-((2S,5R)-4-allyl-2,5-dimethyl-1-piperazinyl)-3-methoxybenzyl]-N,N-diethylbenzamide
DAMGO: [D-Ala²,N-MePhe⁴,Gly-ol]enkephalin
U-69593: (+)-(5α,7α,8β)-N-methyl-N-[7-(1-pyrrolidinyl)-1-oxaspiro[4.5]deca-8-yl]benzeneacetamide

Accession number and catalog number
δ: Catalog No. CT6607, accession No. NM_000911.2
µ: Catalog No. CT6605, accession No. NM_000914
κ: Catalog No. CT6606, accession No. NM_000912 (ChanTest Corporation)

**[Table 1]**

| Compound number | EC₅₀:nM (Eₘₐₓ:%) | | |
|---|---|---|---|
| | δ receptor | µ receptor | κ receptor |
| 20 | 0.89(86) | 8.1(10) | 8.7(12) |
| 21 | 0.76(81) | N.C. | N.C. |
| 22 | 0.18(107) | 15(15) | 13(35) |
| 23 | 0.48(95) | N.C. | N.C. |
| 24 | 0.14(108) | N.C. | 17(23) |
| 27 | 0.83(51) | N.C. | N.C. |
| 28 | 0.33(101) | N.C. | 58(15) |
| 30, 31 | 0.70(86) | N.C. | N.C. |
| 32 | 0.089(111) | N.C. | N.C. |
| 33 | 0.14(108) | N.C. | 44(13) |
| 34, 35 | 1.3(42) | N.C. | N.C. |
| 36 | 3.4(65) | N.C. | N.C. |
| 42 | 1.0(90) | N.C. | N.C. |
| 45 | 0.75(72) | N.C. | N.C. |
| 46 | 0.17(105) | N.C. | N.C. |
| 49 | 0.50(73) | N.C. | N.C. |
| 51 | 0.94(54) | N.C. | N.C. |
| 61 | 15(91) | 257(63) | 1033(14) |
| 68 | 1.4(100) | N.C. | N.C. |
| 82 | 0.93(39) | N.C. | N.C. |
| 83 | 0.13(99) | 11(16) | 50(30) |
| 84 | 0.34(95) | N.C. | N.C. |
| 85 | 0.092(104) | 16(26) | 17(25) |
| 86 | 0.12(101) | N.C. | N.C. |
| 87 | 0.11(106) | 28(24) | 59(30) |
| 88 | 0.086(113) | N.C. | N.C. |
| 89 | 0.069(112) | N.C. | N.C. |
| 90 | 0.092(116) | N.C. | 8.1(15) |
| 91 | 0.23(112) | 32(12) | 15(25) |
| 92 | 0.51(103) | 52(17) | 16(20) |

| | | | |
|---|---|---|---|
| N.C.: EC₅₀ value was not calculated as no dose response was obtained. | | | |

As shown in Table 1, it was confirmed that the compound of the present invention showed high selectivity for the opioid δ receptor, and a large number of compounds had strong agonist activity. Since the compound of the present invention has no agonist activity for µ and κ receptors or shows only weak agonist activity, reduction of side effects is expected.

### Example 66

### Ether-a-go-go related gene (hERG) potassium channel inhibition test

A test was performed using hERG channel stably expressing CHO cells (purchased from Channelopathy Foundation) with a Port-a-Patch auto patch clamping device (Nanion Technologies). A membrane potential of the cells was held at -80 mV, and then a test pulse of -50 mV for 1.5 seconds was applied at a frequency of once every 10 seconds following a depolarization pulse of +20 mV for 1.5 seconds. An hERG current was confirmed by a tail current induced by the test pulse. A test compound was dissolved in an extracellular solution (13,740 mM NaCl, 4 mM KCl, 1.82 mM CaCl₂, 1 mM MgCl₂, 105 mM D(+)-glucose, 10 mM HEPES, pH 7.4) and perfused at room temperature for 5 minutes. An inhibition ratio was determined from a ratio of a tail current value after application of the compound when a maximum tail current value before application of the compound was taken as 100%. For the test, cells having a peak current value of the tail current of 300 pA or more, tail current run-down of less than 10% of a current initial value, and a leak current of less than 200 pA were used.

For example, the compound 22, the compound 23, the compound 24, and the compound 44 showed only weak inhibitory effect. It is presumed from this that the compound of the present invention has a low risk of delaying ventricular repolarization and prolonging QT interval in humans.

## Claims

1. A compound represented by the following general formula (I), a stereoisomer of the compound, a pharmaceutically acceptable salt thereof, or a solvate thereof: 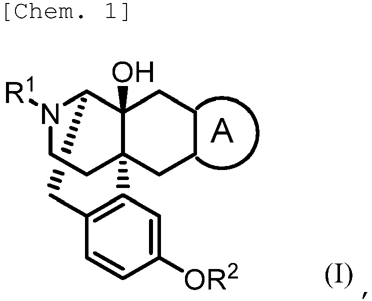 in the formula, 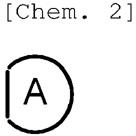 is represented by 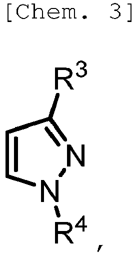 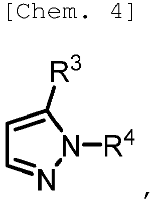 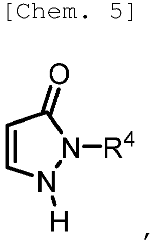 or 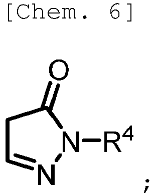
R¹ represents a hydrogen atom, a C₁₋₆ alkyl group which may have a substituent, a C₂₋₆ alkenyl group which may have a substituent, or a C₃₋₁₀ cycloalkylmethyl group which may have a substituent;
R² represents a hydrogen atom or a hydroxy protecting group;
R³ represents a hydroxy group, a C₁₋₆ alkyl group which may have a substituent, a C₃₋₁₀ cycloalkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent; and
R⁴ represents a hydrogen atom, a C₁₋₆ alkyl group which may have a substituent, a C₁₋₁₂ acyl group which may have a substituent, a C₇₋₁₂ aralkyl group which may have a substituent, a C₃₋₁₀ cycloalkyl group which may have a substituent, a C₃₋₁₀ cycloalkyl C₁₋₆ alkyl group which may have a substituent, a saturated heterocyclic group which may have a substituent, a partially unsaturated heterocyclic group which may have a substituent, a C₆₋₁₀ aryl group which may have a substituent, or a 5- to 10-membered heteroaryl group which may have a substituent.

2. The compound, the stereoisomer of the compound, the pharmaceutically acceptable salt thereof, or the solvate thereof according to claim 1, wherein 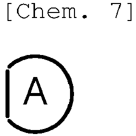 is

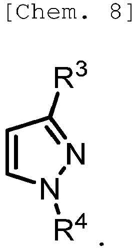

3. The compound, the stereoisomer of the compound, the pharmaceutically acceptable salt thereof, or the solvate thereof according to claim 1, wherein 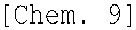 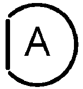 is

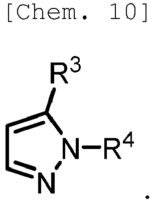

4. The compound, the stereoisomer of the compound, the pharmaceutically acceptable salt thereof, or the solvate thereof according to any one of claims 1 to 3, wherein R¹ is a methyl group, an allyl group, or a cyclopropylmethyl group.

5. The compound, the stereoisomer of the compound, the pharmaceutically acceptable salt thereof, or the solvate thereof according to any one of claims 1 to 3, wherein R¹ is a methyl group or a cyclopropylmethyl group.

6. The compound, the stereoisomer of the compound, the pharmaceutically acceptable salt thereof, or the solvate thereof according to any one of claims 1 to 3, wherein R¹ is a cyclopropylmethyl group.

7. The compound, the stereoisomer of the compound, the pharmaceutically acceptable salt thereof, or the solvate thereof according to any one of claims 1 to 6, wherein R³ is a hydroxy group, a C₁₋₆ alkyl group, a halo C₁₋₆ alkyl group, or a C₆₋₁₀ aryl group which may have a substituent.

8. The compound, the stereoisomer of the compound, the pharmaceutically acceptable salt thereof, or the solvate thereof according to any one of claims 1 to 6, wherein R³ is a methyl group, a trifluoromethyl group, or a phenyl group.

9. The compound, the stereoisomer of the compound, the pharmaceutically acceptable salt thereof, or the solvate thereof according to any one of claims 1 to 6, wherein R³ is a methyl group.

10. The compound, the stereoisomer of the compound, the pharmaceutically acceptable salt thereof, or the solvate thereof according to any one of claims 1 to 9, wherein R⁴ is a hydrogen atom, a C₁₋₆ alkyl group, a halo C₁₋₆ alkyl group, a C₁₋₆ alkyl group substituted with a hydroxy group, a C₁₋₆ alkyl group substituted with a C₁₋₆ alkoxy group, a benzoyl group which may have a substituent, a C₃₋₁₀ cycloalkyl group which may have a substituent, a C₃₋₁₀ cycloalkylmethyl group which may have a substituent, a C₆₋₁₀ aryl group which may have a substituent, or a 5- to 10-membered heteroaryl group which may have a substituent.

11. The compound, the stereoisomer of the compound, the pharmaceutically acceptable salt thereof, or the solvate thereof according to claim 10, wherein the 5- to 10-membered heteroaryl group which may have a substituent of R⁴ is a nitrogen-containing 5- or 6-membered heteroaryl group which may have a substituent.

12. The compound, the stereoisomer of the compound, the pharmaceutically acceptable salt thereof, or the solvate thereof according to any one of claims 1 to 9, wherein R⁴ is a hydrogen atom, a C₁₋₆ alkyl group, a C₁₋₆ alkyl group substituted with a hydroxy group, a C₁₋₆ alkyl group substituted with a C₁₋₆ alkoxy group, a benzoyl group, a C₃₋₇ cycloalkyl group, a C₃₋₇ cycloalkylmethyl group, a phenyl group, an oxazolyl group, a thiazolyl group, a pyrimidinyl group, or a pyridyl group.

13. The compound, the stereoisomer of the compound, the pharmaceutically acceptable salt thereof, or the solvate thereof according to any one of claims 1 to 9, wherein R⁴ is a 2-oxopyridyl group, a tetrahydropyranyl group, a tetrahydrothiopyranyl group, a 1-methylpiperidyl group, or an S-oxide-tetrahydrothiopyranyl group.

14. The compound, the stereoisomer of the compound, the pharmaceutically acceptable salt thereof, or the solvate thereof according to any one of claims 1 to 9, wherein R⁴ is a hydrogen atom, an isopropyl group, a tert-butyl group, a 2-hydroxyethyl group, a benzoyl group, a cyclohexyl group, a cyclopropylmethyl group, a cyclohexylmethyl group, a phenyl group, a 2-thiazolyl group, or a 2-, 3-, or 4-pyridyl group.

15. The compound, the stereoisomer of the compound, the pharmaceutically acceptable salt thereof, or the solvate thereof according to any one of claims 1 to 9, wherein R⁴ is a 2-oxo-4-pyridyl group, a cyclopropyl group, a cyclobutyl group, a cyclopentyl group, a 4-tetrahydropyranyl group, a 4-tetrahydrothiopyranyl group, a 1-methylpiperidin-4-yl group, or an S-oxide-tetrahydrothiopyran-4-yl group.

16. The compound, the stereoisomer of the compound, the pharmaceutically acceptable salt thereof, or the solvate thereof according to any one of claims 1 to 15, wherein R² is a hydrogen atom.

17. The compound, the stereoisomer of the compound, the pharmaceutically acceptable salt thereof, or the solvate thereof according to claim 1, wherein the compound is selected from
(6R,6aS,11aR)-14-(cyclopropylmethyl)-2-methoxy-8-methyl-9-phenyl-5,6,9,11-tetrahydro-6,11a-(epiminoethano)naphtho[2,1-f]indazol-6a(7H)-ol (Compound 4),
(6R,6aS,11aR)-14-(cyclopropylmethyl)-2-methoxy-8-methyl-10-phenyl-5,6,10,11-tetrahydro-6,11a-(epiminoethano)naphtho[2,1-f]indazol-6a(7H)-ol (Compound 5),
(6R,6aS,11aR)-14-(cyclopropylmethyl)-9-isopropyl-2-methoxy-8-methyl-5,6,9,11-tetrahydro-6,11a-(epiminoethano)naphtho[2,1-f]indazol-6a(7H)-ol (Compound 6),
(6R,6aS,11aR)-14-(cyclopropylmethyl)-10-isopropyl-2-methoxy-8-methyl-5,6,10,11-tetrahydro-6,11a-(epiminoethano)naphtho[2,1-f]indazol-6a(7H)-ol (Compound 7),
(6R,6aS,11aR)-9-(tert-butyl)-14-(cyclopropylmethyl)-2-methoxy-8-methyl-5,6,9,11-tetrahydro-6,11a-(epiminoethano)naphtho[2,1-f]indazol-6a(7H)-ol (Compound 8),
(6R,6aS,11aR)-10-(tert-butyl)-14-(cyclopropylmethyl)-2-methoxy-8-methyl-5,6,10,11-tetrahydro-6,11a-(epiminoethano)naphtho[2,1-f]indazol-6a(7H)-ol (Compound 9),
(6R,6aS,11aR)-10-benzyl-14-(cyclopropylmethyl)-2-methoxy-8-methyl-5,6,10,11-tetrahydro-6,11a-(epiminoethano)naphtho[2,1-f]indazol-6a(7H)-ol (Compound 10),
(6R,6aS,11aR)-9-benzyl-14-(cyclopropylmethyl)-2-methoxy-8-methyl-5,6,9,11-tetrahydro-6,11a-(epiminoethano)naphtho[2,1-f]indazol-6a(7H)-ol (Compound 10a),
(6R,6aS,11aR)-14-(cyclopropylmethyl)-2-methoxy-8-methyl-10-(pyridin-2-yl)-5,6,10,11-tetrahydro-6,11a-(epiminoethano)naphtho[2,1-f]indazol-6a(7H)-ol (Compound 11),
(6R,6aS,11aR)-14-(cyclopropylmethyl)-2-methoxy-8-methyl-9-(pyridin-2-yl)-5,6,9,11-tetrahydro-6,11a-(epiminoethano)naphtho[2,1-f]indazol-6a(7H)-ol (Compound 11a),
(6R,6aS,11aR)-14-(cyclopropylmethyl)-2-methoxy-8-methyl-10-(pyridin-4-yl)-5,6,10,11-tetrahydro-6,11a-(epiminoethano)naphtho[2,1-f]indazol-6a(7H)-ol (Compound 12),
(6R,6aS,11aR)-14-(cyclopropylmethyl)-2-methoxy-8-methyl-9-(pyridin-4-yl)-5,6,9,11-tetrahydro-6,11a-(epiminoethano)naphtho[2,1-f]indazol-6a(7H)-ol (Compound 12a),
(6R,6aS,11aR)-14-(cyclopropylmethyl)-2-methoxy-8-methyl-10-(pyrimidin-2-yl)-5,6,10,11-tetrahydro-6,11a-(epiminoethano)naphtho[2,1-f]indazol-6a(7H)-ol (Compound 13),
(6R,6aS,11aR)-14-(cyclopropylmethyl)-2-methoxy-8-methyl-9-(pyrimidin-2-yl)-5,6,9,11-tetrahydro-6,11a-(epiminoethano)naphtho[2,1-f]indazol-6a(7H)-ol (Compound 13a),
4-((6R,6aS,11aR)-14-(cyclopropylmethyl)-6a-hydroxy-2-methoxy-8-methyl-6,6a,7,11-tetrahydro-6,11a-(epiminoethano)naphtho[2,1-f]indazol-10(5H)-yl)pyridin-2(1H)-one (Compound 14),
4-((6R,6aS,11aR)-14-(cyclopropylmethyl)-6a-hydroxy-2-methoxy-8-methyl-5,6,6a,7-tetrahydro-6,11a-(epiminoethano)naphtho[2,1-f]indazol-9(11H)-yl)pyridin-2(1H)-one (Compound 15),
(6R,6aS,11aR)-10-cyclohexyl-14-(cyclopropylmethyl)-2-methoxy-8-methyl-5,6,10,11-tetrahydro-6,11a-(epiminoethano)naphtho[2,1-f]indazol-6a(7H)-ol (Compound 16),
(6R,6aS,11aR)-9-cyclohexyl-14-(cyclopropylmethyl)-2-methoxy-8-methyl-5,6,9,11-tetrahydro-6,11a-(epiminoethano)naphtho[2,1-f]indazol-6a(7H)-ol (Compound 17),
(6R,6aS,11aR)-14-(cyclopropylmethyl)-2-methoxy-8-methyl-5,6,9,11-tetrahydro-6,11a-(epiminoethano)naphtho[2,1-f]indazol-6a(7H)-ol (Compound 18),
(6R,6aS,11aR)-14-(cyclopropylmethyl)-2-methoxy-8-methyl-5,6,10,11-tetrahydro-6,11a-(epiminoethano)naphtho[2,1-f]indazol-6a(7H)-ol (Compound 19),
(6R,6aS,11aR)-14-(cyclopropylmethyl)-8-methyl-9-phenyl-5,6,9,11-tetrahydro-6,11a-(epiminoethano)naphtho[2,1-f]indazole-2,6a(7H)-diol (Compound 20),
(6R,6aS,11aR)-14-(cyclopropylmethyl)-8-methyl-10-phenyl-5,6,10,11-tetrahydro-6,11a-(epiminoethano)naphtho[2,1-f]indazole-2,6a(7H)-diol (Compound 21),
(6R,6aS,11aR)-14-(cyclopropylmethyl)-9-isopropyl-8-methyl-5,6,9,11-tetrahydro-6,11a-(epiminoethano)naphtho[2,1-f]indazole-2,6a(7H)-diol (Compound 22),
(6R,6aS,11aR)-14-(cyclopropylmethyl)-10-isopropyl-8-methyl-5,6,10,11-tetrahydro-6,11a-(epiminoethano)naphtho[2,1-f]indazole-2,6a(7H)-diol (Compound 23),
(6R,6aS,11aR)-9-(tert-butyl)-14-(cyclopropylmethyl)-8-methyl-5,6,9,11-tetrahydro-6,11a-(epiminoethano)naphtho[2,1-f]indazole-2,6a(7H)-diol (Compound 24),
(6R,6aS,11aR)-10-(tert-butyl)-14-(cyclopropylmethyl)-8-methyl-5,6,10,11-tetrahydro-6,11a-(epiminoethano)naphtho[2,1-f]indazole-2,6a(7H)-diol (Compound 25),
(6R,6aS,11aR)-10-benzyl-14-(cyclopropylmethyl)-8-methyl-5,6,10,11-tetrahydro-6,11a-(epiminoethano)naphtho[2,1-f]indazole-2,6a(7H)-diol (Compound 26),
(6R,6aS,11aR)-14-(cyclopropylmethyl)-8-methyl-10-(pyridin-2-yl)-5,6,10,11-tetrahydro-6,11a-(epiminoethano)naphtho[2,1-f]indazole-2,6a(7H)-diol (Compound 27),
(6R,6aS,11aR)-14-(cyclopropylmethyl)-8-methyl-10-(pyridin-4-yl)-5,6,10,11-tetrahydro-6,11a-(epiminoethano)naphtho[2,1-f]indazole-2,6a(7H)-diol (Compound 28),
(6R,6aS,11aR)-14-(cyclopropylmethyl)-8-methyl-10-(pyrimidin-2-yl)-5,6,10,11-tetrahydro-6,11a-(epiminoethano)naphtho[2,1-f]indazole-2,6a(7H)-diol (Compound 29),
4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)pyridin-2(1H)-one (Compound 30),
4-((6R,6aS,11aR)-14-(cyclopropylmethyl)-2,6a-dihydroxy-8-methyl-5,6,6a,7-tetrahydro-6,11a-(epiminoethano)naphtho[2,1-f]indazol-9(11H)-yl)pyridin-2(1H)-one (Compound 31),
(6R,6aS,11aR)-10-cyclohexyl-14-(cyclopropylmethyl)-8-methyl-5,6,10,11-tetrahydro-6,11a-(epiminoethano)naphtho[2,1-f]indazole-2,6a(7H)-diol (Compound 32),
(6R,6aS,11aR)-9-cyclohexyl-14-(cyclopropylmethyl)-8-methyl-5,6,9,11-tetrahydro-6,11a-(epiminoethano)naphtho[2,1-f]indazole-2,6a(7H)-diol (Compound 33),
(6R,6aS,11aR)-14-(cyclopropylmethyl)-8-methyl-5,6,9,11-tetrahydro-6,11a-(epiminoethano)naphtho[2,1-f]indazole-2,6a(7H)-diol (Compound 34),
(6R,6aS,11aR)-14-(cyclopropylmethyl)-8-methyl-5,6,10,11-tetrahydro-6,11a-(epiminoethano)naphtho[2,1-f]indazole-2,6a(7H)-diol (Compound 35),
((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)(phenyl)methanone (Compound 36),
(6R,6aS,11aR)-9-(cyclohexylmethyl)-14-(cyclopropylmethyl)-2-methoxy-8-methyl-5,6,9,11-tetrahydro-6,11a-(epiminoethano)naphtho[2,1-f]indazol-6a(7H)-ol (Compound 37),
(6R,6aS,11aR)-10-(cyclohexylmethyl)-14-(cyclopropylmethyl)-2-methoxy-8-methyl-5,6,10,11-tetrahydro-6,11a-(epiminoethano)naphtho[2,1-f]indazol-6a(7H)-ol (Compound 38),
(6R,6aS,11aR)-14-(cyclopropylmethyl)-2-methoxy-10-(2-methoxyethyl)-8-methyl-5,6,10,11-tetrahydro-6,11a-(epiminoethano)naphtho[2,1-f]indazol-6a(7H)-ol (Compound 39),
(6R,6aS,11aR)-10,14-bis(cyclopropylmethyl)-2-methoxy-8-methyl-5,6,10,11-tetrahydro-6,11a-(epiminoethano)naphtho[2,1-f]indazol-6a(7H)-ol (Compound 40),
(6R,6aS,11aR)-9,14-bis(cyclopropylmethyl)-2-methoxy-8-methyl-5,6,9,11-tetrahydro-6,11a-(epiminoethano)naphtho[2,1-f]indazol-6a(7H)-ol (Compound 41),
(6R,6aS,11aR)-9-(cyclohexylmethyl)-14-(cyclopropylmethyl)-8-methyl-5,6,9,11-tetrahydro-6,11a-(epiminoethano)naphtho[2,1-f]indazole-2,6a(7H)-diol (Compound 42),
(6R,6aS,11aR)-10-(cyclohexylmethyl)-14-(cyclopropylmethyl)-8-methyl-5,6,10,11-tetrahydro-6,11a-(epiminoethano)naphtho[2,1-f]indazole-2,6a(7H)-diol (Compound 43),
(6R,6aS,11aR)-14-(cyclopropylmethyl)-10-(2-hydroxyethyl)-8-methyl-5,6,10,11-tetrahydro-6,11a-(epiminoethano)naphtho[2,1-f]indazole-2,6a(7H)-diol (Compound 44),
(6R,6aS,11aR)-10,14-bis(cyclopropylmethyl)-8-methyl-5,6,10,11-tetrahydro-6,11a-(epiminoethano)naphtho[2,1-f]indazole-2,6a(7H)-diol (Compound 45),
(6R,6aS,11aR)-9,14-bis(cyclopropylmethyl)-8-methyl-5,6,9,11-tetrahydro-6,11a-(epiminoethano)naphtho[2,1-f]indazole-2,6a(7H)-diol (Compound 46),
(6R,6aS,11aR)-14-(cyclopropylmethyl)-2-methoxy-8-methyl-10-(pyridin-3-yl)-5,6,10,11-tetrahydro-6,11a-(epiminoethano)naphtho[2,1-f]indazol-6a(7H)-ol (Compound 47),
(6R,6aS,11aR)-14-(cyclopropylmethyl)-2-methoxy-8-methyl-9-(pyridin-3-yl)-5,6,9,11-tetrahydro-6,11a-(epiminoethano)naphtho[2,1-f]indazol-6a(7H)-ol (Compound 48),
(6R,6aS,11aR)-14-(cyclopropylmethyl)-8-methyl-10-(pyridin-3-yl)-5,6,10,11-tetrahydro-6,11a-(epiminoethano)naphtho[2,1-f]indazole-2,6a(7H)-diol (Compound 49),
(6R,6aS,11aR)-14-(cyclopropylmethyl)-2-methoxy-8-methyl-10-(thiazol-2-yl)-5,6,10,11-tetrahydro-6,11a-(epiminoethano)naphtho[2,1-f]indazol-6a(7H)-ol (Compound 50),
(6R,6aS,11aR)-14-(cyclopropylmethyl)-8-methyl-10-(thiazol-2-yl)-5,6,10,11-tetrahydro-6,11a-(epiminoethano)naphtho[2,1-f]indazole-2,6a(7H)-diol (Compound 51),
(6R,6aS,11aR)-2-(benzyloxy)-14-(cyclopropylmethyl)-8-methyl-5,6,9,11-tetrahydro-6,11a-(epiminoethano)naphtho[2,1-f]indazol-6a(7H)-ol (Compound 56),
(6R,6aS,11aR)-2-(benzyloxy)-14-(cyclopropylmethyl)-8-methyl-5,6,10,11-tetrahydro-6,11a-(epiminoethano)naphtho[2,1-f]indazol-6a(7H)-ol (Compound 57),
(6R,6aS,11aR)-2-(benzyloxy)-14-(cyclopropylmethyl)-10-(2-methoxyethyl)-8-methyl-5,6,10,11-tetrahydro-6,11a-(epiminoethano)naphtho[2,1-f]indazol-6a(7H)-ol (Compound 58),
(6R,6aS,11aR)-14-(cyclopropylmethyl)-10-(2-methoxyethyl)-8-methyl-5,6,10,11-tetrahydro-6,11a-(epiminoethano)naphtho[2,1-f]indazole-2,6a(7H)-diol (Compound 59),
(6R,6aS,11aR)-2-methoxy-8,14-dimethyl-10-phenyl-5,6,10,11-tetrahydro-6,11a-(epiminoethano)naphtho[2,1-f]indazol-6a(7H)-ol (Compound 60),
(6R,6aS,11aR)-8,14-dimethyl-10-phenyl-5,6,10,11-tetrahydro-6,11a-(epiminoethano)naphtho[2,1-f]indazole-2,6a(7H)-diol (Compound 61),
(6R,6aS,11aR)-10-benzyl-2-methoxy-8,14-dimethyl-5,6,10,11-tetrahydro-6,11a-(epiminoethano)naphtho[2,1-f]indazol-6a(7H)-ol (Compound 65),
(6R,6aS,11aR)-9-benzyl-2-methoxy-8,14-dimethyl-5,6,9,11-tetrahydro-6,11a-(epiminoethano)naphtho[2,1-f]indazol-6a(7H)-ol (Compound 65a),
(6R,6aS,11aR)-10-benzyl-8,14-dimethyl-5,6,10,11-tetrahydro-6,11a-(epiminoethano)naphtho[2,1-f]indazole-2,6a(7H)-diol (Compound 66),
(6R,6aS,11aR)-2-(benzyloxy)-14-(cyclopropylmethyl)-6a-hydroxy-9-phenyl-6,6a,7,7a,9,11-hexahydro-6,11a-(epiminoethano)naphtho[2,1-f]indazol-8(5H)-one (Compound 67),
(6R,6aS,11aR)-14-(cyclopropylmethyl)-2,6a-dihydroxy-9-phenyl-6,6a,7,9,10,11-hexahydro-6,11a-(epiminoethano)naphtho[2,1-f]indazol-8(5H)-one (Compound 68), and
(6R,6aS,11aR)-14-(cyclopropylmethyl)-2,6a-dihydroxy-9-isopropyl-6,6a,7,9,10,11-hexahydro-6,11a-(epiminoethano)naphtho[2,1-f]indazol-8(5H)-one (Compound 69) .

18. The compound, the stereoisomer of the compound, the pharmaceutically acceptable salt thereof, or the solvate thereof according to claim 1, wherein the compound is selected from
(6R,6aS,11aR)-10-cyclopropyl-14-(cyclopropylmethyl)-2-methoxy-8-methyl-5,6,10,11-tetrahydro-6,11a-(epiminoethano)naphtho[2,1-f]indazol-6a(7H)-ol (Compound 70),
(6R,6aS,11aR)-9-cyclopropyl-14-(cyclopropylmethyl)-2-methoxy-8-methyl-5,6,9,11-tetrahydro-6,11a-(epiminoethano)naphtho[2,1-f]indazol-6a(7H)-ol (Compound 71),
(6R,6aS,11aR)-10-cyclobutyl-14-(cyclopropylmethyl)-2-methoxy-8-methyl-5,6,10,11-tetrahydro-6,11a-(epiminoethano)naphtho[2,1-f]indazol-6a(7H)-ol (Compound 72),
(6R,6aS,11aR)-9-cyclobutyl-14-(cyclopropylmethyl)-2-methoxy-8-methyl-5,6,9,11-tetrahydro-6,11a-(epiminoethano)naphtho[2,1-f]indazol-6a(7H)-ol (Compound 73),
(6R,6aS,11aR)-10-cyclopentyl-14-(cyclopropylmethyl)-2-methoxy-8-methyl-5,6,10,11-tetrahydro-6,11a-(epiminoethano)naphtho[2,1-f]indazol-6a(7H)-ol (Compound 74),
(6R,6aS,11aR)-9-cyclopentyl-14-(cyclopropylmethyl)-2-methoxy-8-methyl-5,6,9,11-tetrahydro-6,11a-(epiminoethano)naphtho[2,1-f]indazol-6a(7H)-ol (Compound 75),
(6R,6aS,11aR)-14-(cyclopropylmethyl)-2-methoxy-8-methyl-10-(tetrahydro-2H-pyran-4-yl)-5,6,10,11-tetrahydro-6,11a-(epiminoethano)naphtho[2,1-f]indazol-6a(7H)-ol (Compound 76),
(6R,6aS,11aR)-14-(cyclopropylmethyl)-2-methoxy-8-methyl-10-(tetrahydro-2H-pyran-4-yl)-5,6,10,11-tetrahydro-6,11a-(epiminoethano)naphtho[2,1-f]indazol-6a(7H)-ol (Compound 76),
(6R,6aS,11aR)-14-(cyclopropylmethyl)-2-methoxy-8-methyl-10-(tetrahydro-2H-pyran-4-yl)-5,6,9,11-tetrahydro-6,11a-(epiminoethano)naphtho[2,1-f]indazol-6a(7H)-ol (Compound 77),
(6R,6aS,11aR)-14-(cyclopropylmethyl)-2-methoxy-8-methyl-10-(tetrahydro-2H-thiopyran-4-yl)-5,6,10,11-tetrahydro-6,11a-(epiminoethano)naphtho[2,1-f]indazol-6a(7H)-ol (Compound 78),
(6R,6aS,11aR)-14-(cyclopropylmethyl)-2-methoxy-8-methyl-10-(tetrahydro-2H-thiopyran-4-yl)-5,6,9,11-tetrahydro-6,11a-(epiminoethano)naphtho[2,1-f]indazol-6a(7H)-ol (Compound 79),
(6R,6aS,11aR)-14-(cyclopropylmethyl)-2-methoxy-8-methyl-10-(1-methylpiperidin-4-yl)-5,6,10,11-tetrahydro-6,11a-(epiminoethano)naphtho[2,1-f]indazol-6a(7H)-ol (Compound 80),
(6R,6aS,11aR)-14-(cyclopropylmethyl)-2-methoxy-8-methyl-10-(1-methylpiperidin-4-yl)-5,6,9,11-tetrahydro-6,11a-(epiminoethano)naphtho[2,1-f]indazol-6a(7H)-ol (Compound 81),
(6R,6aS,11aR)-10-cyclopropyl-14-(cyclopropylmethyl)-8-methyl-5,6,10,11-tetrahydro-6,11a-(epiminoethano)naphtho[2,1-f]indazole-2,6a(7H)-diol (Compound 82),
(6R,6aS,11aR)-9-cyclopropyl-14-(cyclopropylmethyl)-8-methyl-5,6,9,11-tetrahydro-6,11a-(epiminoethano)naphtho[2,1-f]indazole-2,6a(7H)-diol (Compound 83),
(6R,6aS,11aR)-10-cyclobutyl-14-(cyclopropylmethyl)-8-methyl-5,6,10,11-tetrahydro-6,11a-(epiminoethano)naphtho[2,1-f]indazole-2,6a(7H)-diol (Compound 84),
(6R,6aS,11aR)-9-cyclobutyl-14-(cyclopropylmethyl)-8-methyl-5,6,9,11-tetrahydro-6,11a-(epiminoethano)naphtho[2,1-f]indazole-2,6a(7H)-diol (Compound 85),
(6R,6aS,11aR)-10-cyclopentyl-14-(cyclopropylmethyl)-8-methyl-5,6,10,11-tetrahydro-6,11a-(epiminoethano)naphtho[2,1-f]indazole-2,6a(7H)-diol (Compound 86),
(6R,6aS,11aR)-9-cyclopentyl-14-(cyclopropylmethyl)-8-methyl-5,6,9,11-tetrahydro-6,11a-(epiminoethano)naphtho[2,1-f]indazole-2,6a(7H)-diol (Compound 87),
(6R,6aS,11aR)-14-(cyclopropylmethyl)-8-methyl-10-(tetrahydro-2H-pyran-4-yl)-5,6,10,11-tetrahydro-6,11a-(epiminoethano)naphtho[2,1-f]indazole-2,6a(7H)-diol (Compound 88),
(6R,6aS,11aR)-14-(cyclopropylmethyl)-8-methyl-10-(tetrahydro-2H-thiopyran-4-yl)-5,6,10,11-tetrahydro-6,11a-(epiminoethano)naphtho[2,1-f]indazole-2,6a(7H)-diol (Compound 89),
(6R,6aS,11aR)-14-(cyclopropylmethyl)-8-methyl-9-(tetrahydro-2H-thiopyran-4-yl)-5,6,9,11-tetrahydro-6,11a-(epiminoethano)naphtho[2,1-f]indazole-2,6a(7H)-diol (Compound 90),
(6R,6aS,11aR)-14-(cyclopropylmethyl)-8-methyl-10-(1-methylpiperidin-4-yl)-5,6,10,11-tetrahydro-6,11a-(epiminoethano)naphtho[2,1-f]indazole-2,6a(7H)-diol (Compound 91),
(6R,6aS,11aR)-14-(cyclopropylmethyl)-8-methyl-9-(1-methylpiperidin-4-yl)-5,6,9,11-tetrahydro-6,11a-(epiminoethano)naphtho[2,1-f]indazole-2,6a(7H)-diol (Compound 92),
(1S,4S)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 93), and
(1S,4R)-4-((6R,6aS,11aR)-14-(cyclopropylmethyl)-2,6a-dihydroxy-8-methyl-6,6a,7,11-tetrahydro-6,11a-(epiminoethano)naphtho[2,1-f]indazol-10(5H)-yl)tetrahydro-2H-thiopyran 1-oxide (Compound 94).

19. The compound, the stereoisomer of the compound, the pharmaceutically acceptable salt thereof, or the solvate thereof according to any one of claims 1 to 15, wherein R² is a hydroxy protecting group.

20. A pharmaceutical composition comprising the compound, the stereoisomer of the compound, the pharmaceutically acceptable salt thereof, or the solvate thereof according to any one of claims 1 to 18.

21. An opioid δ receptor agonist comprising the compound, the stereoisomer of the compound, the pharmaceutically acceptable salt thereof, or the solvate thereof according to any one of claims 1 to 18.

22. An analgesic comprising the compound, the stereoisomer of the compound, the pharmaceutically acceptable salt thereof, or the solvate thereof according to any one of claims 1 to 18.

23. An antidepressant comprising the compound, the stereoisomer of the compound, the pharmaceutically acceptable salt thereof, or the solvate thereof according to any one of claims 1 to 18.

24. An anxiolytic comprising the compound, the stereoisomer of the compound, the pharmaceutically acceptable salt thereof, or the solvate thereof according to any one of claims 1 to 18.

25. A therapeutic agent for dysuria comprising the compound, the stereoisomer of the compound, the pharmaceutically acceptable salt thereof, or the solvate thereof according to any one of claims 1 to 18.
